(19) Europäisches Patentamt
European Patent Office
Office européen des brevets

(11) EP 2 341 052 A1

(12) EUROPEAN PATENT APPLICATION
published in accordance with Art. 153(4) EPC

(43) Date of publication:
**06.07.2011 Bulletin 2011/27**

(21) Application number: **09811527.2**

(22) Date of filing: **02.09.2009**

(51) Int Cl.:
***C07D 498/04*** *(2006.01)* ***A61K 31/5383*** *(2006.01)*
***A61K 31/541*** *(2006.01)* ***A61P 1/00*** *(2006.01)*
***A61P 1/02*** *(2006.01)* ***A61P 1/04*** *(2006.01)*
***A61P 1/16*** *(2006.01)* ***A61P 1/18*** *(2006.01)*
***A61P 3/04*** *(2006.01)* ***A61P 3/10*** *(2006.01)*
***A61P 5/14*** *(2006.01)* ***A61P 7/00*** *(2006.01)*
***A61P 7/02*** *(2006.01)* ***A61P 7/06*** *(2006.01)*
***A61P 9/00*** *(2006.01)* ***A61P 9/04*** *(2006.01)*
***A61P 9/10*** *(2006.01)* ***A61P 11/00*** *(2006.01)*

(86) International application number:
**PCT/JP2009/065366**

(87) International publication number:
**WO 2010/027002 (11.03.2010 Gazette 2010/10)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **05.09.2008 JP 2008228905**
**17.07.2009 JP 2009169598**

(71) Applicant: **Shionogi & Co., Ltd.**
**Osaka 541-0045 (JP)**

(72) Inventors:
- **FUJIOKA Masahiko**
**Osaka-shi**
**Osaka 553-0002 (JP)**
- **MITSUMORI Susumu**
**Osaka-shi**
**Osaka 553-0002 (JP)**
- **KUGIMIYA Akira**
**Osaka-shi**
**Osaka 553-0002 (JP)**
- **TANIYAMA Daisuke**
**Osaka-shi**
**Osaka 553-0002 (JP)**

(74) Representative: **Harmsen, Dirk**
**Vossius & Partner**
**Siebertstrasse 4**
**81675 München (DE)**

# (54) RING-FUSED MORPHOLINE DERIVATIVE HAVING PI3K-INHIBITING ACTIVITY

(57) The present invention provides compounds or a pharmaceutically acceptable salt thereof which inhibit the activity of PI3K to regulate many biological processes including the growth, differentiation, survival, proliferation, migration, metabolism, and the like of cells and are therefore useful for the prophylaxis/therapy of diseases including inflammatory diseases, arteriosclerosis, vascular/circulatory diseases, cancer/tumors, immune system diseases, cell proliferative diseases, infectious diseases, and the like. The above problem was solved by providing a ring-fused morpholine compound shown in the present specification, or a pharmaceutically acceptable salt thereof.



Printed by Jouve, 75001 PARIS (FR)

## Description

TECHNICAL FIELD

The present invention is related to: a compound that has phosphatidylinositol-3-kinase (hereinafter also referred to as "PI3K") inhibitory activity and is useful in the therapy/prophylaxis of a variety of phosphatidylinositol-3-kinase dependent diseases including cancers, inflammatory diseases, circulatory diseases, and the like; a salt thereof; or the like.

BACKGROUND ART

Phosphatidylinositol-3-kinase is an enzyme that catalyzes not only the production of a specific phospholipase, but also an intracellularmediator from phosphatidylinositol (hereinafter also referred to as "PI") of a membrane lipid. The 3'-OH group of phosphatidylinositol is phosphorylated, and thus, when phosphatidylinositol, phosphatidylinositol 4-phosphate, and phosphatidylinositol 4,5-bisphosphate are used as substrates, phosphatidylinositol 3-phosphate, phosphatidylinositol 3,4-bisphosphate, and phosphatidylinositol 3,4,5-triphosphate (PIP3) are produced respectively.
A phospholipid (PIP3) in which the hydroxyl group at the 3-position of the inositol ring is phosphorylated by this PI3K functions as a second messenger that activates a serine/threonine kinase such as PDK1, Akt/PKB, and the like in a signal transduction route mediated by receptor stimulation. This second messenger is said to regulate many biological processes including growth, differentiation, survival, proliferation, migration and metabolism, and the like of cells.
PI3Ks are classified into three groups (*i.e*. Classes I to III) by primary structure, regulatory mechanism of activity, and specificity to a substrate. Among these, Class I is important in signaling.
Depending on the differences in the heterodimer, Class I is classified into IA (α, β, and δ), containing a subunit of 85 kDa, and IB (γ), containing a subunit of 101 kDa.
Class IA is associated with a variety of cell surface receptors such as hormones/growth factors and the like. For a signal transduction route, it is said to be a protein/kinase receptor type. Class IB is associated with a G protein receptor (GPCR), which is a receptor for chemokine and the like. Furthermore, it is said that when a specific tyrosine residue of a receptor is phosphorylated by stimulation of an activator such as a chemokine and the like, a regulatory subunit is bound to a catalytic subunit via the SH2 domain, and thereby the inhibitory activity of the regulatory subunit is reduced to exhibit enzyme activity.
PIP3 works as a messenger in intracellular signaling. In the immediate downstream of PIP3, AKT (also known as protein kinase B (PKB)) and the like are known. It is said that in the downstream route thereof, a functional protein having the PH domain is activated and thereby, a signal is transmitted.
PI3Kα and PI3Kβ are widely distributed in a variety of cells and related to cell growth/glycometabolism. Based on these actions, inhibitors of PI3Kα and PI3Kβ are utilized as anticancer agents and the like. PI3Kδ and PI3Kγ exist mainly in blood and cells of the immune (lymphatic) system. PI3Kγ is also known to be widely distributed in inflammatory cells.
Regarding PI3Kγ, on the basis of studies of knock-out mice thereof and the like, it was found that respiratory burst of a neutrophil by a chemotactic factor and the migration of a macrophage/neutrophil to an infection focus were blocked, functions of T cells/dendritic cells were thereby decreased, the degranulation of mast cells was thereby blocked, and anaphylaxis was thereby decreased. Accordingly, an inhibitor of PI3Kγ is considered useful as a therapeutic agent for these diseases. Furthermore, on the basis of studies of arthritis, it is considered useful as an inhibitor of the inflammatory-cell infiltration in a part of a joint (Non-patent Documents 1 and 2). Furthermore, studies using a PI3Kγ inhibitor report the inhibition of the activation of a mast cell (Non-patent Document 3), the inhibition of the activation/migration of a leukocyte (Non-patent Documents 4 and 5), the inhibition of lymphocyte activation (Non-patent Document 6), and the like.
On the basis of these studies, a PI3Kγ inhibitor is believed to be useful in the therapy of the following diseases/disorders: thrombus; allergy/anaphylaxis (allergic diseases include, for example, asthma, atopic dermatitis, allergic rhinitis, and the like); inflammation such as pancreatitis (Non-patent Document 7), pneumonia, airway inflammation, chronic obstructive pulmonary disease (COPD) (Non-patent Document 8 and Non-patent Document 9), arthritis (e.g., articular rheumatism (Non-patent Document 8 and 9), glomerulonephritis, and the like; systemic lupus erythematosus (SLE) (Non-patent Document 8 and Non-patent Document 9); autoimmune diseases; pulmonary disorder; circulatory diseases such as heart failure (systolic), cardiac ischemia (systolic), high blood pressure, and the like (Non-patent Document 10); wound healing; infectious diseases (Non-patent Document 11); cancer/tumors such as neoplasm (Patent Document 1); suppression of the immune reaction in organ transplantation and autoimmune diseases (Patent Document 2); and the like.
Regarding PI3Kδ, on the basis of studies of knock-out mice thereof and the like, the B cell differentiation disorder of bone marrow is induced and in immunomodulation, the role thereof is expected.

PI3K is reported to be deeply involved in various stages of articular rheumatism, such as: the T cell/D cell activation by presenting an antigen; the inflammatory cell infiltration such as neutrophil, macrophage, or the like; the synovial cell proliferation; the mast cell activation; and the like (Non-Patent Document 12).

As examples of compounds that have PI3 kinase inhibitory activity, classically, wortmannin (Non-Patent Document 13), 2-(4-morpholinyl)-8-phenyl-4H-1-benzopyran-4-one (Patent Document 2), 17β-hydroxywortmannin and a derivative thereof (Patent Document 1), and the like are known.

As ring-fused morpholine derivatives useful as medicaments, compounds disclosed in Patent Document 3, 4, 5, 6, 7, 8, 9, 10, 11, 12, 13, or 14, Non-patent Document 14 or 16, or the like are known.

Furthermore, Patent Document 15 discloses a ring-fused morpholine derivative useful as a herbicide, but does not disclose PI3K inhibitory activity thereof. Non-patent Documents 15, 17, and 18 disclose methods for synthesizing ring-fused morpholine derivatives, but does not disclose PI3K inhibitory activity thereof. Patent Document 16 and Non-patent Document 19 disclose ring-fused morpholine derivatives which are substituted with a fused thiazolyl and have PI3K inhibitory activity, but do not disclose a compound having an aryl group,a heteroaryl group, a heterocyclyl group, or a carbamoyl group of the present invention.

PRIOR ART REFERENCES

[Patent Document]

[Patent Document 1] Japanese Laid-Open Publication No. 7-145051
[Patent Document 2] International Publication No. 95/29673 pamphlet
[Patent Document 3] International Publication No. 2004/056820 pamphlet
[Patent Document 4] International Publication No. 2004/052373 pamphlet
[Patent Document 5] International Publication No. 2004/007491 pamphlet
[Patent Document 6] International Publication No. 2004/006916 pamphlet
[Patent Document 7] International Publication No. 2006/040318 pamphlet
[Patent Document 8] International Publication No. 2007/089034 pamphlet
[Patent Document 9] United States Patent Application Publication No. 2007/0117785 Specification
[Patent Document 10] International Publication No. 2006/114706 pamphlet
[Patent Document 11] International Publication No. 2006/018443 pamphlet
[Patent Document 12] International Publication No. 2005/100349 pamphlet
[Patent Document 13] International Publication No. 2002/020011 pamphlet
[Patent Document 14] International Publication No. 2002/014315 pamphlet
[Patent Document 15] International Publication No. 2001/030782 pamphlet
[Patent Document 16] International Publication No. 2008/044022 pamphlet

[Non-patent document]

[Non-patent document 1] M.P. Wymann, et al., Biochemical Society Transactions 2003, 31, pp.275-280
[Non-patent document 2] Rueckle T. et al., NATURE REVIEWS DRUG DISCOVERY 2006, 5, pp. 903-918
[Non-patent document 3] Laffargue M. et al., Immunity 2002 16: pp. 441-451
[Non-patent document 4] Hirsch E. et al., Science 2000 287: pp. 1049-1053
[Non-patent document 5] Li Z. et al., Science 2000 287; pp.982-983
[Non-patent document 6] Sasaki T. et al., Science 2000 287; pp.1040-1046
[Non-patent document 7] Lupia E. et al., Am J Pathol. 2004; 165, pp.2003-2011
[Non-patent document 8] Barber DF et al., Nat Med 2005 11: pp.933-935
[Non-patent document 9] Camps, Nat Med 2005 11: pp.936-943 [Non-patent document 10] Campbell et al., Circ Res. 2005, 96, pp.197-206
[Non-patent document 11] Yadav M. et al., J Immunol. 2006, 176, pp.5494-503
[Non-patent document 12] Japanese Journal of Clinical Immunology, Vol.30, 2007, 5, pp.369-374
[Non-patent document 13] Ui MT et al., Trends Biochem. Sci., 1995, 20, pp.303-307
[Non-patent document 14] Grice C. A. et al., Bioorganic & Medicinal Chemistry Letters, 2006, 16, 8, pp.2209-2212
[Non-patent document 15] Hecheng Huaxue, 2003, 11, 6, pp.513-516
[Non-patent document 16] Chemical & Pharmaceutical Bulletin, 1999, 47, 7, pp.971-979

[Non-patent document 17] Shin D.-S. and Yoon Y.-J. et al., Journal of Organic Chemistry, 2003, 68, 20, pp.7918-7920
[Non-patent document 18] Enguang Feng et al., Journal of Organic Chemistry, 2009, 74, 7, pp.2846-2849
[Non-patent document 19] Benjamin Perry et al., Bioorganic & Medicinal Chemistry Letters, 2008, 18, pp.4700-4704

## SUMMARY OF THE INVENTION

### PROBLEMS TO BE SOLVED BY THE INVENTION

It is an object of the present invention to provide ring-fused morpholine derivatives or a pharmaceutically acceptable salt thereof which inhibit the activity of PI3K to regulate many biological processes including the growth, differentiation, survival, proliferation, migration, metabolism, and the like of cells, and are therefore useful for the prophylaxis/therapy of diseases including inflammatory diseases (allergic diseases (allergic dermatitis/allergic rhinitis, and the like), articular rheumatism, anaphylaxis, and the like), arteriosclerosis, vascular/circulatory diseases, cancer/tumors, immune system diseases, cell-proliferative diseases, infectious diseases, and the like.

### MEANS FOR SOLVING PROBLEM

Accordingly, for example, the present invention provides the following items:
(1) A compound represented by formula (1):
[Formula 1]

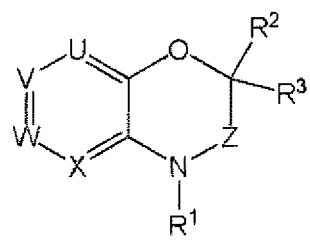

(I)

wherein
U and W are each independently =C(-R')- or =N-;
V and X are each independently =C(-R")- or =N-;
R' and R" are each independently hydrogen, halogen, cyano, nitro, hydroxy, carboxy, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted alkoxy, substituted or unsubstituted aryloxy, substituted or unsubstituted cycloalkyloxy, substituted or unsubstituted heteroaryloxy, substituted or unsubstituted heterocyclyloxy, substituted or unsubstituted acyl, substituted or unsubstituted alkoxycarbonyl, a group represented by the formula: $-SO-R^A$, a group represented by the formula: $-SO_2R^A$, or a group represented by the formula: $-SR^A$;
$R^A$ is hydrogen, substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
$R^1$ is substituted or unsubstituted alkyl, substituted or unsubstituted alkenyl, substituted or unsubstituted aryl, substituted or unsubstituted cycloalkyl, substituted or unsubstituted heteroaryl, substituted or unsubstituted heterocyclyl, a group represented by the formula: $-C(=O)-NR^BR^C$, or a group represented by the formula: $-C(=S)-NR^BR^C$;
$R^B$ and $R^C$ are each independently hydrogen, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted alkoxy, substituted or unsubstituted alkenyloxy, substituted or unsubstituted alkynyloxy, substituted or unsubstituted aryloxy, substituted or unsubstituted cycloalkyloxy, substituted or unsubstituted cycloalkenyloxy, substituted or unsubstituted heteroaryloxy, substituted or unsubstituted heterocyclyloxy, or substituted or unsubstituted amino, or
$R^B$ and $R^C$ may be taken together with the adjacent nitrogen atom to form substituted or unsubstituted nitrogenated heterocycle;

$R^2$ and $R^3$ are each independently hydrogen, hydroxy, halogen, cyano, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, or substituted or unsubstituted alkoxy, or

$R^2$ and $R^3$ may be taken together with the adjacent carbon atom to form substituted or unsubstituted saturated hydrocarbon ring or substituted or unsubstituted heterocycle, or

$R^2$ and $R^3$ may be taken together to form oxo;

Z is a single bond or $-(CR^4R^5)_n-$;

$R^4$ and $R^5$ are each independently hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino, or $R^4$ and $R^5$ may be taken together to form oxo (when a plurality of $R^4$ and a plurality of $R^5$ are present, $R^4$ and $R^5$ may be different from one another);

n is an integer from 1 to 2; and

provided that at least one of U, V, W, and X is =N-, and all four of them are not simultaneously =N-;

Z is not -C (=O) - when U and W are each =C (-R') -, V is =C (-R") -,

X is =N-, and $R^1$ is substituted or unsubstituted aryl, substituted or unsubstituted heterocyclyl, or unsubstituted cycloalkyl;

Z is not -C(=O)- when U is =N-, V and X are each =C(-R")-, and W is =C(-R')-; and

$R^1$ is not substituted or unsubstituted thiazolyl or fused thiazolyl when U is =N-; and

provided that the compounds shown below are excluded:

[Formula 2]

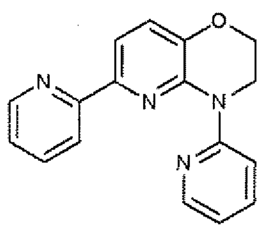

,

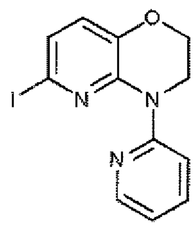

,

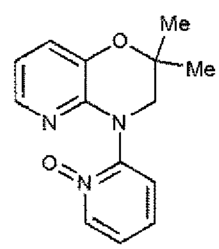

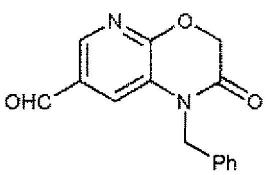

,

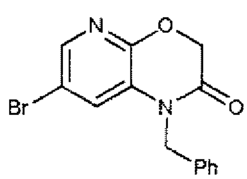

and

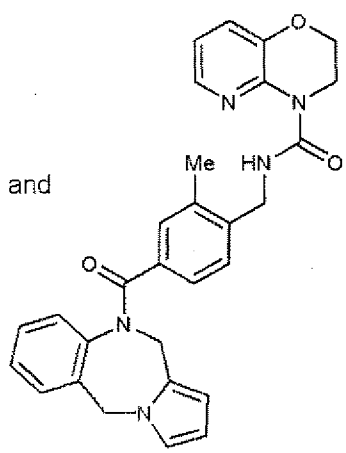

,

or a pharmaceutically acceptable salt thereof, or a solvate thereof.

(2) The compound according to the preceding item (1), wherein formula (I):

[Formula 3]

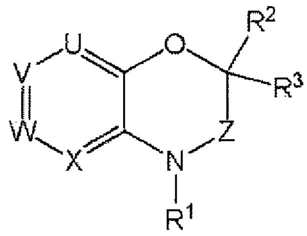

(I)

is selected from formula (II) or formula (III):

[Formula 4]

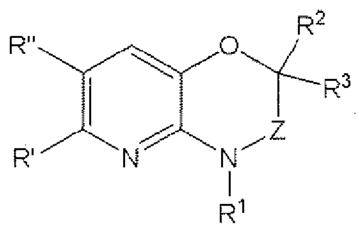

(II)

or

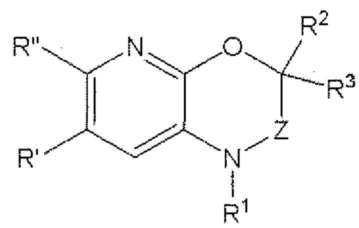

(III)

,

wherein R', R", $R^2$, and $R^3$ are defined as in item (1); $R^1$ is substituted or unsubstituted aryl, substituted or unsubstituted cycloalkyl, substituted or unsubstituted heteroaryl, substituted or unsubstituted heterocyclyl, a group represented by the formula: -C(=O)-$NR^BR^C$ wherein $R^B$ and $R^C$ are defined as in item (1), or a group represented by the formula: -C(=S)-$NR^BR^C$ wherein $R^B$ and $R^C$ are defined as in item (1); Z is $-(CR^4R^5)_n-$; $R^4$ and $R^5$ are defined as in item (1); and n is 1,

or a pharmaceutically acceptable salt thereof, or a solvate thereof.

(3) The compound according to the preceding item (1) or (2), wherein formula (I):

[Formula 5]

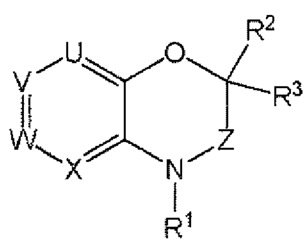

(I)

is formula (II):

[Formula 6]

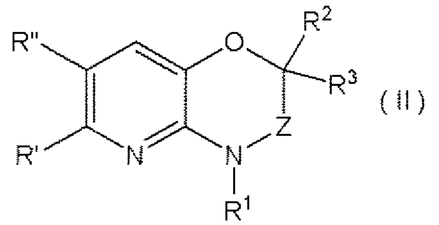

wherein R', R", $R^2$ and $R^3$ are defined as in item (1); $R^1$ is defined as in item (2) ; Z is $-(CR^4R^5)_n-$; $R^4$ and $R^5$ are defined as in item (1); and n is 1,
or a pharmaceutically acceptable salt thereof, or a solvate thereof.

(4) The compound according to the preceding item (1) or (2), wherein formula (I):

[Formula 7]

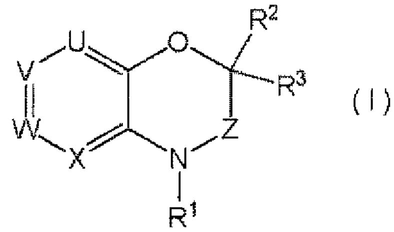

is formula (III):

[Formula 8]

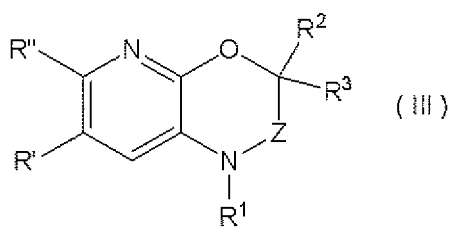

wherein R', R", $R^2$, and $R^3$ are defined as in item (1) ; $R^1$ is defined as in item (2) ; Z is $-(CR^4R^5)_n-$; $R^4$ and $R^5$ are defined as in item (1); and n is 1,
or a pharmaceutically acceptable salt thereof, or a solvate thereof.

(5) The compound according to any of the preceding items (1) to (4), wherein R' and R" are each independently hydrogen, halogen, nitro, carboxy, substituted or unsubstituted alkyl, substituted or unsubstituted alkenyl, substituted or unsubstitutedaryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted acyl, or substituted or unsubstituted alkoxycarbonyl,
or a pharmaceutically acceptable salt thereof, or a solvate thereof.

(6) The compound according to any of the preceding items (1) to (5), wherein R' is hydrogen, halogen, nitro, carboxy, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted acyl, or substituted or unsubstituted alkoxycarbonyl; and R" is hydrogen, halogen, substituted or unsubstituted alkyl, substituted or unsubstituted aryl, substituted or unsubstituted amino, or substituted or unsubstituted

carbamoyl,
or a pharmaceutically acceptable salt thereof, or a solvate thereof.

(7) The compound according to any of the preceding items (1) to (6), wherein $R^1$ is substituted or unsubstituted aryl, substituted or unsubstituted heteroaryl, substituted or unsubstituted heterocyclyl, or a group represented by the formula: -C(=O)-$NR^BR^C$ wherein $R^B$ and $R^C$ are defined as in item (1),
or a pharmaceutically acceptable salt thereof, or a solvate thereof.

(8) The compound according to any of the preceding items (1) to (7), wherein $R^1$ is a group represented by the formula: -C(=O)-$NR^BR^C$ wherein $R^B$ and $R^C$ are defined as in item (1) or a pharmaceutically acceptable salt thereof, or a solvate thereof.

(9) The compound according to any of the preceding items (1) to (8), wherein $R^B$ is hydrogen; and $R^C$ is substituted or unsubstituted alkyl, substituted or unsubstituted aryl, substituted or unsubstituted cycloalkyl, substituted or unsubstituted heteroaryl, or substituted or unsubstituted heterocyclyl,
or a pharmaceutically acceptable salt thereof, or a solvate thereof.

(10) The compound according to any of the preceding items (1) to (9), wherein $R^2$ and $R^3$ are each hydrogen, or a pharmaceutically acceptable salt thereof, or a solvate thereof.

(11) The compound according to any of the preceding items (1) to (10), wherein Z is $-(CR^4R^5)_n-$ wherein $R^4$, $R^5$, and n is defined as in item (1), or a pharmaceutically acceptable salt thereof, or a solvate thereof.

(12) The compound according to any of the preceding items (1) to (11), wherein $R^4$ and $R^5$ are each hydrogen; and n is 1, or a pharmaceutically acceptable salt thereof, or a solvate thereof.

(13) A pharmaceutical composition containing the compound according to any of the preceding items (1) to (12), or a pharmaceutically acceptable salt thereof, or a solvate thereof.

(14) The pharmaceutical composition according to the preceding item (13), which is a phosphatidylinositol-3-kinase inhibitor.

(15) The pharmaceutical composition according to any of the preceding items (13) and (14), which is a therapeutic agent and/or a prophylactic agent for inflammation.

(16) Use of the compound, or a pharmaceutically acceptable salt thereof, or a solvate thereof according to any of the preceding items (1) to (12) for producing a therapeutic agent and/or a prophylactic agent for inflammation.

(17) The compound, or a pharmaceutically acceptable salt thereof, or a solvate thereof according to any of the preceding items (1) to (12) for the therapy and/or prophylaxis of inflammation.

(18) A method for the therapy and/or prophylaxis of inflammation, characterized by administering the compound, or a pharmaceutically acceptable salt thereof, or a solvate thereof according to any of the preceding items (1) to (12).

(19) A method for the therapy and/or prophylaxis of inflammation, characterized by administering the pharmaceutical composition having PI3K inhibitory activity according to any of the preceding items (13) and (14).

(20) A phosphatidylinositol-3-kinase inhibitor containing the compound, or a pharmaceutically acceptable salt thereof, or a solvate thereof according to any of the preceding items (1) to (12) as an active ingredient.

(21) The inhibitor according to the preceding item (20), which is specific to one or more types of $\alpha$, $\beta$, $\gamma$, and $\delta$ phosphatidylinositol-3-kinase inhibitors.

(22) The pharmaceutical composition according to any of the precedingitems (13) to (15) for the treatment of the following phosphatidylinositol-3-kinase dependent diseases: encephalitis, myelitis and encephalomyelitis, meningitis, inflammatory polyneuropathy, neuritis, dacryoadenitis, orbital inflammation, conjunctivitis (allergic conjunctivitis, vernal keratocon junctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpits, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumocaniosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alvealitis, chronic hypersensitivity pneumonitis, multiple sclerosis, primary biliary cirrhosis, uveitis, keratoconjunctivitis sicca, interstitial

pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like; or burn, traumatic inflammation, and the like.

(23) A phosphatidylinositol-3-kinase inhibitor containing the compound, or a pharmaceutically acceptable salt thereof, or a solvate thereof according to any of the preceding items (1) to (12).

(24) A protein kinase B (AKT) inhibitor containing the compound, or a pharmaceutically acceptable salt thereof, or a solvate thereof according to any of the preceding items (1) to (12).

(25) An anticancer agent containing the compound, or a pharmaceutically acceptable salt thereof, or a solvate thereof according to any of the preceding items (1) to (12).

(26) An anti-inflammatory or a therapeutic agent for inflammatory diseases (such as pancreatitis, pneumonia, airway inflammation, COPD (such as pulmonary emphysema, chronic bronchitis, and the like), arthritis, glomerulonephritis, and the like) wherein the anti-inflammatory or the therapeutic agent contains the compound, or a pharmaceutically acceptable salt thereof, or a solvate thereof according to any of the preceding items (1) to (12).

(27) An antiallergic agent (asthma, atopic dermatitis, allergic rhinitis, and the like) containing the compound, or a pharmaceutically acceptable salt thereof, or a solvate thereof according to any of the preceding items (1) to (12).

(28) A therapeutic agent for immune system diseases wherein the therapeutic agent contains the compound, or a pharmaceutically acceptable salt thereof, or a solvate thereof according to any of the preceding items (1) to (12) .

(29) An immunosuppressant containing the compound, or a pharmaceutically acceptable salt thereof, or a solvate thereof according to any of the preceding items (1) to (12).

(30) A therapeutic agent for autoimmune diseases wherein the therapeutic agent contains the compound, or a pharmaceutically acceptable salt thereof, or a solvate thereof according to any of the preceding items (1) to (12).

(31) An anti-circulatory-disease agent (such as antihypertensive agent and the like) wherein the agent contains the compound, or a pharmaceutically acceptable salt thereof, or a solvate thereof according to any of the preceding items (1) to (12).

(32) An antiinfectant containing the compound, or a pharmaceutically acceptable salt thereof, or a solvate thereof according to any of the preceding items (1) to (12).

(33) A wound-healing agent containing the compound, or a pharmaceutically acceptable salt thereof, or a solvate thereof according to any of the preceding items (1) to (12).

(34) A method, a system, an apparatus, a kit, and the like for producing the compound, or a pharmaceutically acceptable salt thereof, or a solvate thereof according to any of the preceding items (1) to (12).

(35) Method, a system, an apparatus, a kit, and the like for preparing a pharmaceutical composition containing the compound, or a pharmaceutically acceptable salt thereof, or a solvate thereof according to any of the preceding items (1) to (12).

(36) A method, a system, an apparatus, a kit, and the like using the compound, or a pharmaceutically acceptable salt thereof, or a solvate thereof according to any of the preceding items (1) to (12).

In one aspect, the present invention relates to a compound represented by formula (I):

[Formula 9]

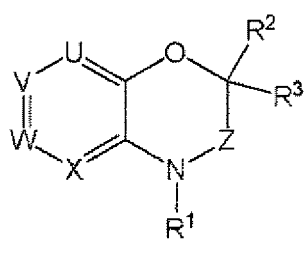

(I)

wherein

U and W are each independently =C(-R')- or =N-;

V and X are each independently =C(-R")- or -N-;

R' and R" are each independently hydrogen, halogen, cyano, nitro, hydroxy, carboxy, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted

or unsubstituted heterocyclyl, substituted or unsubstituted amino, substituted or unsubstituted carbamoyl, substituted or unsubstituted alkoxy, substituted or unsubstituted aryloxy, substituted or unsubstituted cycloalkyloxy, substituted or unsubstituted heteroaryloxy, substituted or unsubstituted heterocyclyloxy, substituted or unsubstituted acyl, substituted or unsubstituted alkoxycarbonyl, $-SO-R^A$, $-SO_2R^A$ or $-SR^A$;

$R^A$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;

$R^1$ is substituted or unsubstituted alkyl, substituted or unsubstituted alkenyl, substituted or unsubstituted aryl, substituted or unsubstituted cycloalkyl, substituted or unsubstituted heteroaryl, substituted or unsubstituted heterocyclyl, a group represented by the formula: $-C(=O)-NR^BR^C$, or a group represented by the formula: $-C(=S)-NR^BR^C$;

$R^B$ and $R^C$ are each independently hydrogen, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted alkoxy, substituted or unsubstituted alkenyloxy, substituted or unsubstituted alkynyloxy, substituted or unsubstituted aryloxy, substituted or unsubstituted cycloalkyloxy, substituted or unsubstituted cycloalkenyloxy, substituted or unsubstituted heteroaryloxy, substituted or unsubstituted heterocyclyloxy, or substituted or unsubstituted amino, or

$R^B$ and $R^C$ may be taken together with the adjacent nitrogen atom to form substituted or unsubstituted nitrogenated heterocycle;

$R^2$ and $R^3$ are each independently hydrogen, hydroxy, halogen, cyano, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, or substituted or unsubstituted alkoxy, or

$R^2$ and $R^3$ may be taken together with the adjacent carbon atom to form substituted or unsubstituted saturated hydrocarbon ring or substituted or unsubstituted heterocycle, or $R^2$ and $R^3$ may be taken together to form oxo;

Z is a single bond or $-(CR^4R^5)_n-$;

$R^4$ and $R^5$ are each independently hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino, or

$R^4$ and $R^5$ may be taken together to form oxo (when a plurality of $R^4$ and a plurality of $R^5$ are present, $R^4$ and $R^5$ may be different from one another);

n is an integer from 1 to 2; and

provided that at least one of U, V, W, and X is =N-, and all four of them are not simultaneously =N-;

Z is not -C(=O)- when U and W are each =C(-R')-, V is =C(-R")-, X is =N-, and $R^1$ is substituted or unsubstituted aryl, substituted or unsubstituted heterocyclyl, or unsubstituted cycloalkyl; and

Z is not -C(=O)- when U is =N-, V and X are each =C(-R")-, and W is =C(-R')-; and

provided that the compounds shown below are excluded:

[Formula 10]

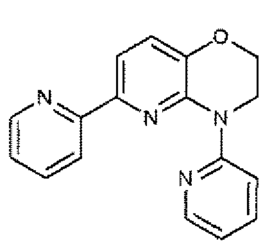,
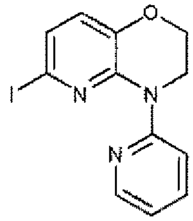,
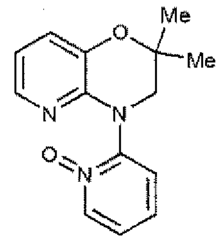

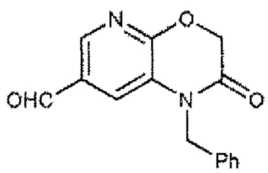,
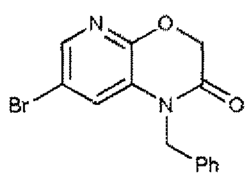
and
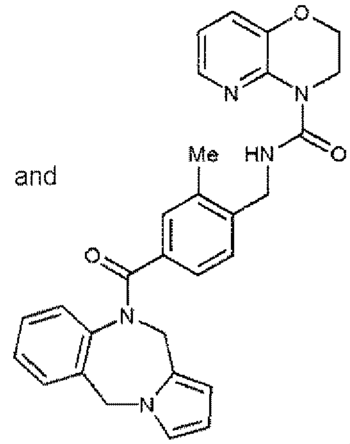,

or a pharmaceutically acceptable salt thereof, or a solvate thereof.

In one embodiment, the formula (I):

[Formula 11]

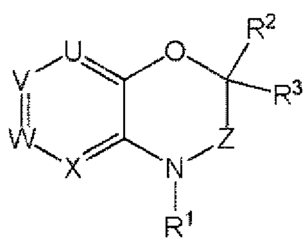
(I)

is selected from formula (II) or formula (III):

[Formula 12]

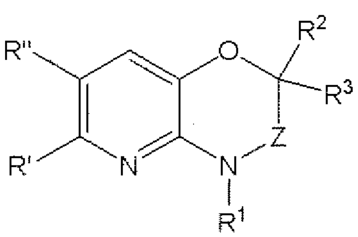
(II)

or

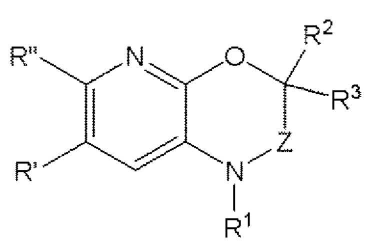
(III)

wherein each symbol is defined as above, and $R^1$ is substituted or unsubstituted aryl, substituted or unsubstituted cycloalkyl, substituted or unsubstituted heteroaryl, substituted or unsubstituted heterocyclyl, a group represented by the formula: -C(=O)-$NR^BR^C$ wherein $R^B$ and $R^C$ is defined as above, or a group represented by the formula: -C(=S)-$NR^BR^C$ wherein $R^B$ and $R^C$ is defined as above.

In one embodiment, the formula (I):

[Formula 13]

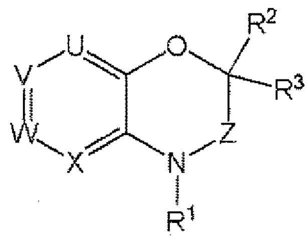

(I)

is formula (II):

[Formula 14]

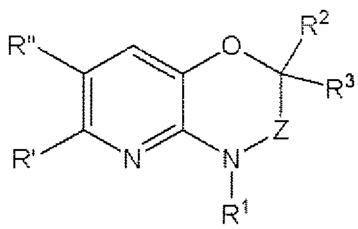

(II)

wherein each symbol is defined as above.

In one embodiment, the formula (I):

[Formula 15]

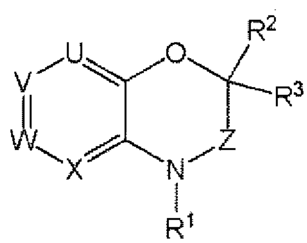

(I)

is formula (III):

[Formula 16]

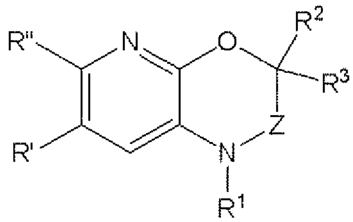

(III)

wherein each symbol is defined as above.

In one embodiment, R' and R" are each independently hydrogen, halogen, nitro, carboxy, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted acyl, or substituted or unsubstituted alkoxycarbonyl.

In one embodiment, R' is hydrogen, halogen, nitro, carboxy, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstitutedcarbamoyl, substituted or unsubstituted acyl, or substituted or unsubstituted alkoxycarbonyl; and R" is hydrogen, halogen, substituted or unsubstituted alkyl, substituted or unsubstituted aryl, substituted or unsubstituted amino, or substituted or unsubstituted carbamoyl.

In one embodiment, $R^1$ is substituted or unsubstituted aryl, substituted or unsubstituted heteroaryl, substituted or unsubstituted heterocyclyl, or a group represented by the formula: $-C(=O)-NR^BR^C$ wherein $R^B$ and $R^C$ are defined as above.

In one embodiment, $R^1$ is a group represented by the formula: $-C(=O)-NR^BR^C$ wherein $R^B$ and $R^C$ are defined as above.

In one embodiment, $R^B$ is hydrogen, and $R^C$ is substituted or unsubstituted alkyl, substituted, or unsubstituted aryl, substituted or unsubstituted cycloalkyl, substituted or unsubstituted heteroaryl, or substituted or unsubstituted heterocyclyl.

In one embodiment, $R^2$ and $R^3$ are each hydrogen.

In one embodiment, Z is $-(CR^4R^5)_n-$.

In one embodiment, $R^4$ and $R^5$ are each hydrogen, and n is 1.

The present invention includes compounds shown by combining a part or all of the above-described embodiments.

In one embodiment, the present invention relates to a pharmaceutical composition containing the compound, or a pharmaceutically acceptable salt thereof, or a solvate thereof.

In one embodiment, the pharmaceutical composition is a phosphatidylinositol-3-kinase inhibitor.

In another aspect, the present invention relates to a phosphatidylinositol-3-kinase inhibitor containing the compound of the present invention, or a pharmaceutically acceptable salt thereof, or a solvate thereof as an active ingredient.

In one embodiment, the pharmaceutical composition is a therapeutic agent and/or a prophylactic agent for inflammation.

In another aspect, the present invention relates to use of the above-described compound, or a pharmaceutically acceptable salt thereof, or a solvate thereof for producing a therapeutic agent and/or a prophylactic agent for inflammation.

In one embodiment, the present invention relates to the above-described compound, or a pharmaceutically acceptable salt thereof, or a solvate thereof for the therapy and/or prophylaxis of inflammation.

In another aspect, the present invention relates to a method for the therapy and/or prophylaxis of inflammation, the method being characterized by administering the above-described compound, or a pharmaceutically acceptable salt thereof, or a solvate thereof.

In another aspect, the present invention relates to a phosphatidylinositol-3-kinase inhibitor containing the compound of the invention, or a pharmaceutically acceptable salt thereof, or a solvate thereof as an active ingredient.

In one embodiment, the inhibitor of the present invention may be specific to one or more types of $\alpha$, $\beta$, $\gamma$, and $\delta$ phosphatidylinositol-3-kinase inhibitors.

In pharmaceutical aspect, in a preferred embodiment, the pharmaceutical composition of the present invention may be a composition for the treatment of phosphatidylinositol-3-kinase dependent diseases. Such phosphatidylinositol-3-kinase dependent diseases can include: diseases such as encephalitis, myelitis and encephalomyelitis, meningitis,

inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary disease, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like; and burn, traumatic inflammation, and the like.

In an embodiment, the present invention relates to a phosphatidylinositol-3-kinase inhibitor containing the compound of the invention, or a pharmaceutically acceptable salt thereof, or a solvate thereof.

In an embodiment, the present invention relates to a protein kinase B (AKT) inhibitor containing the compound of the invention, or a pharmaceutically acceptable salt thereof, or a solvate thereof.

In an embodiment, the present invention relates to an anticancer agent containing the compound of the invention, or a pharmaceutically acceptable salt thereof, or a solvate thereof.

In an embodiment, the present invention relates to an anti-inflammatory or a therapeutic agent for inflammatory diseases (such as pancreatitis, pneumonia, airway inflammation, COPD (such as pulmonary emphysema, chronic bronchitis, and the like), arthritis, glomerulonephritis, and the like) wherein the anti-inflammatory or the therapeutic agent contains the compound of the invention, or a pharmaceutically acceptable salt thereof, or a solvate thereof.

In an embodiment, the present invention relates to an antiallergic agent (asthma, atopic dermatitis, allergic rhinitis, and the like) containing the compound of the invention, or a pharmaceutically acceptable salt thereof, or a solvate thereof.

In an embodiment, the present invention relates to a therapeutic agent for immune system diseases wherein the therapeutic agent contains the compound of the invention, or a pharmaceutically acceptable salt thereof, or a solvate thereof.

In an embodiment, the present invention relates to an immunosuppressant containing the compound of the invention, or a pharmaceutically acceptable salt thereof, or a solvate thereof.

In an embodiment, the present invention relates to a therapeutic agent for autoimmune diseases wherein the therapeutic agent contains the compound of the invention, or a pharmaceutically acceptable salt thereof, or a solvate thereof.

In an embodiment, the present invention relates to an anti-circulatory-disease agent (such as antihypertensive agent and the like) wherein the agent contains the compound of the invention, or a pharmaceutically acceptable salt thereof, or a solvate thereof.

In an embodiment, the present invention relates to an antiinfectant containing the compound of the invention, or a pharmaceutically acceptable salt thereof, or a solvate thereof.

In an embodiment, the present invention relates to a wound-healing agent containing the compound of the invention, or a pharmaceutically acceptable salt thereof, or a solvate thereof.

The present invention also relates to a method, a system, an apparatus, a kit, and the like for producing the compound of the invention, or a pharmaceutically acceptable salt thereof, or a solvate thereof.

The present invention also relates to a method, a system, an apparatus, a kit, and the like for preparing a pharmaceutical composition containing the compound of the invention, or a pharmaceutically acceptable salt thereof, or a solvate thereof.

The present invention also relates to a method, a system, an apparatus, a kit, and the like using the compound of the invention, or a pharmaceutically acceptable salt thereof, or a solvate thereof.
Thus, these and other advantages of the present invention are apparent when the following detailed description is read.

EFFECT OF THE INVENTION

The present invention provides a medicament for the treatment of phosphatidylinositol-3-kinase dependent diseases; a compound used therefor; or a pharmaceutically acceptable salt thereof; or a solvate thereof. The compound of the present invention exhibits excellent PI3K inhibitory activity as described in Examples below. Furthermore, the compound of the present invention encompasses compounds exhibiting PI3Kα and γ inhibitory activity. Accordingly, the pharmaceutical composition of the present invention may be used for the prophylaxis and/or as a therapeutic agent for diseases such as encephalitis, myelitis and encephalomyelitis, meningitis, inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like, or used as a therapeutic agent for burn or traumatic inflammation.
The compound of the present invention is a compound having utility as a medicament. Here, utility as a medicament includes the following points: the compound has good metabolic stability; the induction of a drug-metabolizing enzyme is low; the inhibition of a drug-metabolizing enzyme which metabolizes another drug is also low; the compound has high oral absorbency; the drug has high solubility; the clearance is low; the half-life is sufficiently long to express the efficacy; or the like.

MODE FOR CARRYING OUT THE INVENTION

Hereinafter, the present invention is described with reference to embodiments. It should be understood that throughout the present specification, the expression of a singular form includes the concept of its plural form unless specified otherwise. Accordingly, it should be understood that an article in singular form (for example, in the English language, "a," "an," "the," and the like) includes the concept of its plural form unless specified otherwise. Furthermore, it should be understood that the terms used herein are used in a meaning normally used in the art unless specified otherwise. Thus, unless defined otherwise, all technical and scientific terms used herein have the same meaning as those generally understood by those skilled in the art in the field to which the present invention pertains. If there is a contradiction, the present specification (including definitions) precedes.
Each meaning of terms used herein is described below. In the present specification, each term is used in an unequivocal meaning. Both when used alone and in combination with another word, each term is used in the same meaning.
As used herein, the term "halogen" means fluorine, chlorine, bromine, and iodine. Examples thereof include

fluorine, chlorine, and bromine.

As used herein, the term "alkyl" encompasses a linear or branched monovalent hydrocarbon group having 1 to 8 carbon atoms. Examples thereof include methyl, ethyl, *n*-propyl, isopropyl, *n*-butyl, isobutyl, *sec*-butyl, *tert*-butyl, *n*-pentyl, isopentyl, *neo*-pentyl, *n*-hexyl, isohexyl, *n*-heptyl, *n*-octyl, and the like. An example is C1-C6 alkyl. Another example is C1-C4 alkyl. When the carbon number is specified in particular, an "alkyl" having carbon in a range of the number is meant.

As used herein, the term "alkenyl" encompasses a linear or branched monovalent hydrocarbon group having 2 to 8 carbon atoms and one or more double bonds. Examples thereof include vinyl, allyl, 1-propenyl, 2-butenyl, 2-pentenyl, 2-hexenyl, 2-heptenyl, 2-octenyl, and the like. An example is C2-CC alkenyl. Another example is C2-C4 alkenyl.

As used herein, the term "alkynyl" encompasses a linear or branched monovalent hydrocarbon group having 2 to 8 carbon atoms and one or more triple bonds. Examples thereof include ethynyl, 1-propynyl, 2-propynyl, 2-butynyl, 2-pentynyl, 2-hexynyl, 2-heptynyl, 2-octynyl, and the like. An example is C2-C6 alkynyl. Another example is C2-C4 alkynyl.

As used herein, the term "cycloalkyl" encompasses cycloalkyl having 3 to 8 carbon atoms. Examples thereof include cyclopropyl, cyclobutyl, cyclopentyl, cyclohexyl, cycloheptyl, and cyclooctyl. An example is C3-C6 cycloalkyl.

As used herein, the term "cycloalkenyl" encompasses cycloalkenyl having 3 to 8 carbon atoms. Examples thereof include cyclopropenyl, cyclobutenyl, cyclopentenyl, cyclohexenyl, and cycloheptenyl. An example is C3-C6 cycloalkenyl.

As used herein, the term "alkoxy" includes methoxy, ethoxy, *n*-propoxy, isopropoxy, *n*-butoxy, isobutoxy, *sec*-butoxy, *tert*-butoxy, *n*-pentyloxy, isopentyloxy, 2-pentyloxy, 3-pentyloxy, *n*-hexyloxy, isohexyloxy, 2-hexyloxy, 3-hexyloxy, *n*-heptyloxy, *n*-octyloxy, and the like. An example is C1-C6 alkoxy. Another example is C1-C4 alkoxy. When the carbon number is specified in particular, an "alkoxy" having carbon in a range of the number is meant.

As used herein, the term "alkylthio" includes methylthio, ethylthio, *n*-propylthio, isopropylthio, *n*-butylthio, isobutylthio, *sec*-butylthio, *tert*-butylthio, *n*-pentylthio, isopentylthio, 2-pentylthio, 3-pentylthio, *n*-hexylthio, isohexylthio, 2-hexylthio, 3-hexylthio, *n*-heptylthio, *n*-octylthio, and the like. An example is C1-C6 alkylthio. Another example is C1-C4 alkylthio. When the carbon number is specified in particular, an "alkylthio" having carbon in a range of the number is meant.

As used herein, the term "alkylsulfonyl" includes methylsulfonyl, ethylsulfonyl, *n*-propylsulfonyl, isopropylsulfonyl, *n*-butylsulfonyl, isobutylsulfonyl, *sec*-butylsulfonyl, *tert*-butylsulfonyl, *n*-pentylsulfonyl, isopentylsulfonyl, 2-pentylsulfonyl, 3-pentylsulfonyl, *n*-hexylsulfonyl, isohexylsulfonyl, 2-hexylsulfonyl, 3-hexylsulfonyl, *n*-heptylsulfonyl, *n*-octylsulfonyl, and the like. An example is C1-C6 alkylsulfonyl. Another example is C1-C4 alkylsulfonyl.

As used herein, the term "alkoxycarbonyl" includes methoxycarbonyl, ethoxycarbonyl, *n*-propoxycarbonyl, isopropoxycarbonyl, *n*-butoxycarbonyl, isobutoxycarbonyl, *sec*-butoxycarbonyl, *tert*-butoxycarbonyl, *n*-pentyloxycarbonyl, and the like. An example is C1-C4 alkyloxycarbonyl. Particularly, another example is C1-C2 alkyloxycarbonyl.

As used herein, the term "acryl" encompasses formyl, alkylcarbonyl, alkenylcarbonyl, alkynylcarbonyl, cycloalkylcarbonyl, cycloalkenylcarbonyl, arylcarbonyl, heteroarylcarbonyl, heterocyclylcarbonyl. Examples thereof include acetyl, propionyl, butyloyl, benzoyl, and the like.

As used herein, the term "substituted or unsubstituted amino" encompasses amino that may be substituted with the aforementioned "alkyl," the below-mentioned "aryl," the below-mentioned "heteroaryl," the bellow-mentioned "heterocyclyl," the aforementioned "acyl, " the aforementioned "alkoxycarbonyl," the aforementioned "alkylsulfonyl," the below-mentioned "arylsulfonyl," the below-mentioned "heteroarylsulfonyl," and/or the below-mentioned "heterocyclylsulfonyl" at 1 or 2 positions. Examples thereof include amino, methylamino, dimethylamino, ethylamino, diethylamino, ethylmethylamino, benzylamino, acetylamino, benzoylamino, methyloxycarbonylamino, methylsulfonylamino, and the like. Examples thereof include amino, methylamino, dimethylamino, ethylmethylamino, diethylamino, acetylamino, methylsulfonylamino, and the like.

As used herein, the term "substituted or unsubstituted carbamoyl" encompasses substituted or unsubstituted aminocarbonyl in which the substituted or unsubstituted amino portion is the aforementioned "substituted or unsubstituted amino." Examples thereof include carbamoyl, N-methylcarbamoyl, N,N-dimethylcarbamoyl, N-ethyl-N-methylcarbamoyl, N,N-diethylcarbamoyl, N-benzylcarbamoyl, N-acetylcarbamoyl, N-methylsul-fonylcarbamoyl, and the like. Examples thereof include carbamoyl, N-methylcarbamoyl, N,N-dimethylcarbamoyl, N-methylsulfonylcarbamoyl, and the like.

As used herein, the term "aryl" encompasses monocyclic or fused-cyclic aromatic hydrocarbon, which may be fused with the aforementioned "cycloalkyl" at any possible position. Both in the cases where aryl is monocyclic and fused-cyclic, it may be bound at any possible position. Examples thereof include phenyl, 1-naphthyl, 2-naphthyl, anthryl, tetrahydronaphthyl, and the like. Examples thereof include phenyl, 1-naphthyl, and 2-naphthyl. An example is phenyl.

As used herein, the term "heteroaryl" encompasses a 5 to 6-membered aromatic ring containing one or more optionally-selected oxygen atoms, sulfur atoms, or nitrogen atoms in the ring. This may be fused with the aforementioned "cycloalkyl," the aforementioned "aryl," the below-mentioned "heterocyclyl", or another heteroaryl at any possible position. Both in the cases that heteroaryl is monocyclic and fused-cyclic, it may be bound at any possible position. Examples thereof include pyrrolyl (e.g., 1-pyrrolyl, 2-pyrrolyl, 3-pyrrolyl), furyl (e.g., 2-furyl, 3-furyl), thienyl (e.g., 2-thienyl, 3-thienyl), imidazolyl (e.g., 2-imidazolyl, 4-imidazolyl), pyrazolyl (e.g., 1-pyrazolyl, 3-pyrazolyl, 4-pyrazolyl), isothiazolyl (e.g., 3-isothiazolyl), isoxazolyl (e.g., 3-isoxazolyl), oxazolyl (e.g., 2-oxazolyl, 4-oxazolyl, 5-oxazolyl), thiazolyl (e.g., 2-thiazolyl, 4-thiazolyl, 5-thiazolyl), pyridyl (e.g., 2-pyridyl, 3-pyridyl, 4-pyridyl), pyrazinyl (e.g., 2-pyrazinyl), pyrimidinyl (e.g., 2-pyrimidinyl, 4-pyrimidinyl), pyridazinyl (e.g., 3-pyridazinyl), tetrazolyl (e.g., 1H-tetrazolyl), oxadiazolyl (e.g., 1,3,4-oxadiazolyl), thiadiazolyl (e.g., 1,3,4-thiadiazolyl), indolizinyl (e.g., 2-indolizinyl, 6-indolizinyl), isoindolyl (e.g., 2-isoindolyl), indolyl (e.g., 1-indolyl, 2-indolyl, 3-indolyl), indazolyl (e.g., 3-indazolyl), purinyl (e.g., 8-purinyl), quinolizinyl (e.g., 2-quinolizinyl), isoquinolyl (e.g., 3-isoquinolyl), quinolyl (e.g., 2-quinolyl, 5-quinolyl), phthalazinyl (e.g., 1-phthalazinyl), naphthyridinyl (e.g., 2-naphthyridinyl), quinazolinyl (e.g., 2-quinazolinyl), cinnolinyl (e.g., 3-cinnolinyl), pteridinyl (e.g., 2-pteridinyl), carbazolyl (e.g., 2-carbazolyl, 4-carbazolyl), phenanthridinyl (e.g., 2-phenanthridinyl, 3-phenanthridinyl), acridinyl (e.g., 1-acridinyl, 2-acridinyl), dibenzofuranyl (e.g., 1-dibenzofuranyl, 2-dibenzofuranyl), benzimidazolyl (e.g., 2-benzimidazolyl), benzisoxazolyl (e.g., 3-benzisoxazolyl), benzoxazolyl (e.g., 2-benzoxazolyl), benzoxadiazolyl (e.g., 4-benzoxadiazolyl), benzisothiazolyl (e.g., 3-benzisothiazolyl), benzothiazolyl (e.g., 2-benzothiazolyl), benzofuryl (e.g., 3-benzofuryl), benzothienyl (e.g., 2-benzothienyl), dibenzothienyl (e.g., 2-dibenzothienyl), benzodioxolyl (e.g., 1,3-benzodioxolyl), and the like.

As used herein, the term "heterocyclyl" encompasses a non-aromatic heterocyclic group that may have 1 to 4 oxygen, sulfur, and/or nitrogen atoms in the ring and may be substituted at any possible position. Additionally, such a non-aromatic heterocyclic group may be further crosslinked via C1-C4 alkyl chain, or may be fused with cycloalkane (a 5- or 6-membered ring is preferable) or a benzene ring. The heterocyclic group may be saturated or unsaturated as long as it is non-aromatic. Examples thereof are 5- to 8- membered rings. Examples thereof include pyrrolinyl (e.g., 1-pyrrolinyl, 2-pyrrolinyl, 3-pyrrolinyl), pyrrolidinyl (e.g., 1-pyrrolidinyl, 2-pyrrolidinyl, 3-pyrrolidinyl), pyrrolidinone, imidazolinyl (e.g., 1-imidazolinyl, 2-imidazolinyl, 4-imidazolinyl), imidazolidinyl (e.g., 1-imidazolidinyl, 2-imidazolidinyl, 4-imidazolidinyl), imidazolidinone, pyrazolinyl (e.g., 1-pyrazolinyl, 3-pyrazolinyl, 4-pyrazolinyl, 1-pyrazolidinyl, 3-pyrazolidinyl, 4-pyrazolidinyl), piperidinone, piperidino, piperidinyl (e.g., 2-piperidinyl, 3-piperidinyl, 4-piperidinyl), piperazinyl (e.g., 1-piperazinyl, 2-piperazinyl.), piperazinone, morpholinyl (e.g., 2-morpholinyl, 3-morpholinyl), morpholino, tetrahydropyranyl, tetrahydrofuranyl, and the like.

In the present specification, a "substituted or unsubstituted nitrogenated heterocycle formed after $R^B$ and $R^C$ are taken together with the adjacent nitrogen atom" encompasses a ring that has at least one N in the ring and may further have O, S, and/or N. The ring encompasses a monocycle and a fused ring, and may be an aromatic heterocycle or non-aromatic heterocycle. Examples thereof include the following:

[Formula 17]

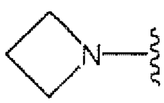 , 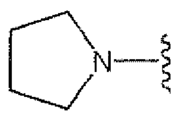 , 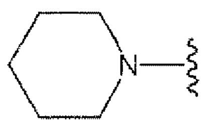 , 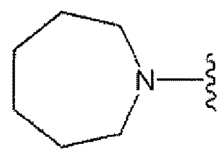 ,

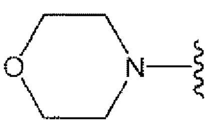 , 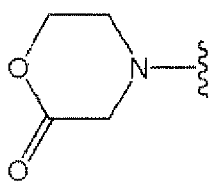 , 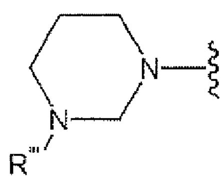 , 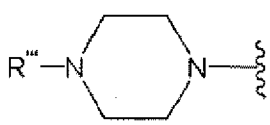 ,

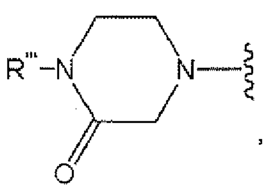 , 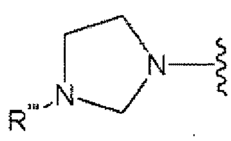 , 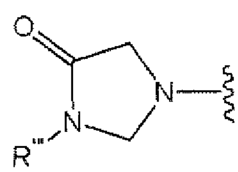 , 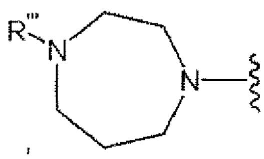 ,

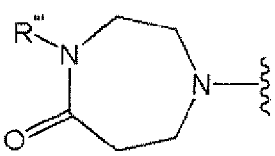

wherein R''' is, for example, hydrogen, substituted or unsubstituted alkyl, substituted or unsubstituted acyl,

substituted or unsubstituted amino, hydroxy, or the like.

In the present specification, a "substituted or unsubstituted saturated hydrocarbon formed after $R^2$ and $R^3$ are taken together with the adjacent carbon atom" encompasses a monocycle or a fused ring. Examples thereof include the following:

[Formula 18]

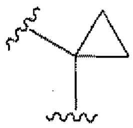 , 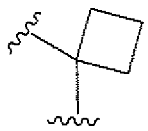 , 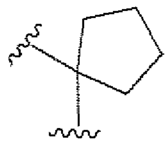 , 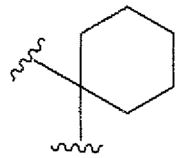 .

In the present specification, a "substituted or unsubstituted heterocycle formed after $R^2$ and $R^3$ are taken together with the adjacent carbon atom" encompasses a ring that has at least one N, O, or S in the ring. The ring encompasses a monocycle or a fused ring, and may be an aromatic heterocycle or non-aromatic heterocycle. Examples thereof include the following:

[Formula 19]

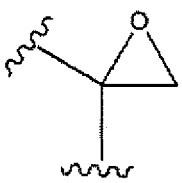 , 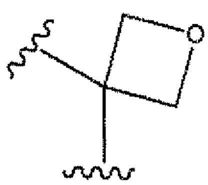 , 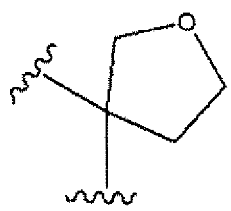 , 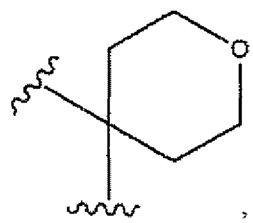 ,

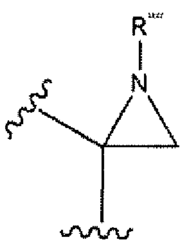 , 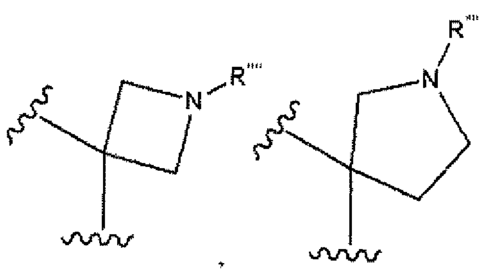 , , 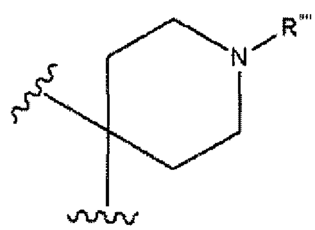

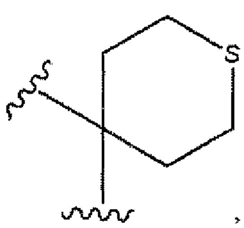 , 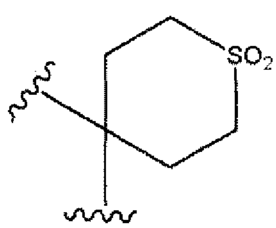

wherein R'''' is, for example, hydrogen, substituted or unsubstituted alkyl, substituted or unsubstituted acyl, substituted or unsubstituted amino, hydroxy, or the like.

In the present specification, an "oxo formed after $R^2$ and $R^3$ are taken together" means the following substituents.

[Formula 20]

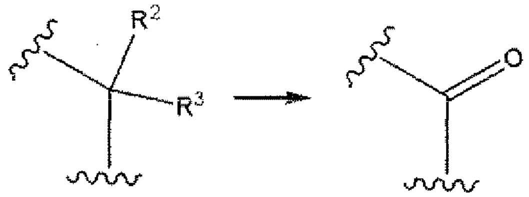

In the present specification, an "oxo formed after $R^4$ and $R^5$ are taken together" means the following substituent.
[Formula 21]

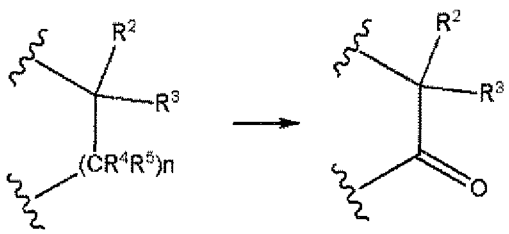

In the present specification, when Z is $-(CR^4R^5)_n-$ and n is 2, Z encompasses the following substituents.
[Formula 22]

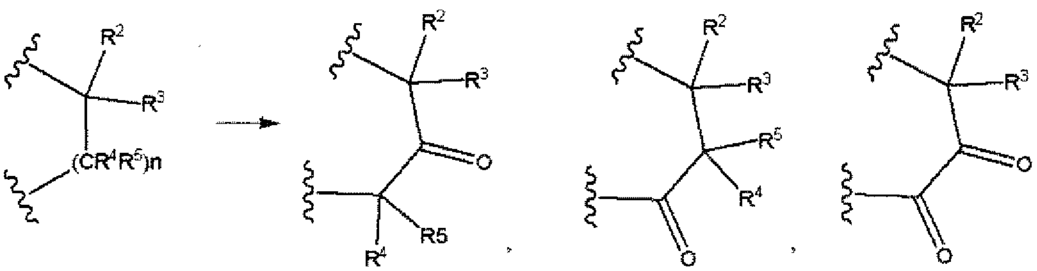

As used herein, the alkyl portion of "alkoxy," "alkylsulfonyl," "alkoxycarbonyl," "alkylthio," and "alkylcarbonyl" means the aforementioned "alkyl."
As used herein, the alkenyl portion of "alkenyloxy" and "alkenylcarbonyl" means the aforementioned "alkenyl."
As used herein, the alkynyl portion of "alkynyloxy" and "alkynylcarbonyl" means the aforementioned "alkynyl."
As used herein, the cycloalkyl portion of "cycloalkyloxy," "cycloalkylsulfonyl," and "cycloalkylcarbonyl" means the aforementioned "cycloalkyl."
As used herein, the cycloalkenyl portion of "cycloalkenyloxy" and "cycloalkenylcarbonyl" means the aforementioned "cycloalkenyl."
As used herein, the aryl portion of "aryloxy," "arylsulfonyl," "aryloxycarbonyl," and "arylcarbonyl" means the aforementioned "aryl."
As used herein, the heteroaryl portion of "heteroaryloxy," "heteroarylsulfonyl," "heteroarylcarbonyl," and "heteroaryloxycarbonyl" means the aforementioned "heteroaryl."
As used herein, the heterocyclyl portion of "heterooyclyloxy," "heterocyclylsulfonyl," "heterocyclylcarbonyl," and "heterocyclyloxycarbonyl" means the aforementioned "heterocyclyl."
As used herein, the term "fused thiazolyl" means a group in which the thiazolyl group is fused with a substituted or unsubstituted aryl, a substituted or unsubstituted cycloalkyl, a substituted or unsubstituted cycloalkenyl, a substituted

or unsubstituted heteroaryl, and/or a substituted or unsubstituted heterocyclyl. Examples thereof include the following groups:

[Formula 23]

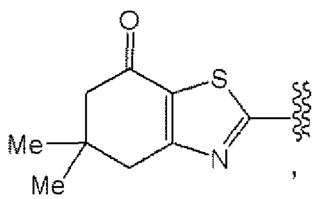, 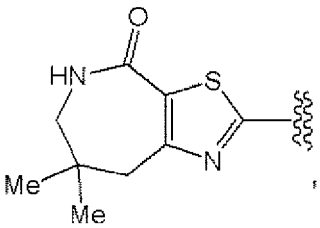, and 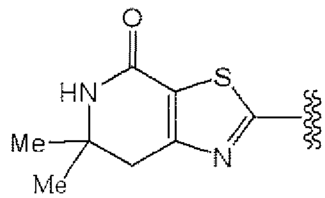.

As used herein, substituents of "substituted or unsubstituted alkyl," "substituted or unsubstituted alkenyl," "substituted or unsubstituted alkynyl," "substituted or unsubstituted aryl," "substituted or unsubstituted cycloalkyl," "substituted or unsubstituted cycloalkenyl," "substituted or unsubstituted heteroaryl," "substituted or unsubstituted heterocyclyl," "substituted or unsubstituted acyl," "substituted or unsubstituted alkoxy," "substituted or unsubstituted aryloxy," "substituted or unsubstituted alkenyloxy, " "substituted or unsubstituted alkynyloxy," "substituted or unsubstituted cycloalkyloxy," "substituted or unsubstituted cycloalkenyloxy," "substituted or unsubstituted heteroaryloxy," "substituted or unsubstituted heterocyclyloxy," "substituted or unsubstituted nitrogenated heterocycle formed after $R^B$ and $R^C$ are taken together with the adjacent nitrogen atom," "substituted or unsubstituted alkoxycarbonyl," "substituted or unsubstituted saturated hydrocarbon formed after $R^2$ and $R^3$ are taken together with the adjacent carbon atom," and "substituted or unsubstituted heterocycle formed after $R^2$ and $R^3$ are taken together with the adjacent carbon atoms" are selected from the group consisting of, for examples, hydroxy, carboxy, halogen, halogenated alkyl (e.g., $CF_3$, $CH_2CF_3$, $CH_2CCl_3$), nitro, nitroso, cyano, alkyl (e.g., methyl, ethyl, isopropyl, *tert*-butyl), alkenyl (e.g., vinyl), alkynyl (e.g., ethynyl), cycloalkyl (e.g., cyclopropyl, adamantyl), cycloalkylalkyl (e.g., cyclohexylmethyl, adamantylmethyl), cycloalkenyl (e.g., cyclopropenyl), aryl (e.g., phenyl, naphthyl), arylalkyl (e.g., benzyl, phenethyl), heteroaryl (e.g., pyridyl, furyl), heteroarylalkyl (e.g., pyridylmethyl), heterocyclyl (e.g., piperidyl), heterocyclylalkyl (e.g., morpholylmethyl), alkoxy (e.g., methoxy, ethoxy, propoxy, butoxy), halogenated alkoxy (e.g., $OCF_3$), alkenyloxy (e.g., vinyloxy, allyloxy), aryloxy (e.g., phenyloxy), alkoxycarbonyl (e.g., methoxycarbonyl, ethoxycarbonyl, tert-butoxycarbonyl), arylalkyloxy (e.g., benzyloxy), unsubstituted amino, substituted amino [(e.g., alkylamino (e.g., methylamino, ethylamino, dimethylamino), acylamino (e.g., acetylamino, benzoylamino), arylalkylamino (e.g., benzylamino, tritylamino), hydroxyamino], alkylaminoalkyl (e.g., diethylaminomethyl), sulfamoyl, carbamoyl, acyl (e.g., acetyl), alkylthio. (e.g., methylthio), oxo, sulfonyl (e.g., alkylsulfonyl, aminosulfonyl), and the like. Substitution may occur with 1 to 4 of such substituents.

As used herein, substituents of "substituted or unsubstituted amino" and "substituted or unsubstituted carbamoyl" include alkyl, alkenyl, aryl, heteroaryl, alkylcarbonyl, arylcarbonyl, heteroarylcarbonyl, heterocyclylcarbonyl, alkoxycarbonyl, aryloxycarbonyl, heteroaryloxycarbonyl, heterocyclyloxycarbonyl, sulfamoyl, alkylsulfonyl, carbamoyl, cycloalkylsulfonyl, arylsulfonyl, heteroarylsulfonyl, heterocyclylsulfonyl, hydroxy, aminosulfonyl, sulfinyl, amino, and the like.

Pharmaceutically acceptable salts of the compound of the present invention include the following salts.

Examples of basic salts thereof include: alkali metal salts such as sodium salts, potassium salts, and the like; alkaline-earth metal salts such as calcium salts, magnesium salts, and the like; ammonium salts; aliphatic amine salts such as trimethylamine salts, triethylamine salts, dicyclohexylamine salts, ethanolamine salts, diethanolamine salts, triethanolamine salts, procaine salts, meglumine salts, diethanolamine salts, ethylenediamine salts, and the like; aralkylamine salts such as N,N-dibenzylethylenediamine salts, benethamine salts, and the like; heterocyclic aromatic amine salts such as pyridine salts, picoline salts, quinoline salts, isoquinoline salts, and the like; quaternary ammonium salts such as tetramethylammonium salts, tetraethylammonium salts, benzyltrimethylammonium salts, benzyltriethylammonium salts, benzyltributylammonium salts, methyltrioctylammonium salts, tetrabutylammonium salts, and the like; basic amino acid salts such as arginine salts, lysine salts; and the like.

Examples of acidic salts thereof include: inorganic acid salts such as hydrochloride, sulfate, nitrate, phosphate, carbonate, bicarbonate, perchlorate, and the like; organic acid salts such as acetate, propionate, lactate, maleate, fumarate, tartrate, malate, citrate, ascorbate, and the like; sulfonate such as methanesulfonate, isethionate, benzenesulfonate, *p*-toluenesulfonate; acidic amino acids salts such as aspartate, glutamate, and the like.

As a pharmaceutically acceptable prodrug of the present invention, any form known in the art can be adopted. A prodrug refers to a compound that, taking advantage of a metabolic machinery *in vivo,* does not exhibit a pharmaceutical effect or merely exhibits very low activity in its original form, but is modified so as to, when metabolized *in vivo*, thereby exhibit or increase pharmacological activity for the first time. Examples of prodrugs can include not only salts, solvates, and the like, but also esters, amides, and the like.

The term "solvate" means a solvate of the compound of the present invention, or a pharmaceutically acceptable salt thereof. Examples thereof include solvates formed with alcohol (e.g., ethanol), hydrates, and the like. Examples of hydrates can include monohydrate, dihydrate, and the like.

Moreover, one or more hydrogen, carbon, or other atoms in the compound of formula (I) may be replaced with isotopes of hydrogen, carbon, or other atoms respectively. The compound of formula (I) encompasses all of radiolabeled compounds of the compound of formula (I). Such "radiolabeling," "a radiolabeled compound," and the like of the compound of formula (I) are each encompassed by the present invention, and are useful for studies on metabolized drug pharmacokinetics and studies on binding assay, and/or a diagnostic tool. Furthermore, they are also useful as medicines.

Examples of isotopes that may be incorporated in the compound of formula (I) include hydrogen, carbon, nitrogen, oxygen, phosphorus, sulfur, fluorine, and chlorine, such as $^{2}H$, $^{3}H$, $^{13}C$, $^{14}C$, $^{15}N$, $^{18}O$, $^{17}O$, $^{31}P$, $^{32}P$, $^{35}S$, $^{18}F$, and $^{36}Cl$ respectively. A radiolabeled compound of the present invention can be prepared using a well-known method in the relevant technical field. For example, a tritium-labeled compound of formula (I) can be prepared by introducing a tritium to a certain compound of formula (I), for example, through a catalytic dehalogenation reaction using a tritium. This method may comprise reacting with an appropriately-halogenated precursor of the compound of formula (I) with tritium gas in the presence of an appropriate catalyst, such as Pd/C, and in the presence or absent of a base. For another appropriate method of preparing a tritium-labeled compound, the document: Isotopes in the Physical and Biomedical Science, Vol. 1, Labeled Compounds (Part A), Chapter 6 (1987) can be referred to. A $^{14}C$-labeled compound can be prepared by using a raw material having $^{14}C$.

In the compound of the present invention, the following embodiments of compounds are included. Combinations of U, V, W, and X include the following:

[U, V, W, X] = [=C (-R') -, =C (-R")-, =C (-R')-, =N-], [=C (-R')-, =C(-R")-, =N-, =C(-R")-], [=C(-R')-, =N-, =C(-R')-, =C(-R")-], [=N-, =C(-R")-, =C(-R')-, =C(-R")-], [=C(-R')-, =N-, =N-, =C(-R")-], [=C(-R')-, =C(-R")-, =N-, =N-], [=C(-R')-, =N-, =C(-R')-, =N-].

Furthermore, in the compound of the present invention represented by formula (I), the following embodiments of compounds are include.

(A)

Each substituent is defined as in item (1) above unless specified otherwise.

In general formula (I),

U and W are =C(-R')-;

V is -C(-R")-;

X is =N-;

R' is hydrogen, halogen, nitro, carboxy, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted acyl, or substituted or unsubstituted alkoxycarbonyl;

R" is hydrogen, halogen, substituted or unsubstituted alkyl, substituted or unsubstituted aryl, substituted or unsubstituted amino, or substituted or unsubstituted carbamoyl;

$R^1$ is substituted or unsubstituted aryl, substituted or unsubstituted heteroaryl, substituted or unsubstituted heterocyclyl, or a group represented by the formula:

$$-C(=O)-NR^BR^C;$$

$R^B$ is hydrogen;

$R^C$ is substituted or unsubstituted alkyl, substituted or unsubstituted aryl, substituted or unsubstituted cycloalkyl, substituted or unsubstituted heteroaryl, or substituted or unsubstituted heterocyclyl;

$R^2$ and $R^3$ are each hydrogen; and

Z is $-CH_2-$.

(B)

Each substituent is defined as in item (1) above unless specified otherwise.

In general formula (I),

U and W are =C(-R')-;

V is =C(-R")-;

X is =N-;

R' is hydrogen, halogen, nitro, carboxy, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstitutedamino, substituted or unsubstituted carbamoyl, substituted or unsubstituted acyl, or substituted or unsubstituted alkoxycarbonyl;
R" is hydrogen, halogen, substituted or unsubstituted alkyl, substituted or unsubstituted aryl, substituted or unsubstituted amino, or substituted or unsubstituted carbamoyl;
$R^1$ is a group represented by the formula: -C(=O)-$NR^BR^C$;
$R^B$ is hydrogen;
$R^C$ is substituted or unsubstituted alkyl, substituted or unsubstituted aryl, substituted or unsubstituted cycloalkyl, substituted or unsubstituted heteroaryl, or substituted or unsubstituted heterocyclyl;
$R^2$ and $R^3$ are each hydrogen; and
Z is $-CH_2-$.

(C)

Each substituent is defined as in item (1) above unless specified otherwise.
In general formula (I),
U is =C(-H)-;
W is =C(-R')-;
V is =C(-R")-;
X is =N-;
R' is hydrogen, halogen, nitro, carboxy, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted acyl, or substituted or unsubstituted alkoxycarbonyl;
R" is hydrogen, halogen, substituted or unsubstituted alkyl, substituted or unsubstituted aryl, substituted or unsubstituted amino, or substituted or unsubstituted carbamoyl;
$R^1$ is substituted or unsubstituted aryl, substituted or unsubstituted heteroaryl, substituted or unsubstituted heterocyclyl, or a group represented by the formula:

$$-C(=O)-NR^BR^C;$$

$R^B$ is hydrogen;
$R^C$ is substituted or unsubstituted alkyl, substituted or unsubstituted aryl, substituted or unsubstituted cycloalkyl, substituted or unsubstituted heteroaryl, or substituted or unsubstituted heterocyclyl;
$R^2$ and $R^3$ are each hydrogen; and
Z is $-CH_2-$.

(D)

Each substituent is defined as in item (1) above unless specified otherwise.
In general formula (I),
U is =C(-H)-;
W is =C(-R')-;
V is =C(-R")-;
X is =N-;
R' is hydrogen, halogen, nitro, carboxy, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted acyl, or substituted or unsubstituted alkoxycarbonyl;
R" is hydrogen, halogen, substituted or unsubstituted alkyl, substituted or unsubstituted aryl, substituted or unsubstituted amino, or substituted or unsubstituted carbamoyl;
$R^1$ is a group represented by the formula: -C(=O)-$NR^BR^C$;
$R^B$ is hydrogen;
$R^C$ is substituted or unsubstituted alkyl, substituted or unsubstituted aryl, substituted or unsubstituted cycloalkyl, substituted or unsubstituted heteroaryl, or substituted or unsubstituted heterocyclyl;
$R^2$ and $R^3$ are each hydrogen; and
Z is $-CH_2-$.

(E)

Each substituent is defined as in item (1) above unless specified otherwise.
In general formula (I),
U is =N-;

W is =C(-R')-;
V and X are -C(-R")-;
R' is hydrogen, halogen, nitro, carboxy, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted acyl, or substituted or unsubstituted alkoxycarbonyl;
R" is hydrogen, halogen, substituted or unsubstituted alkyl, substituted or unsubstituted aryl, substituted or unsubstituted amino, or substituted or unsubstituted carbamoyl;
$R^1$ is substituted or unsubstituted aryl, substituted or unsubstituted heteroaryl, substituted or unsubstituted heterocyclyl, or a group represented by the formula:

$$-C(=O)-NR^BR^C;$$

$R^B$ is hydrogen;
$R^C$ is substituted or unsubstituted alkyl, substituted or unsubstituted aryl, substituted or unsubstituted cycloalkyl, substituted or unsubstituted heteroaryl, or substituted or unsubstituted heterocyclyl;
$R^2$ and $R^3$ are each hydrogen; and
Z is $-CH_2-$.

(F)
Each substituent is defined as in item (1) above unless specified otherwise.
In general formula (I),
U is =N-;
W is =C(-R')-;
V and X are =C(-R")-;
R' is hydrogen, halogen, nitro, carboxy, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted acyl, or substituted or unsubstituted alkoxycarbonyl;
R" is hydrogen, halogen, substituted or unsubstituted alkyl, substituted or unsubstituted aryl, substituted or unsubstituted amino, or substituted or unsubstituted carbamoyl;
$R^1$ is a group represented by the formula: $-C(=O)-NR^BR^C$;
$R^B$ is hydrogen;
$R^C$ is substituted or unsubstituted alkyl, substituted or unsubstituted aryl, substituted or unsubstituted cycloalkyl, substituted or unsubstituted heteroaryl, or substituted or unsubstituted heterocyclyl;
$R^2$ and $R^3$ are each hydrogen; and
Z is $-CH_2-$.

(G)
Each substituent is defined as in item (1) above unless specified otherwise.
In general formula (I),
U is =N-;
W is =C(-R')-;
V is =C(-R")-; X is =C(-H)-;
R' is hydrogen, halogen, nitro, carboxy, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted acyl, or substituted or unsubstituted alkoxycarbonyl;
R" is hydrogen, halogen, substituted or unsubstituted alkyl, substituted or unsubstituted aryl, substituted or unsubstituted amino, or substituted or unsubstituted carbamoyl;
$R^1$ is substituted or unsubstituted aryl, substituted or unsubstituted heteroaryl, substituted or unsubstituted heterocyclyl, or a group represented by the formula:

$$-C(=O)-NR^BR^C;$$

$R^B$ is hydrogen;
$R^C$ is substituted or unsubstituted alkyl, substituted or unsubstituted aryl, substituted or unsubstituted cycloalkyl, substituted or unsubstituted heteroaryl, or substituted or unsubstituted heterocyclyl;
$R^2$ and $R^3$ are each hydrogen; and
Z is $-CH_2-$.

(H)
Each substituent is defined as in item (1) above unless specified otherwise.
In general formula (I),
U is =N-;
W is =C(-R')-;
V is =C(-R")-;
X is =C(-H)-;
R' is hydrogen, halogen, nitro, carboxy, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted acyl, or substituted or unsubstituted alkoxycarbonyl;
R" is hydrogen, halogen, substituted or unsubstituted alkyl, substituted or unsubstituted aryl, substituted or unsubstituted amino, or substituted or unsubstituted carbamoyl;
$R^1$ is a group represented by the formula: $-C(=O)-NR^BR^C$;
$R^B$ is hydrogen;
$R^C$ is substituted or unsubstituted alkyl, substituted or unsubstituted aryl, substituted or unsubstituted cycloalkyl, substituted or unsubstituted heteroaryl, or substituted or unsubstituted heterocyclyl;
$R^2$ and $R^3$ are each hydrogen; and
Z is $-CH_2-$.

(J)
Each substituent is defined as in item (1) above unless specified otherwise.
In general formula (I):
$R^1$ includes a group represented by the formula:

$$-C(=O)-NR^BR^C.$$

$R^B$ includes hydrogen.

$R^C$ includes substituted or unsubstituted alkyl, substituted or unsubstituted aryl, substituted or unsubstituted heteroaryl, or substituted or unsubstituted heterocyclyl.

$R^C$ includes unsubstituted C1-C6 alkyl, or C1-C6 alkyl substituted with: C1-C6 alkoxy; aryl; or acyl.

$R^C$ includes unsubstituted aryl, or aryl substituted with: halogen; C1-C6 alkoxy; C1-C6 alkyl; aryl; acyl ; nitro; cyano; aryloxy,; substituted amino; C1-C6 alkylthio; C1-C6 alkoxycarbonyl; carboxy; or heteroaryl (e.g., oxadiazole).

$R^C$ includes substituted or unsubstituted heteroaryl.
Examples thereof include substituted or unsubstituted pyridyl, and substituted or unsubstituted pyrimidinyl.

$R^C$ includes substituted or unsubstituted heterocyclyl. An example thereof is substituted or unsubstituted piperidinyl.

When $R^C$ is substituted or unsubstituted heteroaryl, or substituted or unsubstituted heterocyclyl, examples of substituents thereof include C1-C6 alkoxy, C1-C6 alkoxycarbonyl, and carboxy.

$R^1$ includes substituted or unsubstituted aryl, or substituted or unsubstituted heteroaryl (e.g., dihydrothiazolyl, dihydroquinolyl, chromenyl).

When $R^1$ is substituted or unsubstituted aryl, or substituted or unsubstituted heteroaryl, examples of substituents thereof include C1-C6 alkoxy and C1-C6 alkyl.

R' includes hydrogen, halogen, carboxy, substituted or unsubstituted alkoxycarbonyl, substituted or unsubstituted aryl, substituted or unsubstituted carbamoyl, substituted or unsubstituted acyl, substituted or unsubstituted heteroaryl, substituted or unsubstituted cycloalkyl, substituted or unsubstituted cycloalkenyl, substituted or unsubstituted alkyl, substituted or unsubstituted alkenyl, substituted or unsubstituted amino, nitro, or substituted or unsubstituted heterocyclyl.

R' includes unsubstituted carbamoyl, or carbamoyl substituted with: C1-C6 alkyl or aryl.

R' includes unsubstituted aryl, or aryl substituted with: carboxy; halogen; C1-C6 alkoxy; substituted amino; substituted sulfonyl; carbamoyl; C1-C6 alkyl; hydroxy; or C1-C6 alkoxycarbonyl.

R' includes substituted or unsubstituted heteroaryl. Examples thereof include thiophenyl, dihydrobenzofuranyl, pyrazolyl, benzomorpholyl, indolyl, thiazolyl, oxazolyl, furanyl, isoxazolyl, pyridyl, pyrimidinyl, pyrrolyl, and pyranyl.

R' includes substituted or unsubstituted heterocyclyl. Examples thereof include morpholinyl, piperidinyl, and piperazinyl.

When R' is substituted or unsubstituted heteroaryl, or substituted or unsubstituted heterocyclyl, examples of substituents thereof include C1-C6 alkyl, C1-C6 alkoxycarbonyl, carboxy, amino, oxo, and C1-C6 alkoxy.

R' includes unsubstituted alkyl, or an alkyl substituted with: aryl; C1-C6 alkoxycarbonyl; or carboxy.

R' includes unsubstituted amino, or amino substituted with: aryl; C1-C6 alkyl; acryl; substituted sulfonyl; heteroaryl (e.g., pyridyl); heterocyclyl (e.g., piperidinyl); or carbamoyl.

R" includes hydrogen, substituted or unsubstituted aryl, halogen, substituted or unsubstituted alkyl, substituted or unsubstituted amino, substituted or unsubstituted heteroaryl, substituted or unsubstituted heterocyclyl, substituted or unsubstituted alkoxycarbonyl, carboxy, or substituted or unsubstituted carbamoyl.

R" includes unsubstituted aryl, or aryl substituted with: C1-C6 alkoxycarbonyl or carboxy.

R" includes alkyl substituted with carboxy.

R" includes unsubstituted amino, or amino substituted with: C1-C6 alkyl; aryl; acyl; or heteroaryl (e.g., dihydrobenzofuranyl, benzothiazolyl, and quinolinyl).

R" includes substituted or unsubstituted heteroaryl. Examples thereof include pyrazolyl, pyrrolyl, thiophenyl, and indolyl.

R" includes substituted or unsubstituted heterocyclyl. Examples thereof include benzomorpholyl, and piperazinyl.

When R" is substituted or unsubstituted heteroaryl, or substituted or unsubstituted heterocyclyl, examples of substituents thereof include C1-C6 alkoxycarbonyl, carboxy, carbamoyl, heterocyclyl (e.g., tetrahydropyranyl), C1-C6 alkyl, C1-C6 alkoxy, and oxo.

(A) to (H) and (J), described above, include compounds shown by combining a part or all thereof.

(Production Method)

General production methods of the compound of the present invention are illustrated hereinbelow. Furthermore, with regard to extraction, purification, and the like, treatments as in usual experiments of organic chemistry may be carried out.

Hereinafter, production methods of the compound of the present invention are described.

Synthesis of the compound of the present invention can be performed by reference to a known method in the relevant field.

As a raw material compound, the following is available: commercially available compounds; those described in Patent Document 3, 4, 5, 6, 7, 8, 9, 10, 11, 12, 13, 14, 15, or 16 or Non-patent Document 14, 15, 16, 17, 18, or 19; those described in US 2001/0053853, Journal of Organic Chemistry, 2003, 68, 7918-7920, or the present specification; those described in another document cited herein; and other known compounds.

Regarding some of the compound of the present invention, a tautomer, regioisomer, or optical isomer thereof may exist. The present invention encompasses all possible isomers including these, and mixtures thereof.

When it is desired to obtain a salt of the compound of the present invention, in the case that the compound of the invention is obtained in salt form, it may be purified as it is. Furthermore, in the case that it is obtained in free form, it may be dissolved or suspended in an appropriate organic solvent and then an acid or base may be added thereto to form a salt thereof using a general method.

Furthermore, the compound of the present invention and a pharmaceutically acceptable salt thereof may exist in form of adduct with water or any kind of solvent (hydrate or solvate). These adducts are also encompassed by the present invention.

Derivatives thereof are converted in the body and consequently activated, which are named "prodrug" herein. It is understood that examples of prodrugs include not only the aforementioned salts and solvates, but also esters (e. g. , alkyl ester and the like), amides, and the like.

Various examples of the compound of the present invention are listed in Examples. By reference to these, those skilled in the art can produce and use compounds that are not illustrated in the present invention.

The present invention also relates to a system, an apparatus, and a kit for producing the compound of the present invention. It is understood that, as elements of such a system, an apparatus, and a kit, matters known in the relevant field are available, and those skilled in the art can appropriately design them.

(General Method for Synthesizing Compound I)

[Formula 24]

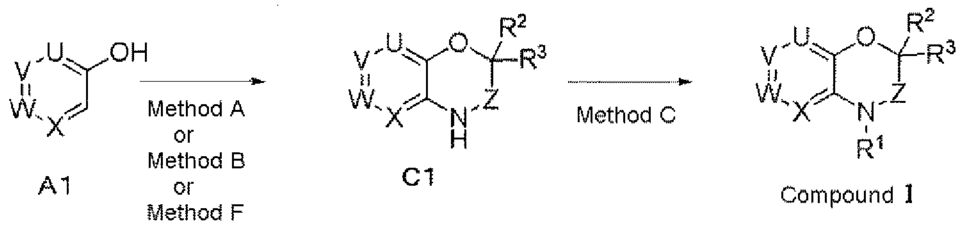


wherein each symbol is defined as above, a known compound may be used as the compound represented by formula (A1), and a compound derived from a known compound using a usual method may be also used.

The above method is for synthesizing Compound I of the present invention from a compound represented by formula (A1). A compound represented by formula (C1) is synthesized from formula (A1) using method A, B, or F. Subsequently, Compound I of the present invention is synthesized using method C. Methods A to C are described in detail below.

1) Method A: Synthesis method of a compound represented by formula (C1)

[Formula 25]

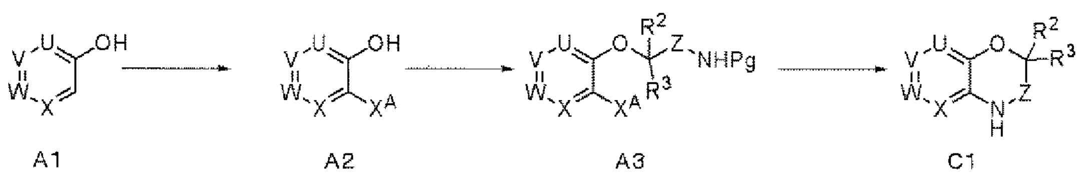


wherein each symbol is defined as above, and $X^A$ includes halogen (e.g., Cl, Br, I, and the like) and leaving groups such as -OMs, -OTs, -OTf, -ONs, and the like. Here, "Ms" refers to a methanesulfonyl group, "Ts" refers to a *para*-toluenesulfonyl group, "Tf" refers to a trifluoromethanesulfonyl group, and "Ns" refers to an ortho-nitrobenzenesulfonyl group. Pg refers to an amino-protecting group (e.g., t-butoxycarbonyl group, benzyl group, benzyloxycarbonyl group, and the like). A known compound may be used as a compound represented by formula (A1), and a compound derived from a known compound using a usual method may be also used.
The above method is for synthesizing a compound represented by formula (C1) from a compound represented by formula (A1). It is synthesized according to a method described in US 2001/0053853A1, p.17.

2) Method B: Synthesis method of a compound of formula (C1)

[Formula 26]

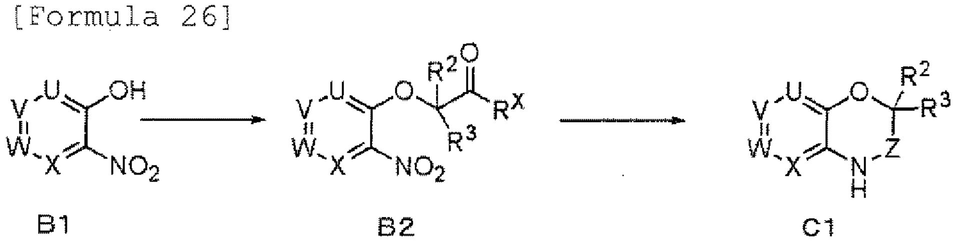


wherein each symbol is defined as above, a known compound may be used as a compound represented by formula (B1), and a compound derived from a known compound using a usual method may be also used.
The above method is for synthesizing a compound represented by formula (C1) from a compound represented by formula (B1).
Hereinafter it is described in detail.

2)-1

[Formula 27]

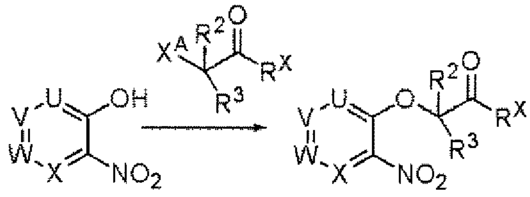

B1 B2

wherein each symbol is defined as above, $X^A$ includes halogen (e.g., Cl, Br, I, and the like) and leaving groups such as -OMs, -OTs, -OTf, -ONs, and the like. Here, "Ms" refers to a methanesulfonyl group, "Ts" refers to a *para*-toluenesulfonyl group, "Tf" refers to a trifluoromethanesulfonyl group, and "Ns" refers to an ortho-nitrobenzenesulfonyl group. $R^x$ refers to an alkoxy (e.g., methoxy, ethoxy, and the like), halogen, or the like. A known compound may be used as a compound represented by formula (B1), and a compound derived from a known compound using a usual method may be also used.
The above step is of reacting a compound represented by formula (B1) with a compound represented by the formula:
[Formula 28]

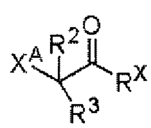

in the presence of a base to synthesize a compound represented by formula (B2).
Reaction solvents include N, N-dimethylformamide (DMF), N-methyl-2-pyrrolidone (NMP), N,N-dimethylacetamide (DMA), dimethylsulfoxide, aromatic hydrocarbons (e.g., toluene, benzene, xylene, and the like), saturated hydrocarbons (e.g., cyclohexane, hexane, and the like), halogenated hydrocarbons (e.g., dichloromethane, chloroform, 1,2-dichloroethane, and the like), ethers (e. g. , tetrahydrofuran, dimethyl ether, dioxane, 1,2-dimethoxyethane, and the like), esters (e.g., methyl acetate, ethyl acetate, and the like), ketones (e.g., acetone, methylethylketone, and the like), nitriles (e.g., acetonitrile, and the like), alcohols (e.g., methanol, ethanol, t-butanol, and the like), pyridines (pyridine, 2,6-lutidine, and the like), water, and mixed solvents thereof, and the like.
Examples of bases include metal hydrides (e.g., sodium hydride, and the like), metal hydroxides (e.g., sodium hydroxide, potassium hydroxide, lithium hydroxide, barium hydroxide, and the line), metal carbonate salts (e.g. , sodium carbonate, potassium carbonate, calcium carbonate, cesium carbonate, and the like), metal alkoxides (e.g., sodium methoxide, sodium ethoxide, potassium t-butoxide, and the like), sodium bicarbonate, metallic sodium, organic amines (e.g., triethylamine, diisopropylethylamine, 1,8-diazabicyclo[5.4.0]undec-7-ene (DBU), pyridine, 2,6-lutidine, and the like), alkyl lithium (*n*-butyl lithium (*n*-BuLi), sec-BuLi, *tert*-BuLi), and the like.
Preferably, an ether (e.g., tetrahydrofuran, diethyl ether, dioxane, and the like), N,N-dimethylformamide, or acetonitrile is used as reaction solvent, as well as a metal carbonate salt (e.g., sodium carbonate, potassium carbonate, calcium carbonate, cesium carbonate, and the like) may be used as a base. A reaction temperature and a reaction time are not specifically limited, but the reaction may be carried out for a period of time from 0.5 to 12 hours at temperatures from -20˚C to a temperature at which a solvent used is refluxed.
2)-2
[Formula 29]

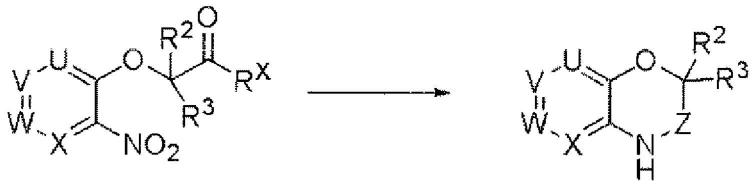

B2 C1

wherein each symbol is defined as above, and $R^X$ refers to an alkoxy (e.g., methoxy, ethoxy, and the like), halogen, or the like.

The above step is of reducing the nitro group of a compound represented by formula (B2) to an amino group, followed by intramolecular cyclization, to synthesize a compound represented by formula (C1).

Fe, Sn, tin chloride ($SnCl_2$), Raney nickel, or the like can be used as a reducing agent. The above redaction step can be carried out in the presence of acid (e.g., hydrochloric acid, sulfuric acid, acetic acid, and the like).

A solvent described in Step 2)-1 above can be used as solvent. An acid used may be also used as solvent.

The reaction may be carried out for a period of time from 0.5 to 12 hours at temperatures from -20˚C to a temperature at which a solvent used is refluxed.

When Z is $-(CR^4R^5)_n-$, a compound represented by formula (C1) can be synthesized by the aforementioned cyclization followed by reaction with a reducing agent.

For example, sodium tetrahydroborate, borane complex, sodium cyanoborohydride, lithium aluminum hydride, or the like is used as a reducing agent. A solvent described in 2)-1 can be used as solvent. Preferably, the reaction may be carried out in ether solvent.

The reaction may be carried out for a period of time from 0.5 to 12 hours at temperatures from -20˚C to a temperature at which a solvent used is refluxed.

2') Method F: Synthesis method of a compound represented by formula (C1)

[Formula 30]

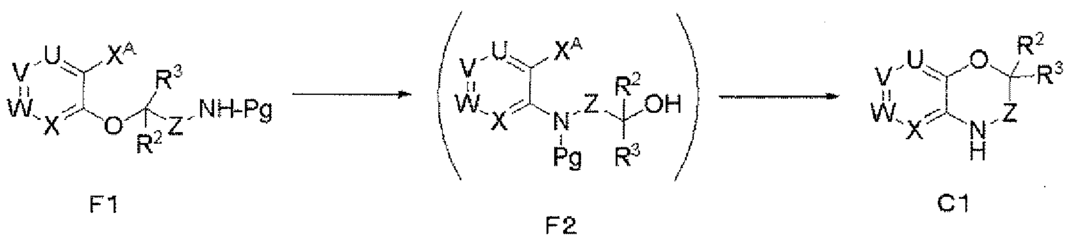

F1 F2 C1

wherein each symbol is defined as above, and $X^A$ refers to a halogen (e.g., C1, Br, I, and the like) or a leaving group described in 1) above (e.g., OTf, OMs, and the like). Pg refers to an amino-protecting group (e.g., t-butoxycarbonyl group, benzyl group, benzyloxycarbonyl group, and the like) . A known compound may be used as a compound represented by formula (F1), and a compound derived from a known compound using a usual method may be also used.

The above step is of converting a compound represented by formula (F1) through a rearrangement reaction to a compound represented by formula (F2), followed by cyclization, to synthesize a compound represented by formula (C1).

According to a method described in Journal of Organic Chemistry, 2003, 68, 7918-7920, the reaction may be carried out in the presence of a solvent and a base described in 2) -1 above.

An ether (e.g., tetrahydrofuran, diethyl ether, dioxane, and the like), and a metal hydride (e.g., sodium hydride, and the like) are used as solvent and a base, respectively, to carry out the reaction at temperatures from -20 to 50˚C for a period of time from 0.5 to 12 hours.

3) Method C: Synthesis method of Compound I

[Formula 31]

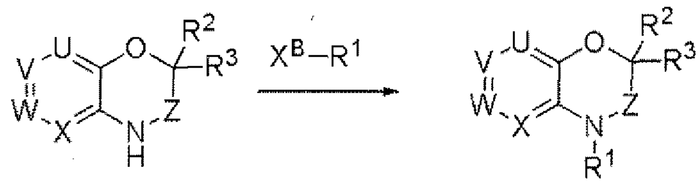

C1

Compound I

wherein each symbol is defined as above, $X^B$ refers to a halogen (e.g., Cl, Br, I, and the like) or a leaving group described in 1) above (e.g., OTf, OMs, and the lake). Known compounds may be used as a compound represented by formula (C1) and a compound represented by the formula $X^B$-$R^1$, and a compound derived from a known compound using a usual method may be also used.

The above step is of reacting a compound represented by formula (C1) with a compound represented by formula $X^B$-$R^1$ in the presence of a base to synthesize Compound I of the present invention. A solvent and a base described in 2) -1 can be used as a solvent and a base.

As a preferable example, a compound represented by formula (C1) can be reacted with an isocyanate in the presence of a base to synthesize Compound I wherein $R^1$ is a group represented by the formula: -C(=O)-$NR^BR^C$.

Another (e.g., tetrahydrofuran, diethylether, dioxane, and the like), and an organic amine (e.g., triethylamine, diisopropylethylamine, 1,8-diazabicyclo[5.4.0]undec-7-ene (DBU), 2,6-lutidine, and the like) may be used as solvent and a base, respectively, to carry out the reaction at temperatures from -20 to 50°C for a period of time from 0.5 to 12 hours.

4) Method D: Synthesis method of Compound I' wherein W of Compound I is =C-$X^A$.

[Formula 32]

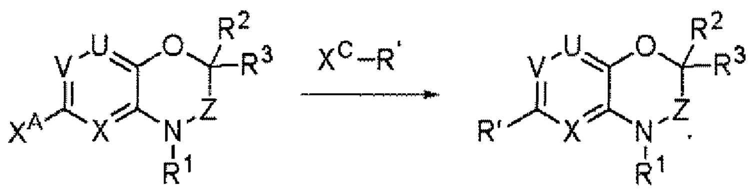

Compound I

Compound I'

wherein each symbol is defined as above, $X^A$ includes halogen (e.g., Cl, Br, I, and the like) and leaving groups such as -OMs, -OTs, -OTf, -ONs, and the like. Here, "Ms" refers to a methanesulfonyl group, "Ts" refers to a para-toluenesulfonyl group, "Tf" refers to a trifluoromethanesulfonyl group, and "Ns" refers to an ortho-nitrobenzenesulfonyl group. $X^C$ refers to a boronic acid, boronic acid ester, trialkyltin, or the like. A known compound may be used as a compound represented by the formula $X^C$-R', and a compound derived from a known compound using a usual method may be also used.

The above step is of reacting the above Compound I with a compound represented by the formula $X^C$-R' in the presence of a base to synthesize Compound I' of the present invention.

A solvent and a base described in 2) -1 can be used as solvent and a base.

Preferably, a coupling reaction can be used with a metal catalyst (e.g., palladium catalyst: palladium tetrakis-triphenylphosphine ($Pd(PPh_3)_4$), palladium chloride ($PdCl_2$), tris(dibenzylideneacetone)bispalladium ($Pd_2(dba)_3$), bis (dibenzylideneacetone) palladium (Pd $(dba)_2$), palladium acetate ($Pd(OAc)_2$), [1,1'-bis(diphenylphosphino) ferrocene] -dichloropalladium(II)-dichloromethane complex ($PdCl_2(dppf)$), bis(triphenylphosphine)palladium chloride ($PdCl_2$ $(PPh_3)_2$), and the like).

For example, an ether (e.g., tetrahydrofuran, diethyl ether, dioxane, and the like), and a metal carbonate salt (e.g., sodium carbonate, potassium carbonate, calcium carbonate, cesium carbonate, and the like) may be used as solvent and a base, respectively, to carry out the reaction for a period of time from 0.5 to 12 hours at temperatures from -20°C to a temperature at which a solvent used is refluxed.

5) Method E: Synthesis method of Compound I" wherein V of Compound I is =C-$X^A$

[Formula 33]

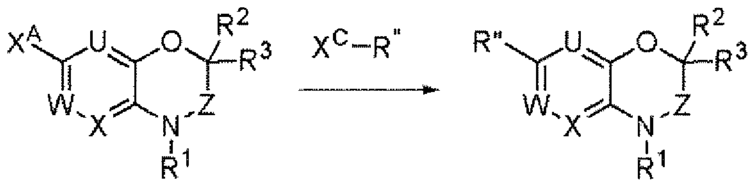


Compound I                Compound I"

wherein each symbol is defined as above, $X^A$ includes halogen (e.g., Cl, Br, I, and the like) and leaving groups such as -OMs, -OTs, -OTf, -ONs, and the like. Here, "Ms" refers to a methanesulfonyl group, "Ts" refers to a para-toluenesulfonyl group, "Tf" refers to a trifluoromethanesulfonyl group, and "Ns" refers to an ortho-nitrobenzenesulfonyl group. $X^C$ refers to boronic acid, a boronic acid ester, trialkyltin, and the like. A known compound may be used as a compound represented by the formula $X^C$-R", and a compound derived from a known compound using a usual method may be also used.
The above step is of reacting the above-described Compound I with a compound represented by the formula $X^C$-R" in the presence of a base to synthesize Compound I" of the present invention, using a method similar to method D.
By treatment similar to methods D and E, a substituent (s) can be introduced to R' and R".
The production of the present invention can be practiced by appropriately improve or combine the above-described preferable embodiments, or add known technique thereto.
In the above general synthesis methods, reaction steps are not limited to the above ones, and the compound of the present invention can be synthesized after changing the order of reactions.
For example, the conversion of a substituent shown in method D or E can be performed prior to the step of method A or B.
Furthermore, examples of protecting groups (amino-protecting groups, hydroxy-protecting groups, and the like) can include protecting groups such as ethoxycarbonyl, t-butoxycarbonyl, acetyl, benzyl, and the like, which are described in Protective Groups in Organic Synthesis, written by T. W. Green, John Wiley & Sons Inc. (1991), or the like. Methods for the introduction and removal of a protecting group are methods commonly used in synthetic organic chemistry (see, for example, methods described in Protective Groups in Organic Synthesis, written by T. W. Greene, John Wiley & Sons Inc. (1991)) or the like, or can be obtained in accordance therewith. Furthermore, a functional group included in each substituent can be converted by a known method (for example, those described in Comprehensive Organic Transformations, written by R. C. Larock (1989), and the like) in addition to the above production methods. Some of the compounds of the present invention can be used as a synthetic intermediate, leading to a new derivative. Intermediates and target compounds produced in each of the above production methods can be isolated and purified by a purification method commonly used in synthetic organic chemistry, for example, subjecting them to neutralization, filtration, extraction, washing, drying, concentration, recrystallization, any kind of chromatography, or the like. Furthermore, intermediates can be subj ected to a next reaction without any purification.

(Medicament)

The compound of the present invention or a pharmaceutically acceptable salt can be administered alone as it is, but it is usually preferable to provide it as a variety of pharmaceutical formulations. Furthermore, those pharmaceutical formulations are used for an animal and a human.
With regard to an administration route, it is preferable to use the most effective route on therapy. It can be peroral administration, or parenteral administration, for example, intrarectal; intraoral; subcutaneous; intramuscular; intravenous; percutaneous such as application; pulmonary with a spray such as powder, aerosol, and the like; or the like.
Administration forms include capsule, tablet, granule, powder, syrup, emulsion, suppository, injection, and the like. A liquid preparation, such as emulsion and syrup, which is suitable for oral administration, can be produced using: water; sugars such as sucrose, sorbite, fructose, and the like; glycols such as polyethylene glycol, propylene glycol, and the like; oils such as sesame oil, olive oil, soybean oil, and the like; antiseptics such as p-hydroxybenzoate esters, and the like; and flavors such as strawberry flavor, peppermint, and the like. Furthermore, a capsule, a tablet, a powder, a granule, and the like can be produced using: an excipient such as lactose, glucose, sucrose, mannite, and the like; a disintegrator such as starch, sodium alginate and the like; a lubricant such as magnesium stearate, talc, and the like; a binder such as polyvinyl alcohol, hydroxypropylcellulose, gelatin, and the like; surfactant such as fatty ester and the like; and a plasticizer such as glycerin and the like.
A formulation suitable for parenteral administration preferably consists of a sterilized-water-based formulation

containing an active compound and being isotonic to blood of a recipient. For example, in the case of injection, a solution for an injection, is prepared using: a carrier consisting of a salt solution, a glucose solution, or a mixture of salt water and a glucose solution; or the like.

A topical formulation is prepared by dissolving or suspending an active compound in one or more kinds of media, such as mineral oil, petroleum, polyalcohol, and the like, or other bases used for a topical pharmaceutical formulation. A formulation for enteral administration is prepared using a general carrier such as cacao butter, hydrogenated fat, hydrogenated fatty carboxylic acid, and the like, and then provided as a suppository.

In the present invention, to a parenteral agent can be added one or more kinds of auxiliary ingredients selected from glycols, oils, flavors, antiseptics (including antioxidants), excipients, disintegrators, lubricants, binders, surfactants, plasticizer, and the like exemplified in an oral agent.

An effective dose and the frequency of administration of the compound of the present invention or a pharmaceutically acceptable salt are different according to administration form, the age of a patient, weight, characteristics or the severity, and the like of a condition to be treated. Generally, a dose is 0.01 to 1000 mg/person per day, preferably 5 to 500 mg/person per day, and a frequency of administration is preferably once per day or divided administration.

All of the compounds of the present invention are immediately applicable to therapeutic use as a kinase inhibitor for controlling kinase dependent diseases in mammals, particularly, a kinase inhibitor related to phosphatidylinositol-3-kinase.

The compound of the present invention is preferably a compound having an $IC_{50}$ value in a range of 0.1 nmol/L to 10 $\mu$mol/L. A certain compound of the present invention wherein the compound is capable of specifically inhibiting one of four types of Class I phosphatidylinositol-3-kinase (e.g., $\alpha$, $\beta$, $\gamma$, and $\delta$) can be selected. For example, by utilizing a compound selectively inhibiting only $\gamma$ type, merely diseases related to inflammation, such as a lymphocyte and the like can be treated. In the case that a compound is $\alpha$-type selective, the utility as a selective anticancer agent can be found.

Phosphatidylinositol-3-kinase dependent diseases include inflammatory diseases (allergic diseases (allergic dermatitis/allergic rhinitis, and the like), articular rheumatism, anaphylaxis, and the like), arteriosclerosis, vascular/circulatory diseases, cancer/tumors (hyperproliferative malfunction), immune system diseases, cell-proliferative diseases, infectious diseases, and the like initiated/maintained by unusual phosphatidylinositol-3-kinase enzyme activity. Examples thereof include psoriasis, pulmonary fibrosis, glomerulonephritis, cancers, atherosclerosis, and antiangiogenesis (e.g., tumor growth, diabetic retinopathy). Specifically, for example, the pharmaceutical composition of the present invention may be used for the prophylaxis and/or as a therapeutic agent for diseases such as encephalitis, myelitis and encephalomyelitis, meningitis, inflammatory polyneurapathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinaphilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonarydiseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathicthrombocytopenicpurpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like, or used as a therapeutic agent for burn or traumatic inflammation.

The present invention is also related to a system, an apparatus, and a kit for producing the pharmaceutical composition of the present invention. It is understood that, as elements of such a system, an apparatus, and a kit, matters publicly known in the relevant field are available, and those skilled in the art can appropriately design them.

The present invention is also related to a system, an apparatus, and a kit using the compound of the present invention, or a pharmaceutically acceptable salt thereof, or a solvate thereof. It is understood that, as elements of such a system, an apparatus, and a kit, matters publicly known in the relevant field are available, and those skilled in the art can appropriately design them.
Wortmannin, which is a classical PI3K inhibitor, has low inhibition selectivity, high toxicity, and the like, and consequently is highly cytotoxic. Thus, by using a usual test to measure cytotoxicity, a PI3K inhibitor (or another class of a kinase inhibitor) that intends to cause an unpreferable side effect due to lack of the selectivity can be identified.
The compound of the present invention is a compound having utility as a medicament. Here, utility as a medicament includes the following points: the compound has good metabolic stability; the induction of a drug-metabolizing enzyme is low; the inhibition of a drug-metabolizing enzyme which metabolizes another drug is also low; the compound has high oral absorbency; the drug has high solubility; the clearance is low; the half-life is sufficiently long to express the efficacy; and the like.
Reference including scientific literature, patents, patent applications, and the like cited herein is incorporated herein by reference in its entirety at the same level as the case where each reference is specifically described.
Hereinafter, Examples describe the constitution of the present invention in more detail, but the present invention is not limited thereto. Regarding reagents and the like used below, unless specified otherwise, those commercially available are used.

EXAMPLES

Hereinafter, the present invention is described in more detail with Examples. However, the technical scope of the present invention is not limited by the Examples and the like.
In Examples, abbreviations described below are used. DMSO: Dimethylsulfoxide
HPLC: High Performance Liquid Chromatography
Me: Methyl
Ph: Phenyl

(Method for identifying a compound)

The LC/MS data and NMR spectra of compounds of the present application and intermediates thereof are measured under a condition selected from the three conditions below (Methods A to C), and the retention times and $[M+H]^+$ are shown.

(Method A)

Column: Waters Phenomenex Luna C18 (2) (5 μm, 50 x 4.6 mm)
Flow rate: 3 mL/min
UV detection wavelength: 254 nm
Mobile phase: [A] was aqueous 0.1% formic-acid-containing solution, and [B] was 0.1% formic-acid-containing solution in acetonitrile.
From 0 to 3 minutes, the mobile phase was a mixed solution of 90% of [A] and 10% of [B]. Thereafter for 3 minutes, the percentage of [B] in the mobile phase was gradually increased to 100%. Thereafter a solution of 100% of [B] was used as the mobile phase.

(Method B)

Column: Waters Xbridge C18 (5 μm 50 x 4.6 mm)
Flow rate: mL/min
UV detection wavelength: 254 nm
Mobile phase: [A] was aqueous 10 mmol/L ammonium-carbonate-containing solution, and [B] was acetonitrile.
From 0 to 3 minutes, the mobile phase was a mixed solution of 90% of [A] and 10% of [B]. Thereafter for 3 minutes, the percentage of [B] in the mobile phase was gradually increased to 100%. Thereafter a solution of 100% of [B] was used as the mobile phase.

(Method C)

Column: Shimadzu Shim-pack XR-ODS (2.2 μm, 50 x 3.0 mm)
Flow rate: 1.6 mL/min

UV detection wavelength: 254 nm
Mobile phase: [A] was aqueous 0.1% formic-acid-containing solution, and [8] was 0.1% formic-acid-containing solution in acetonitrile.
From 0 to 3 minutes, the mobile phase was a mixed solution of 90% of [A] and 10% of [B]. Thereafter for 3 minutes, the percentage of [B] in the mobile phase was gradually increased to 100%. Thereafter a solution of 100% of [B] was used as the mobile phase.

(Synthesis Examples)

(Reference Example 1 Synthesis of Compound 5)

[Formula 34]

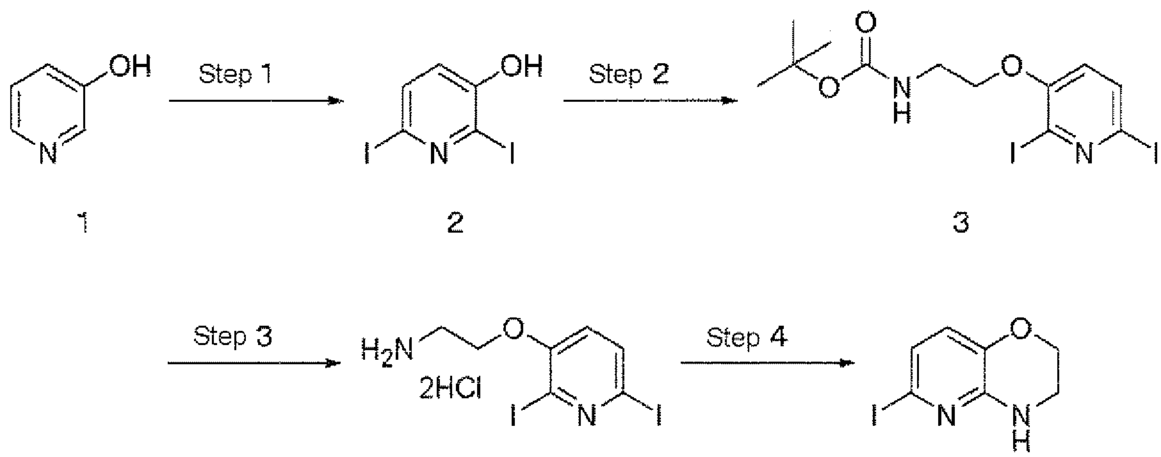


1 2 3

4 5

Compound 5 was synthesized using the above method, which was described in US 2001/0053853, page 17.

(Example 1 Synthesis of Compounds 1-42, 1-96, and I-51)

[Formula 35]

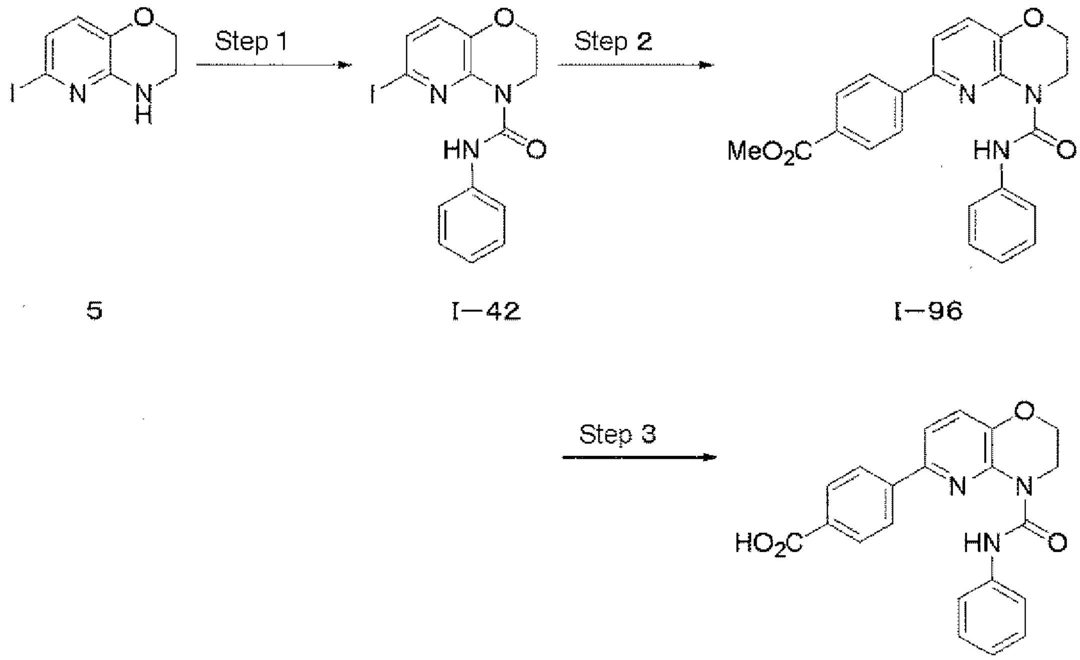


5 I-42 I-96

I-51

Step 1

To a solution of Compound (5) (1.90 g, 7.25 mmol) in tetrahydrofuran (20mL), phenylisocyanate (1.0mL, 9.43mmol) and triethylamine (1.5 mL, 10.9 mmol) were added. The solution was then stirred at room temperature for 1 hour. The precipitated powder was filtered, and then washed with water. The resulting powder was further washed with diethyl ether to yield Compound 1-42 (1.95 g, 63%) as a white powder. $^1$H-NMR (DMSO-$d_6$) δ12.13 (1H, s), 7.57 (2H, d, J = 8.4 Hz), 7.45 (1H, d, J = 8.1 Hz), 7.37 (2H, t, J = 7.5 Hz), 7.17 (1H, d, J = 8.4 Hz), 7.08 (1H, t, J = 7.2 Hz), 4.28 (2H, br s), 4.03 (2H, br s)
LC/MS (Method A): 2.54 min, $[M+H]^+$ = 382.3.

Step 2

To a solution of Compound 1-42 (100 mg, 0.262 mmol) and 4- (methoxycarbonyl)phenylboronic acid (70.8 mg, 0.394 mmol) in dioxane (2.0 mL), tetrakis(triphenylphosphine)palladium (15.2 mg, 0.0130 mmol) and 2 mol/L potassium carbonate solution (0.53 mL, 1.03 mmol) were added. The solution was stirred at 100°C for 3 hours. The reaction solution was poured to water, and then extracted with ethyl acetate. The organic layer was washed sequentially with water and saturated brine, dried with anhydrous magnesium sulfate, and then concentrated *in vacuo* to yield Compound I-96 (123 mg, 74%) as a white powder.
LC/MS (Method A): 2.49 min, $[M+H]^+$ = 390.4.

Step 3

To a solution of Compound 1-96 (50.0 mg, 0.128 mmol) in tetrahydrofuran (0.50 mL) and methanol (0. 50 mL), 2 mol/L sodium hydroxide solution (0.26 mL, 0.514 mmol) was added. The solution was then stirred at 25°C for 2 hours. To the reaction solution, 0.5 mol/L citric acid was then added. The precipitated powder was filtered, and then washed with water. The resulting powder was further washed with ethyl acetate and hexane to yield Compound I-51 (32.5 mg, 67%) as a brown powder.
$^1$H-NMR (DMSO-$d_6$) δ13.11 (1H, br s), 12.59 (1H, s), 8.03-8.1 (4H, m), 7.69 (1H, d, J = 8.4 Hz), 7.52-7.56 (3H, m), 7.37 (2H, t, J = 8.7 Hz), 7.07 (1H, t, J - 7.2 Hz), 4.36 (2H, t, J = 4.8 Hz), 4.12 (2H, t, J = 4.8 Hz)
LC/MS (Method A) : 1.97 min, $[M+H]^+$ = 376.4.

(Example 2 Synthesis of Compounds I-113 and 1-144)

[Formula 36]

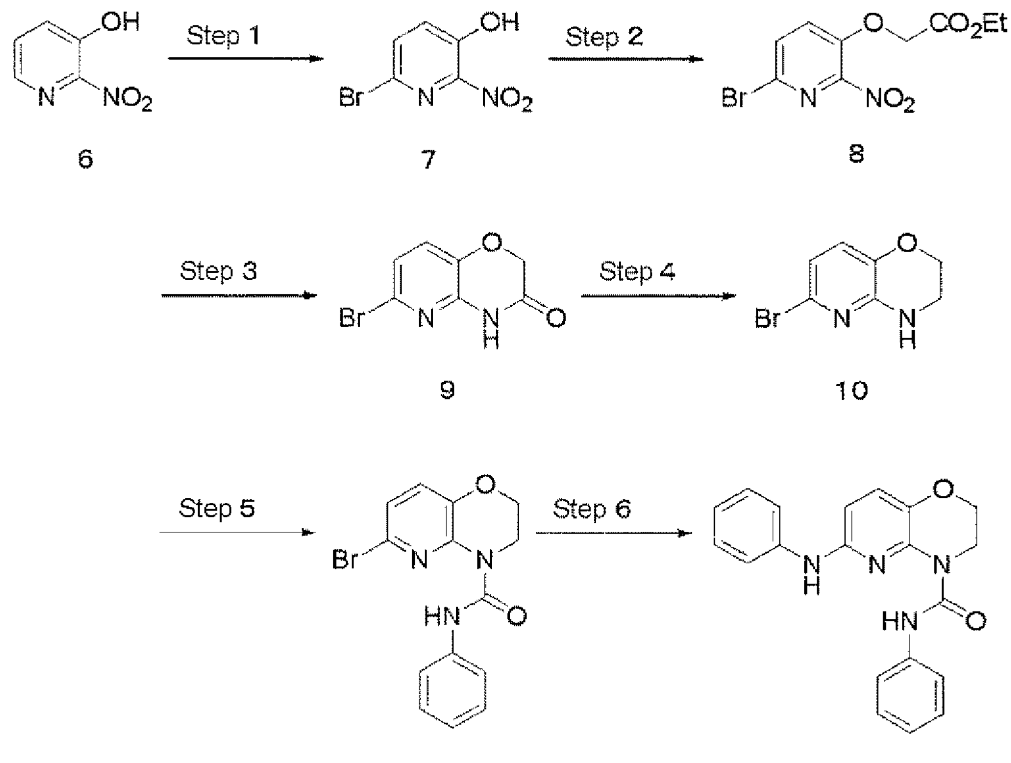


6

7

8

9

10

I-113

I-144

Step 1

A solution of 2-nitropyridin-3-ol (6) (16.0 g, 114 mmol) in dimethylformamide (120 mL) was cooled to 0˚C, and then N-bromosuccinimide (26.4 g, 1.48 mmol) was gradually added thereto. The solution was then stirred at 0 ˚C for 1 hour, and at room temperature for another 2 hours. The reaction solution was concentrated *in vacuo,* and then the residue was washed with diethyl ether. The filtrate was poured to aqueous saturated sodium bicarbonate, followed by extraction with diethyl ether. The organic layer was washed sequentially with water and saturated brine, then dried with anhydrous magnesium sulfate, and concentrated *in vacuo* to yield the subject compound (7) (15.5 g, 62%) as a white powder.

Step 2

To a solution of Compound (7) (6.80 g, 31.1 mmol) in dimethylformamide (80 mL), potassium carbonate (8.58 g, 62.1 mmol) and ethyl bromoacetate (5.2 mL, 46.6 mmol) were added. The solution was then stirred at 25˚C for 5 hours. Water was added to the reaction solution (100mL), and then separated with ethyl acetate. The organic layer was washed sequentially with water and saturated brine, then dried with anhydrous magnesium sulfate, and concentrated *in vacuo.* The residue was then purified by silica gel chromatography (hexane/ethyl acetate = 3/1). The resulting powder was further washed with ethyl acetate and hexane to yield the subject compound (8) (7.75 g, 77%) as a white powder. $^1$H-NMR (DMSO-$d_6$) δ7.92-8.04 (2H, m), 5.12 (2H, s), 4.13-4.20 (2H, m), 1.20 (3H, t, J = 7.2 Hz)
LC/MS (Method A) : 1.91 min, $[M+H]^+$ = 305.0.

Step 3

To a solution of Compound (8) (5.50 g, 18.0 mmol) in acetic acid (60 mL), iron powder (10.1 g, 180 mmol) was

added. The solution was then stirred at 100˚C for 2 hours. The reaction solution was filtered through a Celite pad, and then the filtrate was concentrated *in vacuo.* The residue was dissolved in ethyl acetate, washed with aqueous saturated sodium bicarbonate, dried with anhydrous magnesium sulfate, and then concentrated *in vacuo.* The resulting powder was further washed with ethyl acetate and hexane to yield the subject compound (9) (3.12 g, 76%) as a white powder.
$^1$H-NMR (DMSO-$d_6$) δ11.48 (1H, s), 7.31 (1H, d, J = 8.1 Hz), 7.16 (1H, d, J = 8.1 Hz), 4.66 (2H, s).

Step 4

To a solution of Compound (9) (3.12 g, 13.7 mmol) in tetrahydrofuran (50 mL), borane-tetrahydrofuran complex (33 mL, 33.0 mmol) was added at room temperature. The solution was then stirred at 90˚C for 1.5 hours. Methanol (2.0 mL) was added to the reaction solution, and then the mixture was stirred at 90˚C for 20 minutes. The reaction solution was concentrated *in vacuo*. The residue was dissolved in ethyl acetate, washed with aqueous saturated sodium bicarbonate, dried with anhydrous magnesium sulfate, and then concentrated *in vacuo* to yield subject compound (10) (3.17 g, 100%) as a yellow powder.
$^1$H-NMR (DMSO-$d_6$) 57.04 (1H, br s), 6.69 (1H, d, J = 7.8 Hz), 6.40 (1H, d, J = 7.8 Hz), 3.91 (2H, t, J = 4.2Hz), 3.19-3.23 (2H, m)
LC/MS (Method A): 1.44 min, $[M+H]^+$ = 215.2.

Step 5

To a solution of Compound (10) (2.50 g, 11.6 mmol) in dimethylformamide (20 mL), triethylamine (4.8 mL), 34.9 mmol) and phenylisocyanate (1.6 mL, 15.1 mmol) were added. The solution was then stirred at 25 ˚ C for 24 hours. Water (3.0 mL) was then added to the reaction solution. The precipitated powder was filtered, washed with water, and then dried to yield subject compound I-113 (3.4 g, 85%) as a pale brown powder.
$^1$H-NMR (DMSO-$d_6$) δ11.92 (1H, s), 7.52-7.55 (2H, m), 7.34-7.40 (3H, m), 7.28 (1H, dd, J = 8.4 Hz, J = 1.5 Hz), 4.30 (2H, t, J = 4.8 Hz), 4.04 (2H, t, J = 4.8 Hz)
LC/MS (Method A): 2.45 min, $[M+H]^+$ = 334.0.

Step 6

To a solution of Compound I-113 (100 mg, 0.299 mmol), tris(dibenzylideneacetone)dipalladium(0) (13.7 mg, 0.0150 mmol), 4,5-bis(diphenylphosphino)-9,9-dimethyl-xanthene (34.6 mg, 0.0600 mmol), and cesium carbonate (293 mg, 0.898 mmol) in toluene (3.0 mL), aniline (0.041 mL, 0.449 mmol) was added. After replacement with nitrogen, the solution was stirred at 100˚C for 8 hours. The reaction solution was then concentrated. The residue was then purified by silica gel chromatography (hexane/ethyl acetate = 3/1). The resulting powder was further washed with ethyl acetate and hexane to yield subject compound I-144 (49.3 mg, 45%) as a white powder.
$^1$H-NMR (DMSO-$d_6$) 612.00 (1H, s), 8.92 (1H, s), 7.28-7.32 (3H, m), 7.18-7.23 (2H, m), 7.07-7.12 (2H, m), 6.88-6.94 (4H, m), 6.53 (1H, d, J = 8.7 Hz), 4.19 (2H, t, J= 4.5 Hz), 4.00 (2H, t, J = 4.5 Hz)
LC/MS (Method A): 2.33 min, $[M+H]^+$ = 347.1.

(Example 3 Synthesis of Compounds 1-44, 1-43, I-119, and I-126)

[Formula 37]

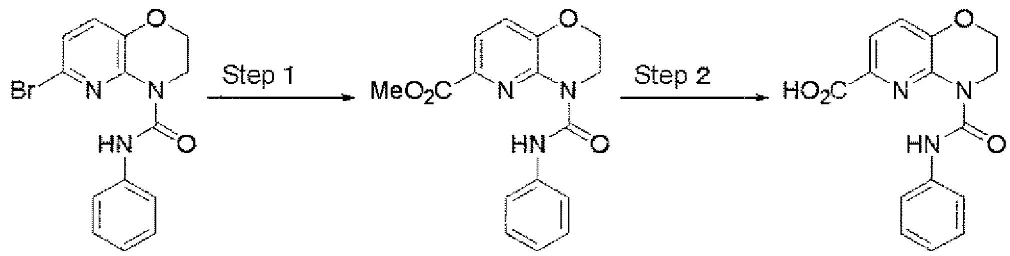


I−113 I−44 I−43

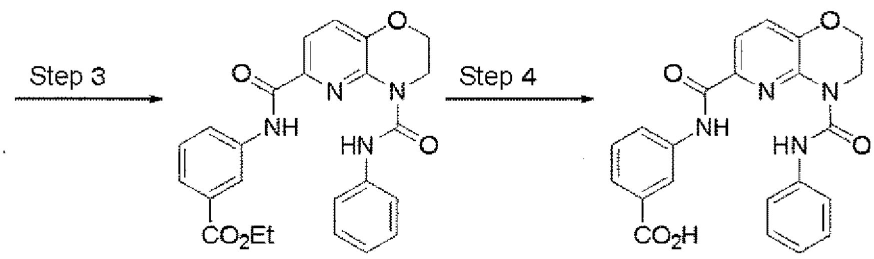


I−119 I−126

Step 1

To a solution of Compound I-113 (2.5 g, 7.48 mmol) in dimethylformamide (15 mL) and methanol (15 mL), diisopropylethylamine (5.2 mL, 29.9 mmol) and dichloro[1,1'-ferrocenylbis(diphenylphosphine)]palladiu m (II) dichloromethane (0.305 g, 0.374 mmol) were added. After replacement with carbon monoxide, the solution was stirred at 80˚C for 8 hours. Water was poured to the reaction solution, followed by separation with ethyl acetate. The organic layer was washed sequentially with water and saturated brine, then dried with anhydrous magnesium sulfate, and concentrated *in vacuo.* The resulting powder was further washed with ethyl acetate and hexane to yield subj ect compound 1-44 (2.23 g, 95%) as a brown powder.
$^1$H-NMR (DMSO-$d_6$) 513.16 (1H, s), 7.77 (1H, d, J = 8.1 Hz), 7.69-7.72 (2H, m), 7.551 (1H, d, J = 8.1 Hz), 7.35-7.41 (2H, m), 7.06 (1H, t, J = 7.2 Hz), 4.39 (2H, t, J = 5.1 Hz), 4.10 (2H, t, J = 5.1 Hz)
LC/MS (Method A): 2.14 min, $[M+H]^+$ = 314.2.

Step 2

To a solution of Compound 1-44 (500 mg, 1.60 mmol) in tetrahydrofuran (1.0 mL) and methanol (1.0 mL), 2 mol/L sodium hydroxide solution (4.0 mL, 7.98 mmol) was added. The solution was then stirred at 25˚C for 3 hours. 0.5 mol/L citric acid was then added to the reaction solution. The precipitated powder was filtered, washed with water, and then dried to yield subject compound 1-43 (440 mg, 92%) as a white powder.
$^1$H-NMR (DMSO-$d_6$) 613.43 (1H, br s), 13.31 (1H, s), 7.71-7.75 (3H, m), 7.49 (1H, d, J = 8.1 Hz), 7.31-7.36 (2H, m), 7.05 (1H, t, J = 7.2 Hz), 4.38 (2H, t, J = 4.8 Hz), 4.10 (2H, t, J = 4.8 Hz)
LC/MS (Method A) : 1.65 min, $[M+H]^+$ = 300.1.

Step 3

To a solution of Compound 1-43 (100 mg, 0.334 mmol) and 2-(1H-7-azabenzotriazol-1-yl)-1,1,3,3-tetramethyluroniu m hexafluorophosphate hexafluorophosphate salt (191 mg, 0.501 mmol) in dimethylformamide (1.0 mL), ethyl 3-aminobenzoate (0.065 mL, 0.434 mmol) and triethylamine (0.093 mL, 0.668 mmol) were added. The solution was then stirred at 25˚C for 4 hours. Water (2.0 mL) was then poured to the reaction solution. The precipitated powder was filtered, washed with water, and then dried. The resulting powder was further washed with ethyl acetate and hexane to yield subject compound I-119 (120 mg, 80%) as a white powder. $^1$H-NMR (DMSO-$d_6$) δ12.37 (1H, s), 10.74 (1H, s), 8.47 (1H, s), 8.12 (1H, d, J = 9.3 Hz), 7.80 (1H, d, J = 8.1 Hz), 7.74 (1H, d, J = 7.8 Hz), 7.50-7.65 (4H, m), 7.22-7.27 (2H, m), 7. 02 (1H, t, J = 7.5 Hz), 4.40 (2H, t, J = 4.8 Hz), 4.30-4.36 (2H, m), 4.12 (2H, t, J = 4.8 Hz), 1.33 (3H, t, J = 6.9

Hz) LC/MS (Method A): 2.37 min, $[M+H]^+$ = 447.3.

Step 4

To a solution of Compound I-119 (70.0 mg, 0.157 mmol) in methanol (1.0 mL) and tetrahydrofuran, (1.0 mL), 2 mol/L sodium hydroxide solution (0.39 mL, 0.784 mmol) was added. The solution was then stirred at 25°C for 5 hours. 0. 5 mol/L citric acid was then added to the reaction solution. The precipitated powder was filtered, washed with water, and then dried to yield subject compound 1-126 (61.9 mg, 94%) as a white powder.
$^1$H-NMR (DMSO-$d_6$) δ12.65 (1H, br s), 12.35 (1H, s), 10.70 (1H, s), 8.45 (1H, s), 8.08 (1H, d, J = 8.4 Hz), 7.80 (1H, d, J = 8.1 Hz), 7.71 (1H, d, J = 7.5 Hz), 7.48-7.64 (4H, m), 7.22-7.27 (2H, m), 7.01 (1H, t, J = 7.2 Hz), 4.40 (2H, t, J = 4.5 Hz), 4.12 (2H, t, J = 4.8 Hz)
LC/MS (Method A): 1.81 min, [M+Hl+ = 419.3.

(Example 4 Synthesis of Compounds I-112, I-108, I-120, and 1-124)

[Formula 38]

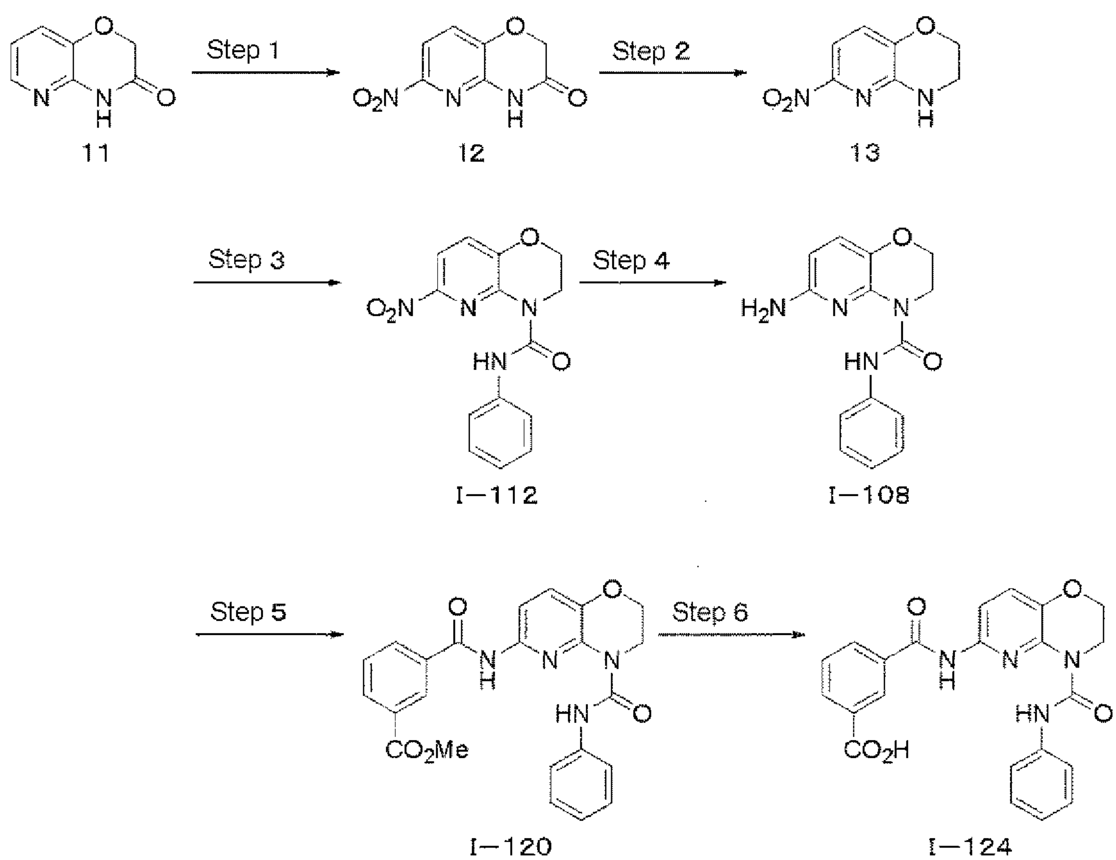


11 12 13

I-112 I-108

I-120 I-124

Step 1

To a commercially available compound (11) (5.00 g, 33.3 mmol) in concentrated sulfuric acid (16.5 mL), concentrated sulfuric acid (3.5 mL) and nitric acid (3.5 mL, 84.0 mmol) were added dropwise at room temperature over 15 minutes. The solution, as it is, was then stirred for 30 minutes. The reaction solution was then poured to ice. The precipitated powder was filtered, washed with water, and then dried to yield subject compound (12) (3.12 g, 48%) as a

pale yellow powder.
$^1$H-NMR (DMSO-$d_6$) 511.66 (1H, s), 7.73 (1H, d, J = 8.7 Hz), 7.38 (1H, d, J = 8.7 Hz), 4.60 (2H, s).

Step 2

To a solution of Compound (12) (1.00 g, 5.12 mmol) in tetrahydrofuran (8.0 mL), borane-tetrahydrofuran complex (33 mL, 33.0 mmol) was added at room temperature. The solution was then stirred at 90˚C for 4 hours. Methanol (2.0 mL) was added to the reaction solution, and then the solution was further stirred at 90˚C for 30 minutes. The reaction solution was then concentrated *in vacuo.* The residue was dissolved in ethyl acetate, washed with aqueous saturated sodium bicarbonate, dried with anhydrous magnesium sulfate, and then concentrated *in vacuo* to yield subject compound (13) (0.895 g, 96%) as a yellow powder.
$^1$H-NMR (DMSO-$d_6$) 57.58 (1H, s), 7.29 (1H, d, J = 8.4 Hz), 7.00 (1H, d, J = 8.4 Hz), 4.06 (2H, t, J = 4.2 Hz), 3.27-3.29 (2H, m).

Step 3

To a solution of Compound (13) (688 mg, 3.80 mmol) in dimethylformamide (5.0 mL), triethylamine (1.1 mL, 7.60 mmol) and phenylisocyanate (0.54 mL, 4.94 mmol) were added. The solution was then stirred at room temperature for 20 hours. Water (1.0 mL) was then added to the reaction solution. The precipitated powder was filtered, washed with water, and then dried to yield subject compound I-112 (810 mg, 47%) as a yellow powder.
$^1$H-NMR (DMSO-$d_6$) 512.30 (1H, s), 8.03 (1H, d, J = 9.0 Hz), 7.64-7.68 (3H, m), 7.35-7.50 (2H, m), 7.08 (1H, t, J = 6.6 Hz), 4.40 (2H, t, J = 4.5 Hz), 4.11 (2H, t, J = 4.5 Hz) LC/MS (Method A): 2.15 min, $[M+H]^+$ = 301.1.

Step 4

To a solution of Compound I-112 (530 mg, 1.77 mmol) in water (5.0 mL) and ethanol (10 mL), iron powder (197 mg, 3.53 mmol) and ammonium chloride (189 mg, 3.53 mmol) were added. The solution was then stirred at 110˚C for 2 hours. The reaction solution was filtered through a Celite pad, and then concentrated *in vacuo.* The residue was dissolved in ethyl acetate, washed with aqueous saturated sodium bicarbonate, dried with anhydrous magnesium sulfate, and then concentrated *in vacuo.* The resulting powder was further washed with ethyl acetate and hexane to yield subj ect compound I-108 (294 mg, 62%).
$^1$H-NMR (DMSO-$d_6$) 612.63 (1H, s), 7.68-7.71 (2H, m), 7.28-7.33 (2H, m), 7.11 (1H, d, J = 8.7 Hz), 7.03 (1H, t, J= 7.2 Hz), 6,17 (1H, d, J = 8.7 Hz), 6.00 (2H, s), 4.11 (2H, t, J = 4.5 Hz), 3.95 (2H, t, J = 4.5 Hz)
LC/MS (Method A): 1.70 min, $[M+H]^+$ = 271.3.

Step 5

To a solution of 3- (methoxycarbonyl) benzoic acid (52.0 mg, 0.289 mmol) and 2-(1H-7-azabenzotriazol-1-yl)-1,1,3,3-tetramethyluronium hexafluorophosphate hexafluorophosphate salt (127 mg, 0.333 mmol) in dimethylformamide (1.0 mL), Compound I-108 (60.0 mg, 0.222 mmol) and triethylamine (0.062 mL, 0.444 mmol) were added. The solution was then stirred at 25˚C for 5 hours. Water (5.0 mL) was then added to the reaction solution. The precipitated powder was filtered, washed with water, and then dried. The resulting powder was further washed with ethyl acetate and hexane to yield subject compound 1-120 (90.0 mg, 94%) as a white powder.
$^1$H-NMR (DMSO-d.$_6$) 612.11 (1H, s), 11.07 (1H br s) 8.56 (1H, s), 8.24 (1H, d, J=7.8Hz), 8.15 (1H, d, J = 7.8 Hz) 7.69-7.73 (3H, m), 7.43 (1H, d, J = 8.7 Hz), 7.27-7.33 (3H, m), 7.03 (1H, d, J = 7.2 Hz), 4.25 (2H, t, J = 4.2 Hz), 4.05 (2H, t, J = 4.2 Hz), 3.90 (3H, s)
LC/MS (Method A): 2.21 min, $[M+H]^+$ = 433.3.

Step 6

To a solution of Compound I-120 (60.0 mg, 0.139 mmol) in methanol (1.0 mL) and tetrahydrofuran (1.0 mL), 2 mol/L sodium hydroxide solution (0.35 mL, 0.694 mmol) was added. The solution was then stirred at 25˚C for 3 hours. 0.5 mol/L citric acid was then added to the reaction solution. The precipitated powder was filtered, washed with water, and then dried to yield subject compound I-124 (12.8 mg, 22%) as a white powder.
$^1$H-NMR (DMSO-$d_6$) δ13.2 (1H, brs), 12.05 (1H, s), 11.03 (1H, s), 8.56 (1H, s), 8.16-8.22 (2H, m), 7.68-7.73 (3H, m), 7.46 (1H, d, J = 8.7 Hz), 7.30-7.39 (3H, m), 7.05 (1H, t, J = 7.5 Hz), 4.28 (2H, t, J= 3.9 Hz), 4.07 (2H, t, J= 3.9 Hz).
LC/MS (Method A): 1.82 min, $[M+H]^+$=419.3.

(Example 5 Synthesis of Compounds I-114, I-110, and I-111)

[Formula 39]

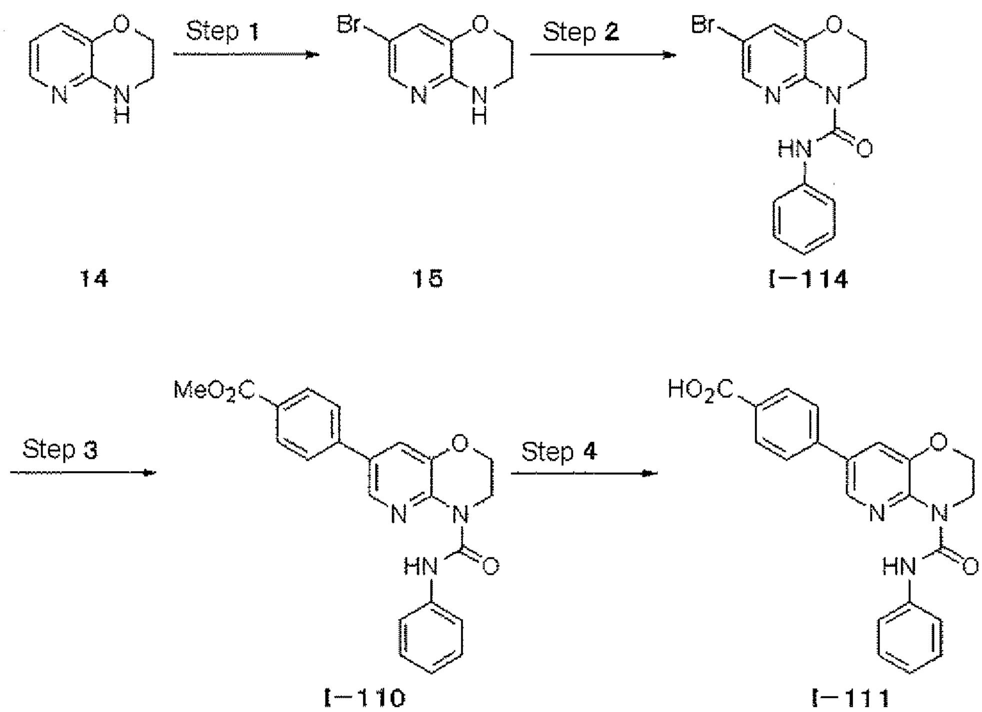


14 15 I-114

I-110 I-111

Step 1

To a solution of Compound (14) (4.00 g, 29.4 mmol) in dimethylformamide (40 mL), N-bromosuccinimide (6.27 g, 35.3 mmol) was added. The solution was then stirred at room temperature for 1 hour. Aqueous saturated sodium bicarbonate (100 mL) and water (100 mL) were then thereto. The precipitated powder was filtered, and then washed with water. The filtrate was then extracted with diisopropyl ether. The organic layer was washed sequentially with water and saturated brine, then dried with anhydrous magnesium sulfate, and concentrated *in vacuo* to yield subject compound (15) (1.79 g, 28%) as a pale yellow powder.
$^1$H-NMR (DMSO-$d_6$) δ7.62 (1H, d, J= 2.1Hz), 7.14 (1H, d, J =2.1Hz), 6.98 (1H, br s), 4.11 (2H, t, J=4.5Hz), 3.36-3.40 (2H, m)
LC/MS (Method A): 0.98 min, $[M+H]^+$= 217.0.

Step 2

To a solution of Compound (15) (1.75 g, 8.14 mmol) in dimethylformamide (18 mL), diisopropylethylamine (4.3 mL, 24.4 mmol) and phenylisocyanate (1.2 mL, 10.6 mmol) were added. The solution was then stirred at room temperature for 20 hours. Water (5.0 mL) was then added to the reaction solution. The precipitated powder was filtered, washed with water, and then dried to yield subject compound I-114 (1.97 mg, 73%) as a white powder.
$^1$H-NMR (DMSO-$d_6$) δ12.25 (1H, s), 8.19 (1H, d, J = 2.1 Hz), 7.71 (1H, d, J = 2.1 Hz), 7.60 (1H, d, J = 7.2 Hz), 7.34 (1H, t, J = 8.4 Hz) 7.06 (1H, t, J = 7.5 Hz), 4.31 (2H, t, J = 4.2 Hz), 4.05 (2H, t, J = 4.2 Hz) LC/MS (Method A): 2.45 min, $[M+H]^+$ = 336.1.

Step 3

To a solution of Compound 1-114 (100 mg, 0.299 mmol) and 4-(methoxycarbonyl)phenylboronic acid (81.0

mg, 0.449 mmol) in dimethylformamide (2.0 mL), tetrakis(triphenylphosphine)palladium (17.3 mg, 0.015 mmol) and 2 mol/L potassium carbonate solution (0.45 mL, 0.898 mmol) were added. The solution was then stirred at 100˚C for 3 hours. The reaction solution was poured to water, and then extracted with ethyl acetate. The organic layer was washed sequentially with water and saturated brine, then dried with anhydrous magnesium sulfate, and concentrated *in vacuo* to yield subject compound I-110 (47.2 mg, 41%) as a gray powder.
$^1$H-NMR (DMSO-$d_6$) δ12.72 (1H, s), 8.50 (1H, d, J = 1.8 Hz), 8.02-8.05 (2H, m), 7.88-7.91 (2H, m), 7.82 (1H, d, J= 1.8 Hz), 7.59-7.62 (2H, m), 7.31-7.36 (2H, m), 7.05 (1H, t, J = 7.2 Hz), 4.34 (2H, t, J = 4.5 Hz), 4.09 (2H, t, J = 4.5 Hz) LC/MS (Method A): 2.56 min, $[M+H]^+$= 390.2.

Step 4

To a solution of Compound I-110 (38.5 mg, 0.099 mmol) in methanol (1.0 mL) and tetrahydrofuran (1.0 mL), 2 mol/L sodium hydroxide solution (0.25 mL, 0.494 mmol) was added. The solution was then stirred at 25 ˚C for 4 hours. 0. 5 mol/L citric acid was then added to the reaction solution. The precipitated powder was filtered, washed with water, and then dried to yield subject compound I-111 (33.7 mg, 91%) as a white powder.
$^1$H-NMR (DMSO-$d_6$) δ13.02 (1H, br s), 12.75 (1H, s), 8.51 (1H, s), 8.03-8.05 (2H, m), 7.83-7.90 (3H, m), 7.62-7.65 (2H, m), 7.33-7.38 (2H, m), 7.08 (1H, t, J = 7.2 Hz), 4.36 (2H, br s), 4.11 (2H, br s)
LC/MS (Method A) : 2.07 min, $[M+H]^+$ = 376.2.

(Example 6 Synthesis of Compound I-132)

[Formula 40]

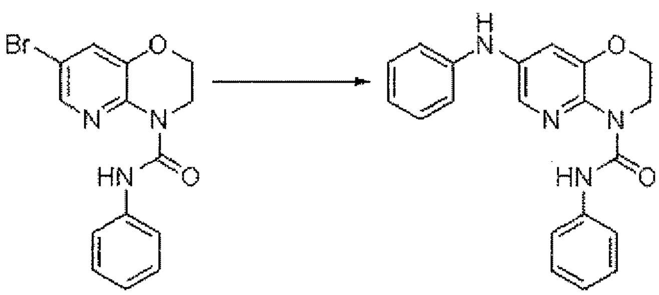

I-114 I-132

Step 1

To a solution of Compound I-114 (50.0 mg, 0.150 mmol), tris (dibenzylideneacetone) dipalladium (0) (3.43mg, 0.00374 mmol), 4,5-bis(diphenylphosphino)-9,9-dimethyl-xanthene (8.66 mg, 0.015 mmol), and cesium carbonate (146 mg, 0.449 mmol) in toluene (2.0 mL), aniline (0.041 mL, 0.449 mmol) was added. After replacement with nitrogen, the solution was stirred at 100˚C for 4 hours. The reaction solution was then concentrated. The residue was then purified by silica gel chromatography (hexane/ethyl acetate = 3/1). The resulting powder was further washed with ethyl acetate and hexane to yield subject compound 1-132 (10.8 mg, 21%) as a white powder.
$^1$H-NMR (DMSO-$d_6$) δ12.41 (1H, s), 8.26 (1H, s), 7.92 (1H, d, J=2.4 Hz), 7.59-5.61 (2H, m), 7.24-7.34 (4H, m), 7.06-7.10 (4H, m), 6.86 (1H, t, J = 7.2 Hz), 4.27 (2H, br s), 4.03 (2H, br s)
LC/MS (Method A): 2.42 min, $[M+H]^+$ = 347.4.

(Example 7 Synthesis of Compounds I-148, 1-160, and 1-161)

[Formula 41]

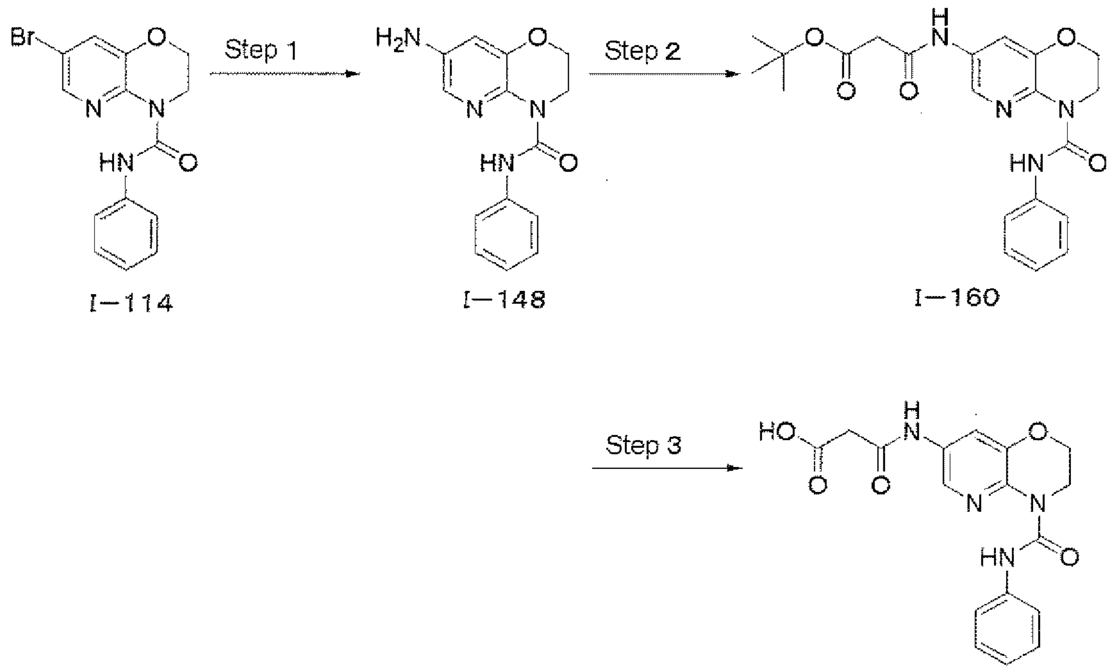


I-114 I-148 I-160

I-161

Step 1

To a solution of Compound I-114 (1.00 g, 2.99 mmol), tris(dibenzylideneacetone)dipalladium(0) (274 mg, 0.299 mmol), and 4,5-bis(dipheaylphosphino)-9,9-dimethyl-xanthene (693 mg, 1.20 mmol), and cesium carbonate (3.90g, 12.0 mmol) in toluene (5.0 mL), benzophenone imine (2.0 mL, 12.0 mmol) was added. After replacement with nitrogen, the solution was stirred at 100˚C for 6 hours. 2 mol/L hydrochloric acid (15 mL) and tetrahydrofuran (15 mL) were added to the reaction solution, which was then stirred at room temperature for 1 hour. Aqueous saturated sodium bicarbonate and ethyl acetate were added to the reaction solution, followed by separation and extraction. The organic layer was washed sequentially with water and saturated brine, then dried with anhydrous magnesium sulfate, and concentrated *in vacuo.* The resulting powder was further washed with ethyl acetate and hexane to yield subject compound I-148 (440 mg, 54%) as a pale yellow powder.
$^1$H-NMR (DMSO-$d_6$) δ12.33 (1H, s), 7.53-7.57 (2H, m), 7.44 (1H, d, J=2.1Hz), 7.28-7.33 (2H, m), 7.01 (1H, d, J=7.2Hz), 6.66 (1H, d, J = 2.1 Hz), 5.22 (2H, s), 4.20 (2H, t, J = 3.9 Hz), 3.97 (2H, t, J = 3.9 Hz) LC/MS (Method A): 1.57 min, $[M+H]^+$ = 271.4.

Step 2

To a solution of mono-tert-butyl malonate (0.034 mL, 0.222 mmol) and 2-(1H-7-azabenzotriazol-1-yl)-1,1,3,3-tetramethyluroniu m hexafluorophosphate hexafluorophosphate salt (91.0 mg, 0.240 mmol) in dimethylformamide (2.0 mL), Compound I-148 (50.0 mg, 0.185 mmol) and triethylamine (0.051 mL, 0.370 mmol) were added. The solution was then stirred at 25˚C for 24 hours. The reaction solution was poured to water, and then extracted with ethyl acetate. The organic layer was washed sequentially with water and saturated brine, then dried with anhydrous magnesium sulfate, and concentrated *in vacuo.* The residue was then purified by silica gel chromatography (hexane/ethyl acetate = 1/1). The resulting powder was further washed with ethyl acetate and hexane to yield subject compound I-160 (35.6mg, 47%) as a white powder. $^1$H-NMR (DMSO-$d_6$) δ12.41 (1H, s), 10.35 (1H, s), 8.29 (1H, d, J = 2.1 Hz), 7.61-7.63 (3H, m), 7.30-7.35 (2H, m), 7.05 (1H, d, J = 7.5 Hz), 4.29 (2H, t, J = 4.5 Hz), 3.97 (2H, t, J = 4.5 Hz), 3.38 (2H, s), 1.43 (9H, s) LC/MS (Method A): 2.15 min, $[M+HH]^+$ = 413.5.

Step 3

To a solution of Compound I-160 (30.0 mg, 0.073 mmol) in dichloromethane (0.30 mL), trifluoroacetic acid (0.30 mL, 3.86 mmol) was added. The solution was stirred at room temperature for 1 hour. Ethyl acetate and aqueous saturated sodium bicarbonate were added to the reaction solution, which was then separated and extracted. The organic layer was washed sequentially with water and saturated brine, then dried with anhydrous magnesium sulfate, and concentrated *in vacuo* to yield subject compound I-161 (9.90 mg, 38%) as a white powder.
$^1$H-NMR (DMSO-$d_6$) δ12.41 (1H, s), 10.43 (1H, s), 8.28 (1H, d, J= 2.1 Hz), 7.60-7.63 (3H, m), 7.30-7.35 (2H, m), 7.05 (1H, d, J = 708 Hz), 4.29 (2H, br s), 4.03 (2H, br s), 3.37 (2H. s)
LC/MS (Method A): 1.48 min, $[M+H]^+$ = 357.3.

(Example 8 Synthesis of Compounds I-267 and 1-257)

[Formula 42]

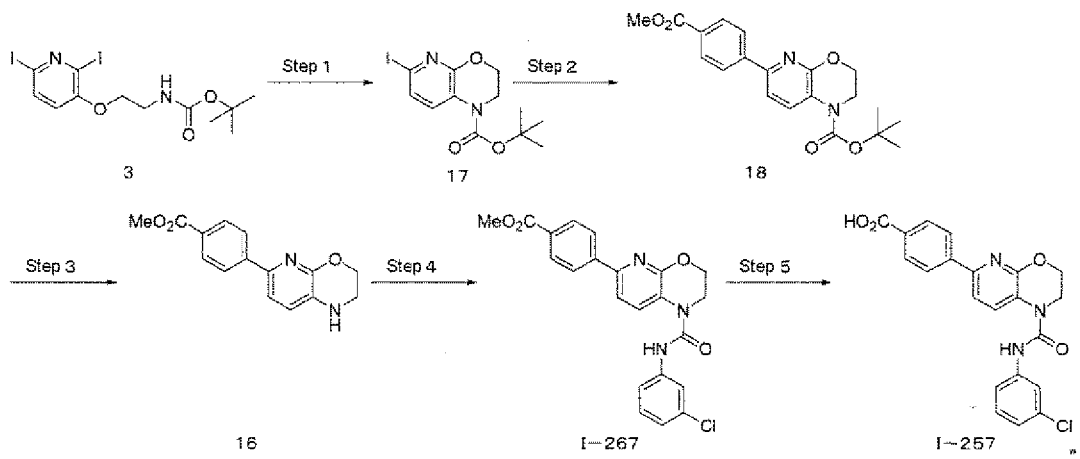


3 17 18 16 I-267 I-257

Step 1

A tetrahydrofuran (200 mL) suspension of sodium hydride (1.96 g, 49.0 mmol) was cooled to 0˚C, and then Compound (3) (20.0g, 40.8 mmol) was added thereto. The mixture was then stirred at room temperature for 1 hour. The reaction solution was then concentrated. Ethyl acetate (50 mL) and water (3.0 mL) were added thereto, followed by separation and extraction. The organic layer was washed sequentially with water and saturatedbrine, then dried with anhydrous magnesium sulfate, and concentrated *in vacuo* to yield subject compound (17) (2.28 mg, 15%) as a white powder.
$^1$H-NMR (DMSO-$d_6$) δ7.92 (1H, d, J = 8.1 Hz), 7.39 (1H, d, J = 8.1 Hz), 4.34 (2H, t, J= 4.8 Hz), 3.79 (2H, t, J= 4.8 Hz), 1.48 (9H, s).

Step 2

To a solution of Compound (17) (2.28 g, 6.30 mmol) and 4-(methoxycarbonyl)phenylboronic acid (1.47 g, 8.18 mmol) in dioxane (50 mL), tetrakis(triphenylphosphine)palladium (364 mg, 0.315 mmol) and 2 mol/L sodium carbonate solution (9.4 mL, 19.0 mmol) were added. The solution was then stirred at 100˚C for 6 hours. The reaction solution was poured to water, and then extracted with ethyl acetate. The organic layer was washed sequentially with water and saturated brine, then dried with anhydrous magnesium sulfate, and concentrated *in vacuo*. The residue was then purified by silica gel chromatography (hexane/ethyl acetate =2/1). The resulting powder was further washed with ethyl acetate and hexane to yield subject compound (18) (1.30 g, 56%) as a white powder. $^1$H-NMR (DMSO-$d_6$) δ8.36 (1H, d, J = 8.4 Hz), 8.14 (1H, d, J = 8.4 Hz), 8.03 (1H, d, J = 8.4 Hz), 7.71 (1H, d, J = 8.1 Hz), 4.39 (2H, t, J= 3.9 Hz), 3.87-3.88 (5H, m), 1.52 (9H, s)
LC/MS (Method A): 2.39 min, $[M+H]^+$ = 371.3.

Step 3

To a solution of Compound (18) (1.06 g, 2.86 mmol) in dichloromethane (5.0 mL), trifluoroacetic acid (1.1 mL, 14.3 mmol) was added. The solution was then stirred at room temperature for 2 hours. Ethyl acetate and aqueous saturated sodium bicarbonate was added to the reaction solution, which was then separated and extracted. The organic layer was washed sequentially with water and saturated brine, then dried with anhydrous magnesium sulfate, and concentrated *in vacuo* to yield subject compound (16) (706 mg, 91%) as a white powder.
$^1$H-NMR (DMSO-$d_6$) δ7.94-8.03 (4H, m), 7.49 (1H, d, J = 7.8 Hz), 6.97 (1H, d, J = 7.8 Hz), 6.44 (1H, s), 4.30 (2H, t, J = 3.6 Hz), 3.85 (3H, s), 3.26 (2H, t, J = 3.6 Hz) LC/MS (Method A): 1.60 min, $[M+H]^+$ = 271.3.

Step 4

To a solution of Compound (16) (5.00 mg, 0.018 mmol) in dimethylformamide (0.50 mL), 1-chloro-3-isocyanatebenzene (8.52 mg, 0.0550 mmol) and diisopropylethylamine (0.0032 mL, 0.018 mmol) were added. The solution was then stirred at 100˚C for 2 hours. The reaction solution was concentrated to yield subject compound I-267 as a crude product. LC/MS (Method A): 2.27 min, $[M+H]^+$ = 424.2.

Step 5

To a solution of Compound 1-267 in dimethylsulfoxide (0.50 mL), 2 mol/L sodium hydroxide solution (0.10 mL) was added. The solution was stirred at room temperature for 1 hour. Ion exchange resin DOWEX 50W-X4 was added to the reaction solution, which was then stirred at room temperature for 1 hour. The reaction solution was filtered, and then washed with dimethylsulfoxide. The resulting filtrate was concentrated to yield subject compound 1-257 (0.50 mg, 7%) as a white powder.
LC/MS (Method A): 1.80 min, $[M+H]^+$ = 410.2.

(Example 9 Synthesis of Compound 1-269)

[Formula 43]

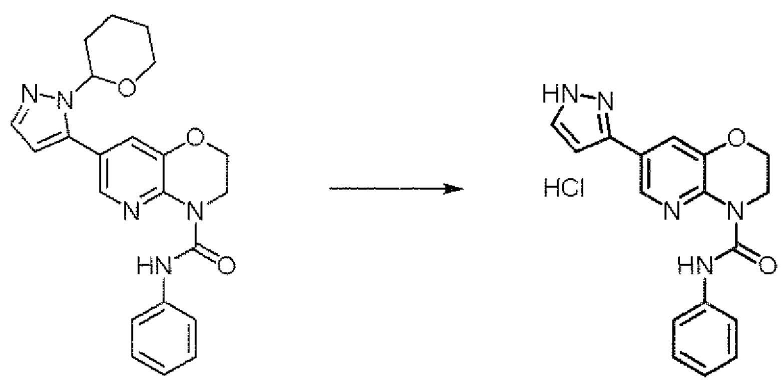

I-250 I-269

To a solution of Compound I-250 (40 mg, 0.10 mmol) in ethyl acetate (1.0 mL), hydrochloric acid (4 mol/L, ethyl acetate solution) (0.74 mL, 2.96 mmol) was added. The reaction solution was then stirred at room temperature for one and a half hours. The solvent was evaporated *in vacuo* to yield Compound 1-269 (38.0 mg).
LC/MS (Method A): 1.80 min, [M+] = 322.1
$^1$H-NMR (DMSO-$d_6$) δ: 1.2. 73 (1H, br s), 8.56 (1H, d, J = 1.3 Hz), 7.82 (2H, dd, J = 5.5,1.7 Hz), 7.65 (2H, d, J = 8.6 Hz), 7.38 (2H, t, J= 7.6 Ha), 7.09 (1H, t, 7.0 Hz), 6.84 (1H, d, J = 2.3 Hz), 4.36 (2H, t, J = 4.4 Hz), 4.12 (2H, t, J = 4.4 Hz).
(Example 9' Synthesis of Compounds I-1 to 41, 45 to 50, 52 to 95, 97 to 107, 109, 115 to 118, 121 to 123, 125, 127 to 131, 133 to 143, 145 to 147, 149 to 159, 162 to 252, 255, 256, 258 to 266, 268, 270 to 337)
Compounds I-1 to 41, 45 to 50, 52 to 95, 97 to 107, 109, 115 to 118, 121 to 123, 125, 127 to 131, 133 to 143, 145 to

147, 149 to 159, 162 to 252, 255, 256, 258 to 266, 268, 270 to 337 were synthesized similarly to Examples above.

(Results)

The following tables show values of the physical properties (retention time, mass spectrum or NMR spectrum, and measurement condition) for Compound Nos. I-1 to 252 and 255 to 337. In a column of MS [M-H]+ of the tables, when (Na+) is described next to a numerical value, it indicates the mass measured as a sodium ion adduct ([M+Na]+).
[Table 1-1]

| Compound No. | Structure | LC/MS Retention Time (min) or NMR Spectrum | MS[M+H]+ | LC Method |
|---|---|---|---|---|
| I-1 | 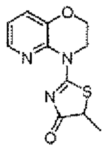 | 1.37 | 250.5 | A |
| I-2 | 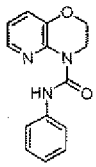 | 2.10 | 256.4 | A |
| I-3 | 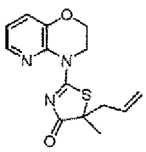 | 1.78 | 290.5 | A |
| I-4 | 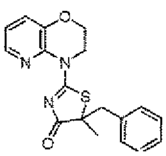 | 2.00 | 340.6 | A |
| I-5 | 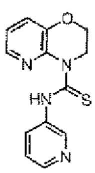 | 1H-NMR($CDCl_3$): δ4.37(2H, dd, J= 4.8, 1.5Hz), 4.85(2H, dd, J= 7.8. 4.8Hz), 7.9B(1H, dd, J=1.5. 8.1Hz). 7.32-7.39 (2H, m), 7.94(1H, dd, J=1.8, 4.8H2), 8.18(1H, m), 8.47(1H, dd, J= 1.5, 4.8Hz), 6.64(1H, d. J=2.4Hz) | | |
| I-6 | 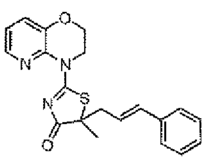 | 2.22 | 366.6 | A |
| I-7 | 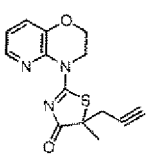 | 1.62 | 288.5 | A |

[Table 1-2]

| Compound No. | Structure | LC/MS Retention Time (min) or NMR Spectrum | MS[M+H]+ | LC Method |
|---|---|---|---|---|
| I-8 | 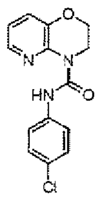 | 2.38 | 290.5 | A |
| I-9 | 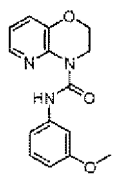 | 2.11 | 286.5 | A |
| I-10 | 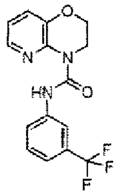 | 2.46 | 324.5 | A |
| I-11 | 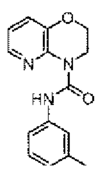 | 2.29 | 270.6 | A |
| I-12 | 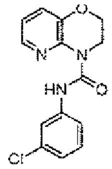 | 2.40 | 290.6 | A |
| I-13 | 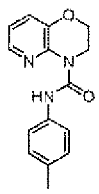 | 2.28 | 270.6 | A |
| I-14 | 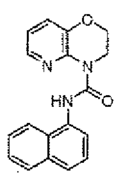 | 2.41 | 306.6 | A |

[Table 1-3]

| Compound No. | Structure | LC/MS Retention Time (min) or NMR Spectrum | MS[M+H]+ | LC Method |
|---|---|---|---|---|
| I-15 | 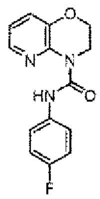 | 2.15 | 274.5 | A |
| I-16 | 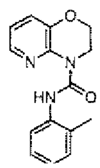 | 2.24 | 270.6 | A |
| I-17 | 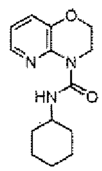 | 2.19 | 262.4 | A |
| I-18 | 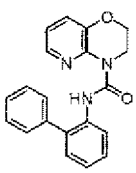 | 2.52 | 332.6 | A |
| I-19 | 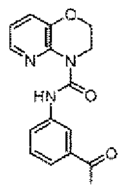 | 1.95 | 298.6 | A |
| I-20 | 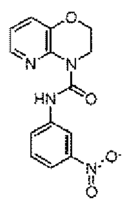 | 2.19 | 301.5 | A |
| I-21 | 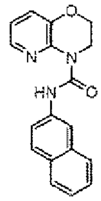 | 2.45 | 306.6 | A |

[Table 1-4]

| Compound No. | Structure | LC/MS Retention Time (min) or NMR Spectrum | MS [M+H]+ | LC Method |
|---|---|---|---|---|
| I-22 | 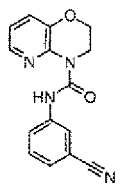 | 2.06 | 281.6 | A |
| I-23 | 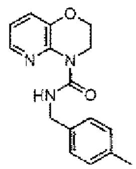 | 2.15 | 284.6 | A |
| I-24 | 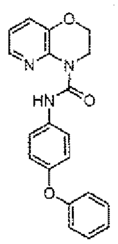 | 2.56 | 348.6 | A |
| I-25 | 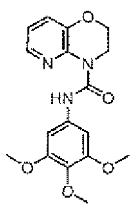 | 1.92 | 346.6 | A |
| I-26 | 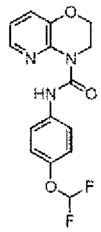 | 2.22 | 322.6 | A |
| I-27 | 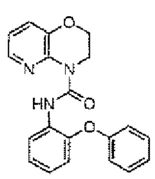 | 2.56 | 348.6 | A |

[Table 1-5]

| Compound No. | Structure | LC/MS Retention Time (min) or NMR Spectrum | MS[M+H]+ | LC Method |
|---|---|---|---|---|
| I-28 | 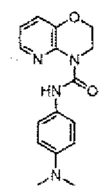 | | 299.6 | A |
| I-29 | 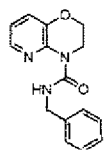 | 1.98 | 270.6 | A |
| I-30 | 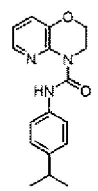 | 2.59 | 298.6 | A |
| I-31 | 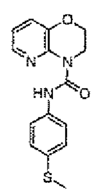 | 2.28 | 302.3 | A |
| I-32 | 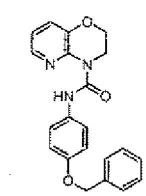 | 2.58 | 362.7 | A |
| I-33 | 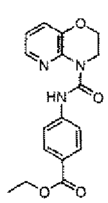 | 1H-NMR(CDCl3): δ1.40(3H, t, J= 6.9Hz), 4.18-4.30(4H, m), 4.36(2H, q. J=6.9Hz). 6.98(1H, dd, J= 6.4. 7.8Hz), 7.27(1H dd, J= 1.5. 7.8Hz). 7.67(2H, d, J= 6.9Hz) 79;(1H, dd, J= 1.5, 10.8Hz), 8.01(2H. d, J= 6.9Hz) | | |

[Table 1-6]

| Compound No. | Structure | LC/MS Retention Time (min) or NMR Spectrum | MS[M+H]+ | LC Method |
|---|---|---|---|---|
| I-34 | 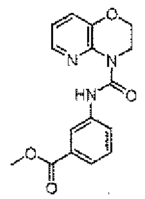 | 2.12 | 314.6 | A |
| I-35 | 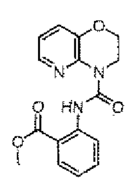 | 1H-NMR(CDCl3): $\delta$3.92(1H, s), 4.19- 4.22(2H, m), 4.28- 4.32(2H, m), 6.99(1H, dd, J=2.1.8.7 Hz), 7001(1H. m), 7.24(1H, d, J= 2.1Hz), 7.49(1H, m). 7.97(1H, dd, J= 1.8, 8.1Hz), 8.09(1H dd, J= 1.8, 4.8Hz), 8.42(1H, dd, J= 12, 87Hz) | | |
| I-36 | 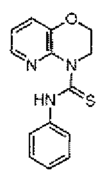 | 2.25 | 272.6 | A |
| I-37 | 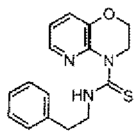 | 2.43 | 300.6 | A |
| I-38 | 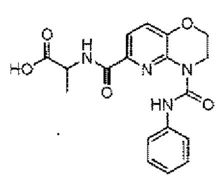 | 1.50 | 371.3 | A |
| I-39 | 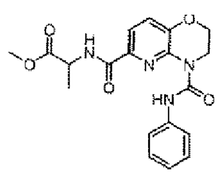 | 1.32 | 407.04(Na+) | A |
| I-40 | 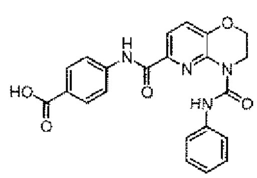 | 1.76 | 419.3 | A |

[Table 1-7]

| Compound No. | Structure | LC/MS Retention Time (min) or NMR Spectrum | MS[M+H]+ | LC Method |
|---|---|---|---|---|
| I-41 | 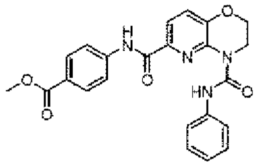 | 1.52 | 454.91(Na+) | A |
| I-42 | 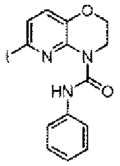 | 2.51 | 382-1 | A |
| I-43 | 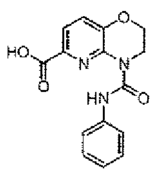 | 1.65 | 300.1 | A |
| I-44 | 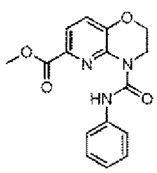 | 2.14 | 314.2 | A |
| I-45 | 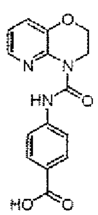 | 1H-NMR(DMSO-d6): δ4.06(2H, dd. J= 4.8. 9.0Hz), 4.31(2H, dd, J= 3.9. 9.0Hz), 7.12(1H, dd, J= 4.8, 8.1 Hz), 7.43(1H, dd, J= 1.5, 8.1Hz), 7.73 (2H, d, J= 6.0Hz), 7.91(2H, d, J= 6.6Hz), 8.08(1H, dd, J= 1.5. 5.1Hz) | | |
| I-46 | 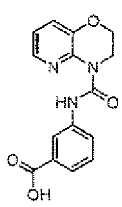 | 1H-NMR(DMSO-d6): δ 4.05-4.09 (2H, m), 4.28-4.32(2H, m), 7.11(1H, dd, J= 4.8, 7.8Hz). 7.41-7.49(1H, m), 7.63(1H, m), 7.77(1H, m), 8.08 (1H, dd, J= 1.8, 48Hz), 8.26(1H, s), 12.90(1H, s) | | |

[Table 1-8]

| Compound No. | Structure | LC/MS Retention Time (min) or NMR Spectrum | MS[M+H]+ | LC Method |
|---|---|---|---|---|
| I-47 | 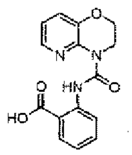 | 1H-NMR(DMSO-d6): δ4.06-4.09(2H, m), 428-4.32(2H, ml, 7.07-7.13(2H, m), 7.38 (1H, dd, J= 1.8. 8, 1Hz), 7.55(1H, m), 7.90 (1H, dd, J= 1.8, 7.8Hz), 7.98(1H, dd, J= 1.5,4.8Hz), 8.41(1H, d. J=8.4Hz), 13.30 (1H, s), 13.41(1H, s) | | |
| I-48 | 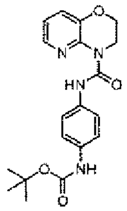 | 2.27 | 371.7 | A |
| I-49 | 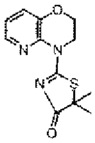 | 1.56 | 264.4 | A |
| I-50 | 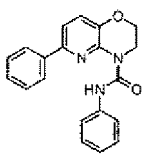 | 2.62 | 332.4 | A |
| I-51 | 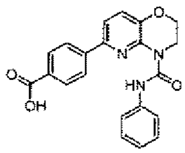 | 1.97 | 376.4 | A |
| I-52 | 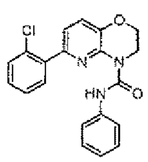 | 2.67 | 376.4 | A |
| I-53 | 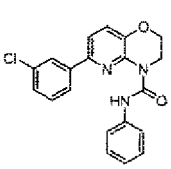 | 2.76 | 376.4 | A |

[Table 1-9]

| Compound No. | Structure | LC/MS Retention Time (min) or NMR Spectrum | MS[M+H]+ | LC Method |
|---|---|---|---|---|
| I-54 | 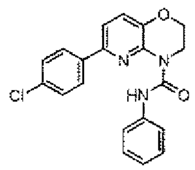 | 2.61 | 376.4 | A |
| I-55 | 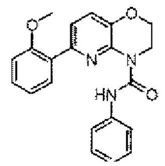 | 2.53 | 362.4 | A |
| I-56 | 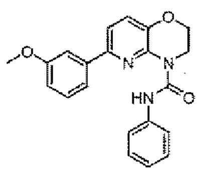 | 2.58 | 362.3 | A |
| I-57 | 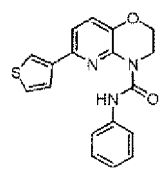 | 2.52 | 338.3 | A |
| I-58 | 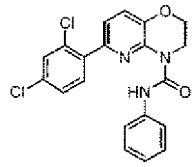 | 2.86 | 400.3 | A |
| I-59 | 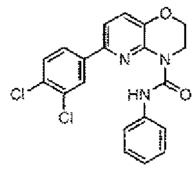 | | | |
| I-60 | 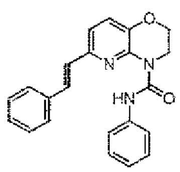 | 2.76 | 358.4 | A |

[Table 1-10]

| Compound No. | Structure | LC/MS Retention Time (min) or NMR Spectrum | MS[M+H]+ | LC Method |
|---|---|---|---|---|
| I-61 | 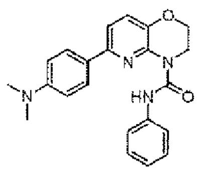 | 2.63 | 375.3 | A |
| I-62 | 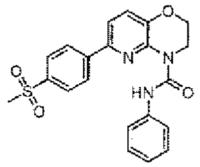 | 2.02 | 410.4 | A |
| I-63 | 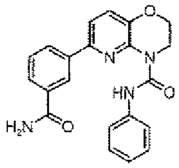 | 1.76 | 375.3 | A |
| I-64 | 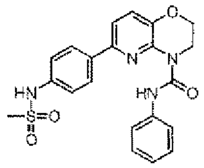 | 1.99 | 425.3 | A |
| I-65 | 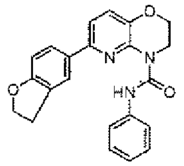 | 2.57 | 374.4 | A |
| I-66 | 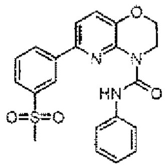 | 2.06 | 410.3 | A |
| I-67 | 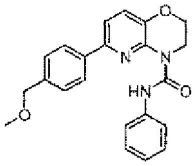 | 2.53 | 376.3 | A |

[Table 1-11]

| Compound No. | Structure | LC/MS Retention Time (min) or NMR Spectrum | MS[M+H]+ | LC Method |
|---|---|---|---|---|
| I-68 | 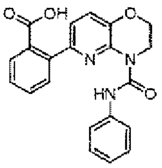 | 1.97 | 376.4 | A |
| I-69 | 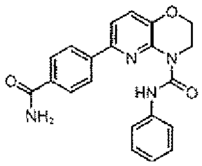 | 1.78 | 375.3 | A |
| I-70 | 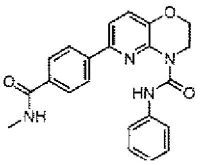 | 1.85 | 389.4 | A |
| I-71 | 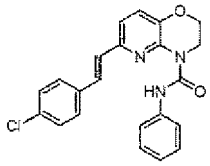 | 2.94 | 389.4 | A |
| I-72 | 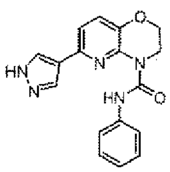 | 1.68 | 322.2 | A |
| I-73 | 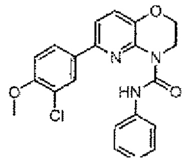 | 2.69 | 396.4 | A |
| I-74 | 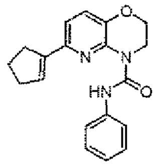 | 2.72 | 322.4 | A |

[Table 1-12]

| Compound No. | Structure | LC/MS Retention Time (min) or NMR Spectrum | MS[M+H]+ | LC Method |
|---|---|---|---|---|
| I-75 | 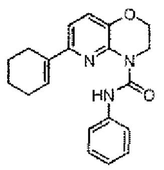 | 2.89 | 336.4 | A |
| I-76 | 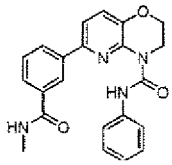 | 1.92 | 389.3 | A |
| I-77 | 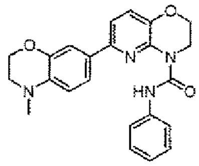 | 2.56 | 403.4 | A |
| I-78 | 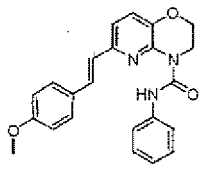 | 2.70 | 388.4 | A |
| I-79 | 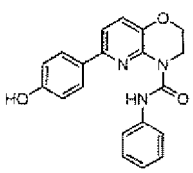 | 2.06 | 348.3 | A |
| I-80 | 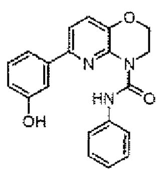 | 2.08 | 348.4 | A |
| I-81 | 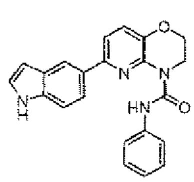 | 2.32 | 371.3 | A |

[Table 1-13]

| Compound No. | Structure | LC/MS Retention Time (min) or NMR Spectrum | MS[M+H]+ | LC Method |
|---|---|---|---|---|
| I-82 | 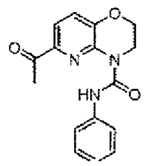 | 1.96 | 298.4 | A |
| I-83 | 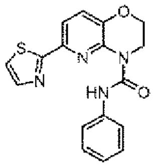 | 2.18 | 339.4 | A |
| I-84 | 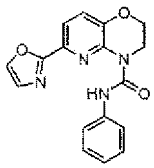 | 2.10 | 323.4 | A |
| I- 85 | 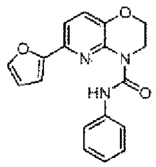 | 2.43 | 322.4 | A |
| I-86 | 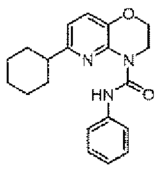 | 2.94 | 338.4 | A |
| I-87 | 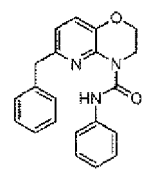 | 2.56 | 346.4 | A |
| I-88 | 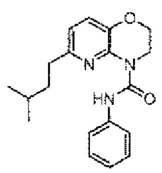 | 2.88 | 326.5 | A |

[table 1-14]

| Compound No. | Structure | LC/MS Retention Time (min) or NMR Spectrum | MS[M+H]+ | LC Method |
|---|---|---|---|---|
| I-89 | 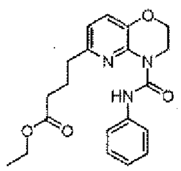 | 2.41 | 370.5 | A |
| I-90 | 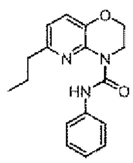 | 2.60 | 298.4 | A |
| I-91 | 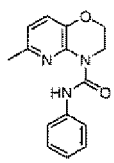 | 2.29 | 270.2 | A |
| I-92 | 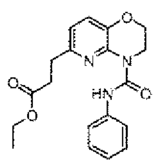 | 2.24 | 356.5 | A |
| I-93 | 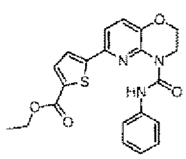 | 2.57 | 410.3 | A |
| I-94 | 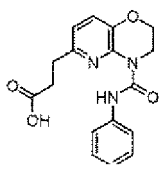 | 1.70 | 328.2 | A |
| I-95 | 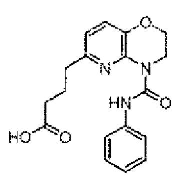 | 1.79 | 342.3 | A |

[Table 1-15]

| Compound No. | Structure | LC/MS Retention Time (min) or NMR Spectrum | MS[M+H]+ | LC Method |
|---|---|---|---|---|
| I-96 | 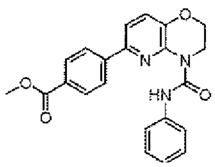 | 2.49 | 390.4 | A |
| I-97 | 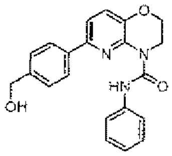 | 1.97 | 362.3 | A |
| I-98 | 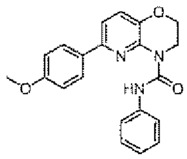 | 2.54 | 362.3 | A |
| I-99 | 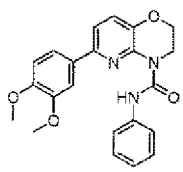 | 2.36 | 392.2 | A |
| I-100 | 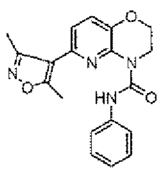 | 2.17 | 351.2 | A |
| I-101 | 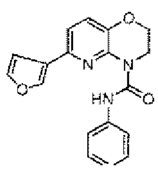 | 2.34 | 322.3 | A |
| I-102 | 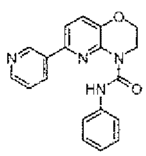 | 1.67 | 333.2 | A |

[Table 1-16]

| Compound No. | Structure | LC/MS Retention Time (min) or NMR Spectrum | MS[M+H]+ | LC Method |
|---|---|---|---|---|
| I-103 | 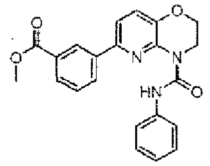 | 2.50 | 390.4 | A |
| I-104 | 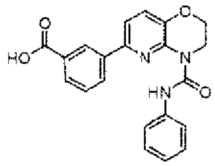 | 2.02 | 378.2 | A |
| I-105 | 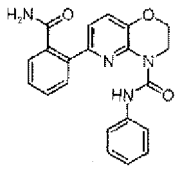 | 1.75 | 375.3 | A |
| I-106 | 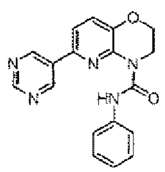 | 1.76 | 334.2 | A |
| I-107 | 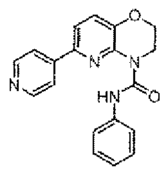 | 1.32 | 333.3 | A |
| I-108 | 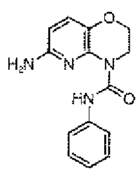 | 1.70 | 277.3 | A |
| I-109 | 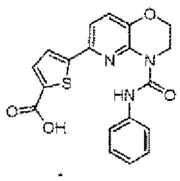 | 1.95 | 382.2 | A |

[Table 1-17]

| Compound No | Structure | LC/MS Retention Time (LC/MS) or NMR Spectrum | MS[M+H]+ | LC Method |
|---|---|---|---|---|
| 1-110 | 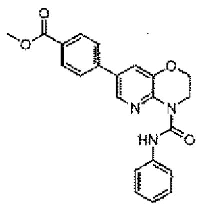 | 256 | 390.2 | A |
| I-111 | 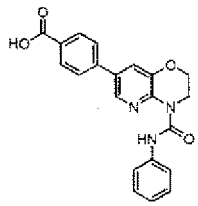 | 2.06 | 376.2 | A |
| I-112 | 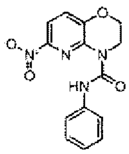 | 2.13 | 301.2 | A |
| I-113 | 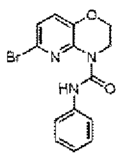 | 2.45 | 335.9 | A |
| I-114 | 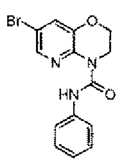 | 2.46 | 336.2 | A |
| I-115 | 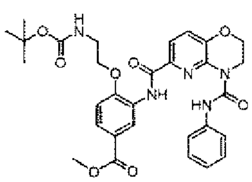 | 2.42 | 592.6 | A |
| I-116 | 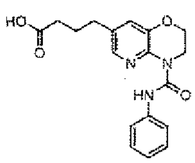 | 1.87 | 342.3 | A |

[Table 1-18]

| Compound No. | Structure | LC/MS Retention Time (min) or NMR Spectrum | MS[M+H]+ | LC Method |
|---|---|---|---|---|
| I-117 | 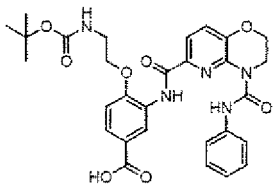 | 2.03 | 578.3 | A |
| I-118 | 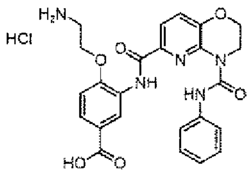 | 0.94 | 478.2 | A |
| I-119 | 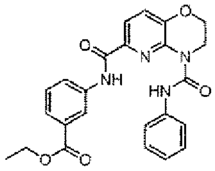 | 2.37 | 447.3 | A |
| I-120 | 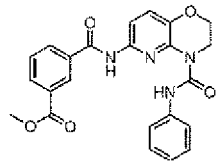 | 2.21 | 433.3 | A |
| I-121 | 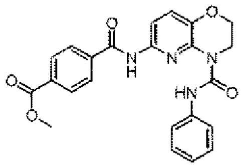 | 2.18 | 433.3 | A |
| I-122 | 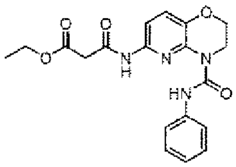 | 1.92 | 385.3 | A |
| I-123 | 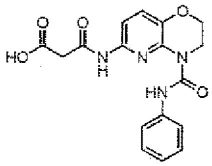 | 1.48 | 357.3 | A |

[Table 1-19]

| Compound No. | Structure | LC/MS Retention Time (min) or NMR Spectrum | MS[M+H]+ | LC Method |
|---|---|---|---|---|
| I-124 | 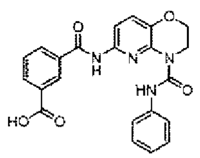 | 1.82 | 419.3 | A |
| I-125 | 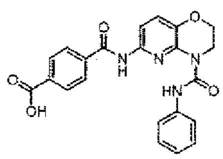 | 1.79 | 419.4 | A |
| I-126 | 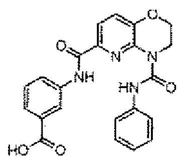 | 1.81 | 419.3 | A |
| I-127 | 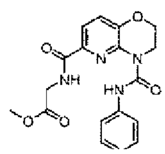 | 1.68 | 371.1 | A |
| I-128 | 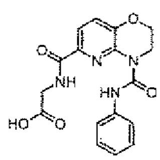 | 1.39 | 357.1 | A |
| I-129 | 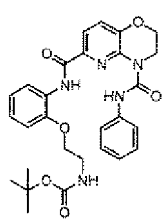 | 2.45 | 534.3 | A |
| I-130 | 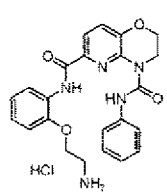 | 1.15 | 434.1 | A |

[Table 1-20]

| Compound No. | Structure | LC/MS Retention Time (min) or NMR Spectrum | MS[M+H]+ | LC Method |
|---|---|---|---|---|
| I-131 | 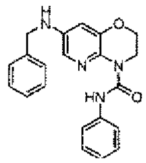 | 2.39 | 361.4 | A |
| I-132 | 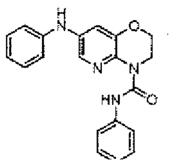 | 2.42 | 347.4 | A |
| I-133 | 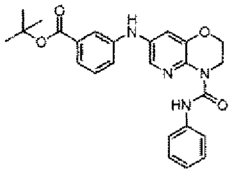 | 2.75 | 447.4 | A |
| I-134 | 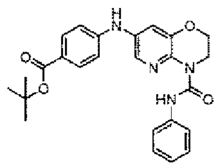 | 2.71 | 447.4 | A |
| I-135 | 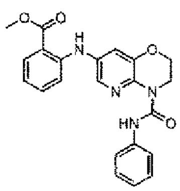 | 2.70 | 405.4 | A |
| I-136 | 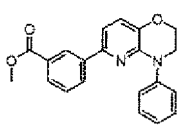 | 2.57 | 347.4 | A |
| I-137 | 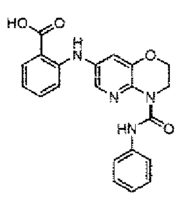 | 2.23 | 391.4 | A |

[Table 1-21]

| Compound No. | Structure | LC/MS Retention Time (min) or NMR Spectrum | MS[M+H]+ | LC Method |
|---|---|---|---|---|
| I-138 | 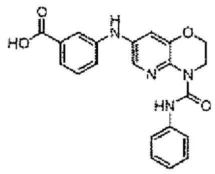 | 1.94 | 391.5 | A |
| I-139 | 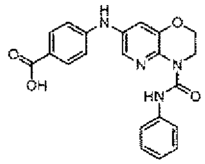 | 1.87 | 391.5 | A |
| I-140 | 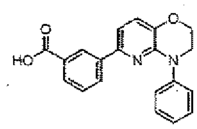 | 2.10 | 333.4 | A |
| 1-141 | 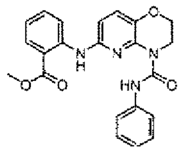 | 2.65 | 405.1 | A |
| I-142 | 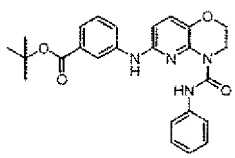 | 2.59 | 447.2 | A |
| I-143 | 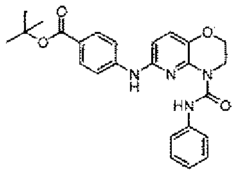 | 2.59 | 447.2 | A |
| I-144 | 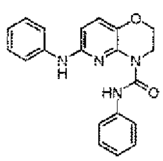 | 2.33 | 347.1 | A |

[Table 1-22]

| Compound No. | Structure | LC/MS Retention Time (min) or NMR Spectrum | MS[M+H]+ | LC Method |
|---|---|---|---|---|
| I-145 | 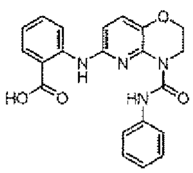 | 2.16 | 391.3 | A |
| I-146 | 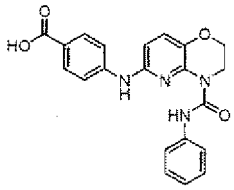 | 1.77 | 391.2 | A |
| I-147 | 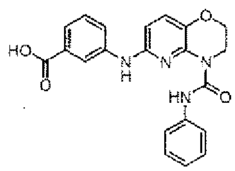 | 1.84 | 391.1 | A |
| I-148 | 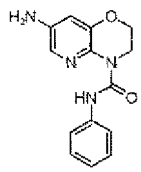 | 1.57 | 271.4 | A |
| I-149 | 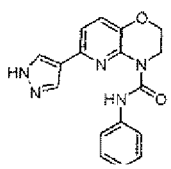 | 1.68 | 322.2 | A |
| I-150 | 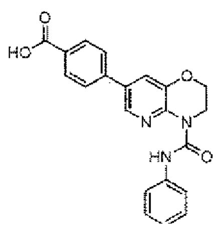 | 2.06 | 376.2 | A |
| I-151 | 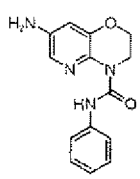 | 1.57 | 271.4 | A |

[Table 1-23]

| Compound No. | Structure | LC/MS Retention Time (min) or NMR Spectrum | MS[M+H]+ | LC Method |
|---|---|---|---|---|
| I-152 | 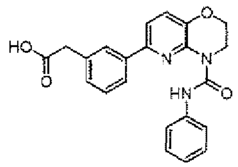 | 2.03 | 390.4 | A |
| I-153 | 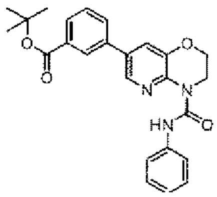 | 2.98 | 432.5 | A |
| I-154 | 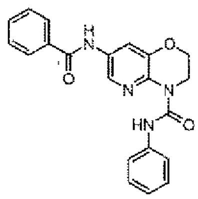 | 2.15 | 375.4 | A |
| I-155 | 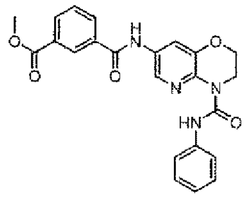 | 2.21 | 433.4 | A |
| I-156 | 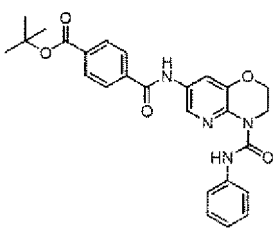 | 2.60 | 475.5 | A |
| I-157 | 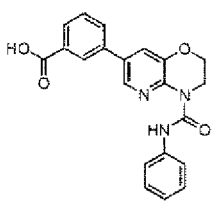 | 2.11 | 376.3 | A |

[Table 1-24]

| 化合物No | Structure | LC/MS Retention Time (min) or NMR Spectrum | MS[M+H]+ | LC Method |
|---|---|---|---|---|
| I-158 | 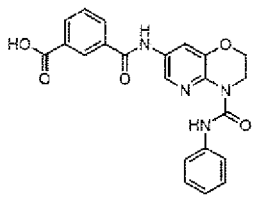 | 1.85 | 419.3 | A |
| I-159 | 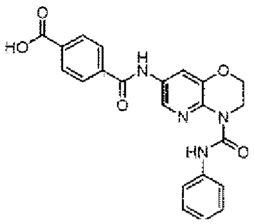 | 1.84 | 419.4 | A |
| I160 | 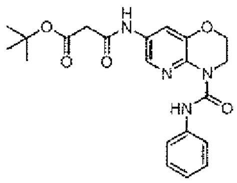 | 2.15 | 413.5 | A |
| I-161 | 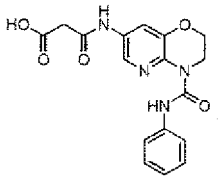 | 1.48 | 357.3 | A |
| I-162 | 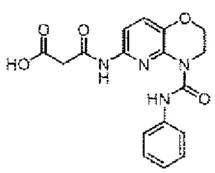 | 1.48 | 357.3 | A |
| I-163 | 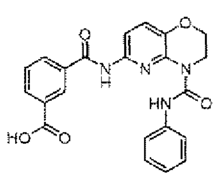 | 1.82 | 419.3 | A |

[Table 1-25]

| Compound No | Structure | LC/MS Retention Time (min) or NMR Spectrum | MS[M+H]+ | LC Method |
|---|---|---|---|---|
| I-154 | 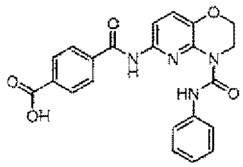 | 1.79 | 419.4 | A |
| I-165 | 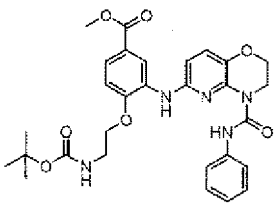 | 2.41 | 564.5 | A |
| I-166 | 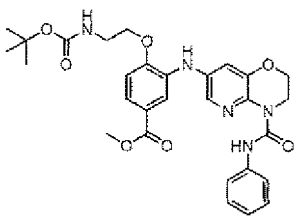 | 2.53 | 564.2 | A |
| I-157 | 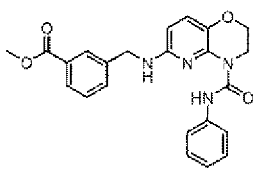 | 1.81 | 421.1 | A |
| I-168 | 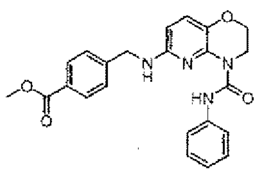 | 1.15 | 406.1 | A |
| I-169 | 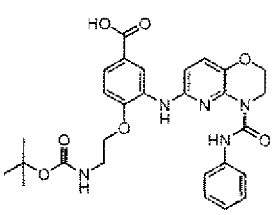 | 2.06 | 550.7 | A |

[Table 1-26]

| Compound No. | Structure | LC/MS Retention Time (min) or NMR Spectrum | MS[M+H]+ | LC Method |
|---|---|---|---|---|
| I-170 | 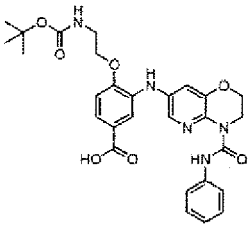 | 2.18 | 550.6 | A |
| I-171 | 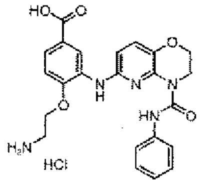 | 0.86 | 450.6 | A |
| I-172 | 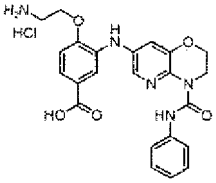 | 1.02 | 450.5 | A |
| I-173 | 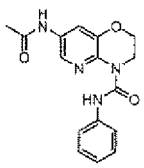 | 1.62 | 313.4 | A |
| I-174 | 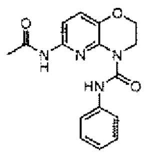 | 1.62 | 313.3 | A |
| I-175 | 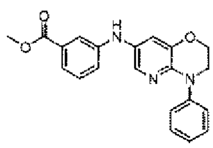 | 1.84 | 362.5 | A |
| I-176 | 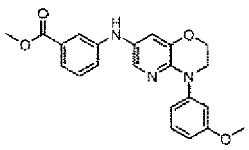 | 1.86 | 392.5 | A |

[Table 1-27]

| Compound No. | Structure | LC/MS Retention Time (min) or NMR Spectrum | MS[M+H]+ | LC Method |
|---|---|---|---|---|
| I-177 | 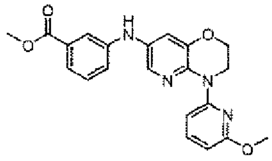 | 1.94 | 393.5 | A |
| I-178 | 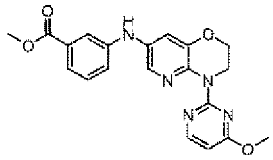 | 1.22 | 394.5 | A |
| I-179 | 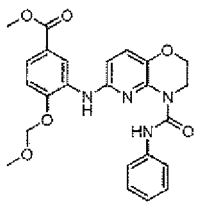 | 2.20 | 465.5 | A |
| I-180 | 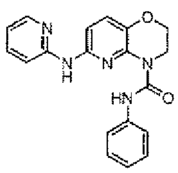 | 1.14 | 348.4 | A |
| I-181 | 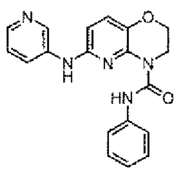 | 1.08 | 348.4 | A |
| I-182 | 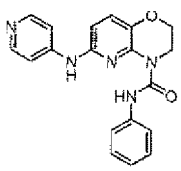 | 0.98 | 348.6 | A |
| I-183 | 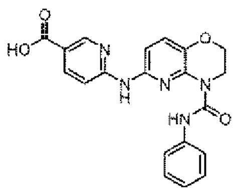 | 1.50 | 392.5 | A |

[Table 1-28]

| Compound No. | Structure | LC/MS Retention Time (min) or NMR Spectrum | MS[M+H]+ | LC Method |
|---|---|---|---|---|
| I-184 | 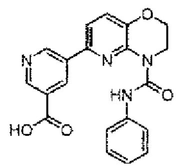 | 1.66 | 377.5 | A |
| I-185 | 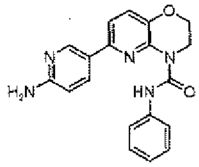 | 1.05 | 348.4 | A |
| I-186 | 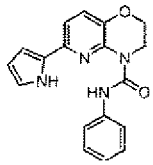 | 2.20 | 321.5 | A |
| I-187 | 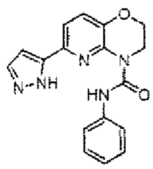 | 1.80 | 322.5 | A |
| I-188 | 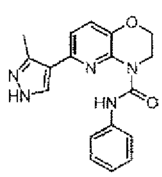 | 1.73 | 336.5 | A |
| I-189 | 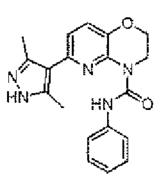 | 1.72 | 350.5 | A |
| I-190 | 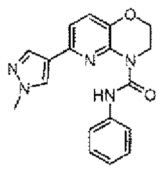 | 1.92 | 336.5 | A |

[Table 1-29]

| 化合物No. | Structure | LC/MS Retention Time (min) or NMR Spectrum | MS[M+H]+ | LC Method |
|---|---|---|---|---|
| I-191 | 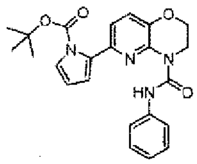 | 2.71 | 421.6 | A |
| I-192 | 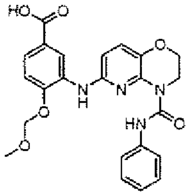 | 1.84 | 451.3 | A |
| I-193 | 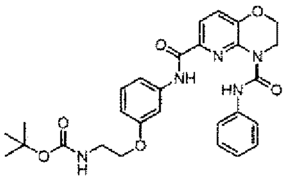 | 2.38 | 534.6 | A |
| I-194 | 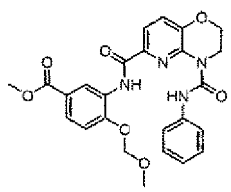 | 2.32 | 494.4 | A |
| I-195 | 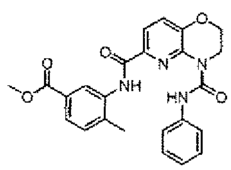 | 2.19 | 447.4 | A |
| I-196 | 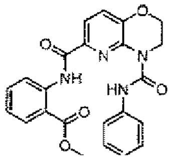 | 2.51 | 433.4 | A |
| I-197 | 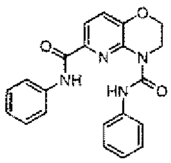 | 2.21 | 375,4 | A |

[Table 1-30]

| Compound No. | Structure | LC/MS Retention Time (min) or NMR Spectrum | MS[M+H]+ | LC Method |
|---|---|---|---|---|
| I-198 | 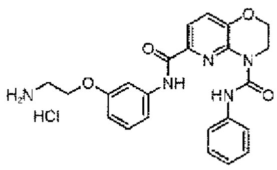 | 1.15 | 424.4 | A |
| I-199 | 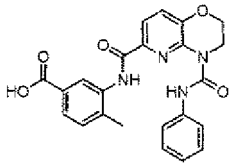 | 1.86 | 433.4 | A |
| I- 200 | 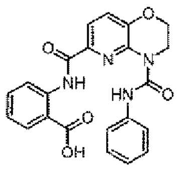 | 2.14 | 419.5 | A |

[Table 1-31]

| Compound No. | Structure | LC/MS Retention Time (min) or NMR Spectrum | MS[M+H]+ | LC Method |
|---|---|---|---|---|
| I-201 | 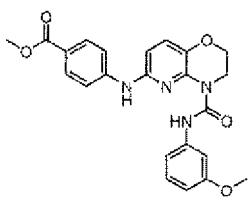 | 2.23 | 435.2 | A |
| I-202 | 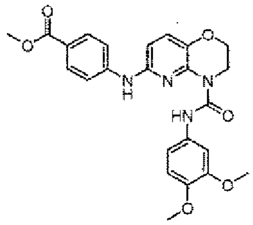 | 2.05 | 465.3 | A |

[Table 1-32]

| Compound No. | Structure | LC/MS Retention Time (min) or NMR Spectrum | MS[M-H]+ | LC Method |
|---|---|---|---|---|
| I-203 | 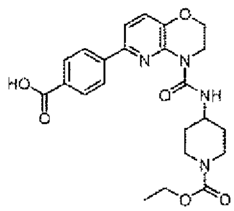 | 1.22 | 455.2 | A |
| I-204 | 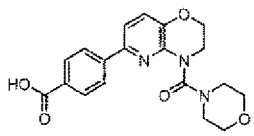 | 1.30 | 370.2 | A |
| I-205 | 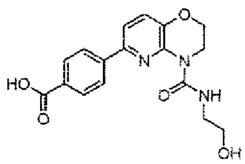 | 1.22 | 344.2 | A |
| I-206 | 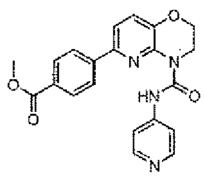 | 1.27 | 391.1 | A |
| I-207 | 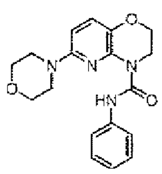 | 2.22 | 341.1 | C |
| I-208 | 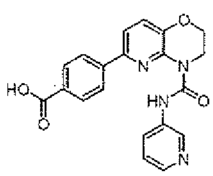 | 1.12 | 377.1 | A |
| I-263 | 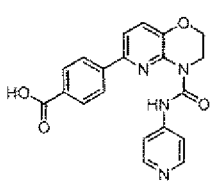 | 1.23 | 377.1 | C |

[Table 1-33]

| Compound No. | Structure | LC/MS Retention Time (min) or NMR Spectrum | MS[M+H]+ | LC Method |
|---|---|---|---|---|
| I-210 | 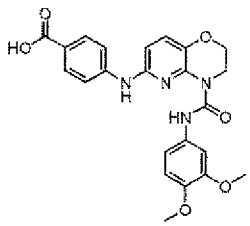 | 1.65 | 451.2 | A |
| I-211 | 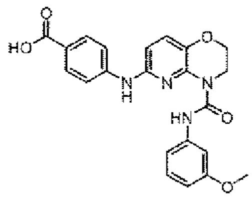 | 1.78 | 421.2 | A |
| I-212 | 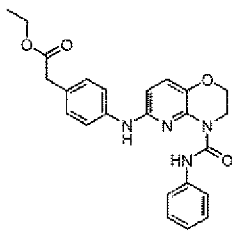 | 2.36 | 433.2 | A |
| I-213 | 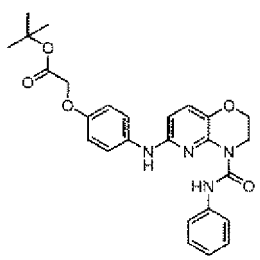 | 2.52 | 477.2 | A |
| I-214 | 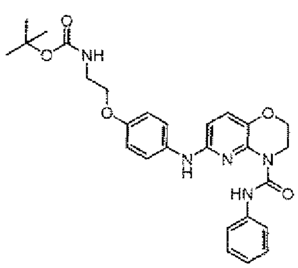 | 2.38 | 506.1 | A |

[Table 1-34]

| Compound No. | Structure | LC/MS Retention Time (min) or NMR Spectrum | MS[M+H]+ | LC Method |
|---|---|---|---|---|
| I-215 | 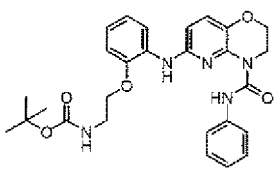 | 2.56 | 506.2 | A |
| I-216 | 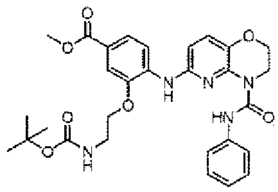 | 2.50 | 564.2 | A |
| I-217 | 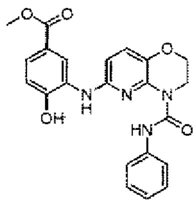 | 1.88 | 421.1 | A |
| I-218 | 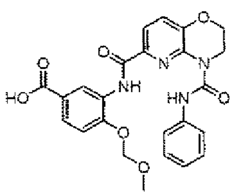 | 1.91 | 479.2 | A |
| I-219 | 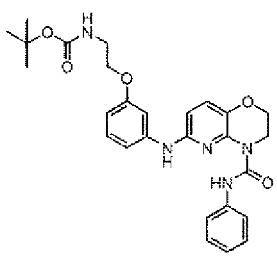 | 2.43 | 506.3 | A |
| I-220 | 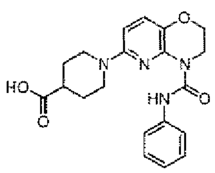 | 1.72 | 383.2 | A |

[Table 1-35]

| Compound No. | Structure | LC/MS Retention Time (min) or NMR Spectrum | MS[M+H]+ | LC Method |
|---|---|---|---|---|
| I-221 | 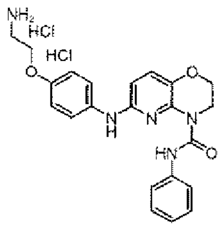 | 1.11 | 406.1 | A |
| I-222 | 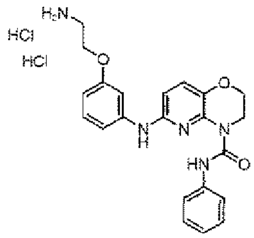 | 1.15 | 406.1 | A |
| I-223 | 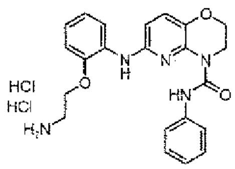 | 1.21 | 4061 | A |
| I-224 | 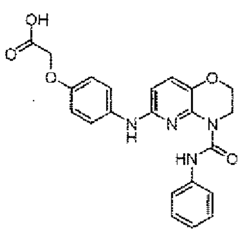 | 1.81 | 421.1 | A |
| I-225 | 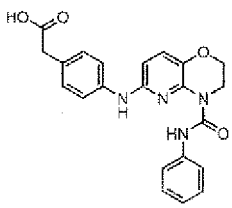 | 1.84 | 405.1 | A |

[Table 1-36]

| Compound No. | Structure | LC/MS Retention Time (min) or NMR Spectrum | MS[M+H]+ | LC Method |
|---|---|---|---|---|
| I-226 | 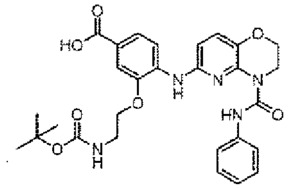 | 2.16 | 550.1 | A |
| I-227 | 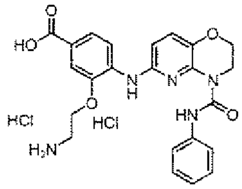 | 1.05 | 450.1 | A |
| I-228 | 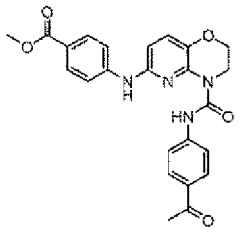 | 2.13 | 447.0 | A |
| I-229 | 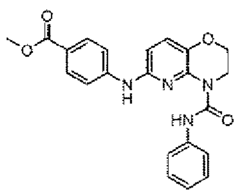 | 2.23 | 405.1 | A |
| I-230 | 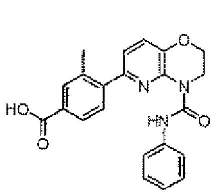 | 1H-NMR (DMSO-d6) δ 2.41 (3H. s), 4.12 (2H, bs), 4.36 (2H, bs), 7,00 (1H, bs), 7.25-7.35 (5H, m), 7.52 (1H, d, J= 8.59 Hz), 7.57 (1H, d. J = 8.59 Hz), 7.90 (1H, d, J=7,33 Hz), 7.94 (1 H, s), 12.8 (1H, s) | | |
| I-231 | 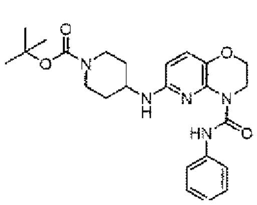 | 2.24 | 454.3 | A |

[Table 1-37]

| Compound No. | Structure | LC/MS Retention Time (min) or NMR Spectrum | MS[M+H]+ | LC Method |
|---|---|---|---|---|
| I-232 | 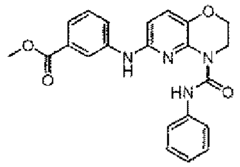 | 2.27 | 405.1 | A |
| I-233 | 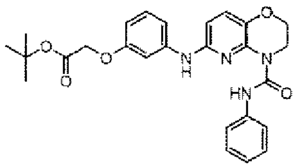 | 2.57 | 477.2 | A |
| I-234 | 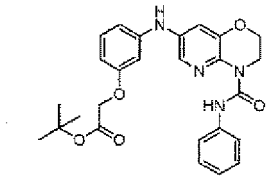 | 2.64 | 477.2 | A |
| I-235 | 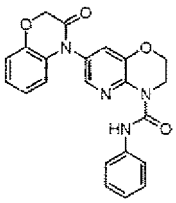 | 2.33 | 403.4 | A |
| I-236 | 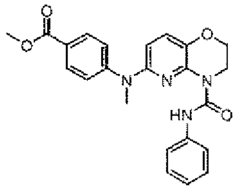 | 2.52 | 419.5 | A |
| I-237 | 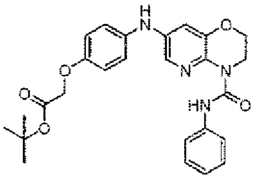 | 2.68 | 477.5 | A |

[Table 1-38]

| Compound No. | Structure | LC/MS Retention Time (min) or NMR Spectrum | MS[M+H]+ | LC Method |
|---|---|---|---|---|
| I-238 | 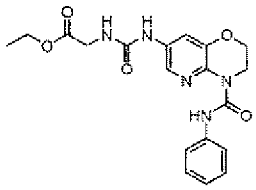 | 1.86 | 400.5 | A |
| I-239 | 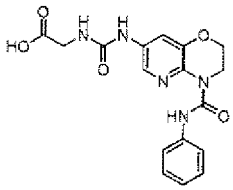 | 1.51 | 372.4 | A |
| I-240 | 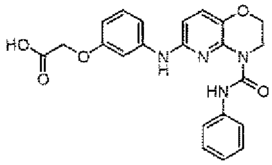 | 1.94 | 421.4 | A |
| I-241 | 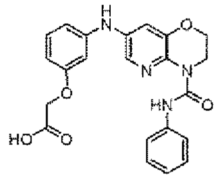 | 2.05 | 421.0 | A |
| I-242 | 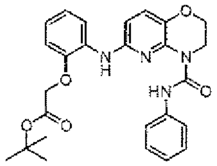 | 2.78 | 477.6 | A |
| I-243 | 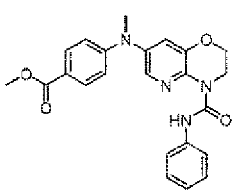 | 2.63 | 419.6 | A |

[Table 1-39]

| Compound No. | Structure | LC/MS Retention Time (min) or NMR Spectrum | MS[M+H]+ | LC Method |
|---|---|---|---|---|
| I-244 | 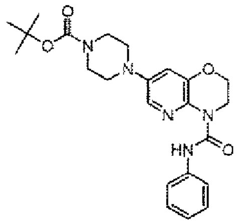 | 2.38 | 440.3 | A |
| I-245 | 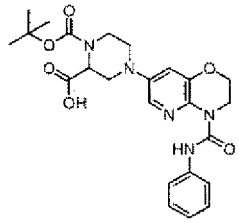 | 2.11 | 494.3 | A |
| I-246 | 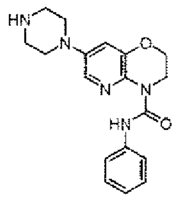 | 1.15 | 340.2 | A |
| I-247 | 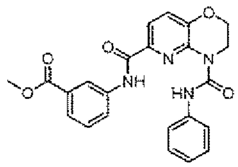 | 2.30 | 433.5 | A |
| I-248 | 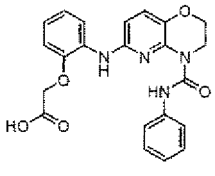 | 2.08 | 421.5 | A |
| I-249 | 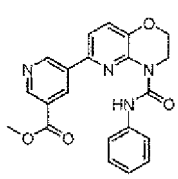 | 2.11 | 391.0 | A |

[Table 1-40]

| Compound No. | Structure | LC/MS Retention Time (min) or NMR Spectrum | MS[M+H]+ | LC Method |
|---|---|---|---|---|
| I-250 | 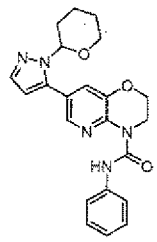 | 2.31 | 406.0 | A |
| I-251 | 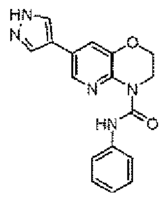 | 1.72 | 322.0 | A |
| I-252 | 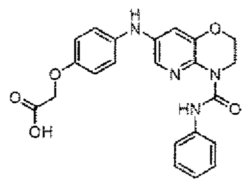 | 2.20 | 421.6 | A |
| I-255 | 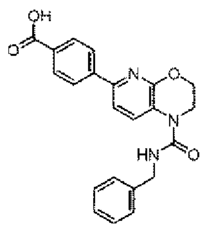 | 1.62 | 390.3 | A |
| I-256 | 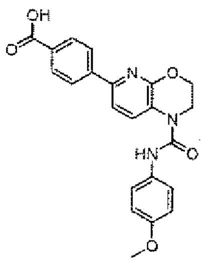 | 1.62 | 406.3 | A |

[Table 1-41]

| Compound No. | Structure | LC/MS Retention Time (min) or NMR Spectrum | MS[M+H]+ | LC Method |
|---|---|---|---|---|
| I-257 | 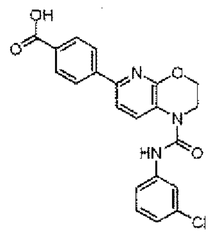 | 1.83 | 410.2 | A |
| I-258 | 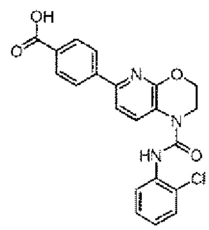 | 1.80 | 410.2 | A |
| I-259 | 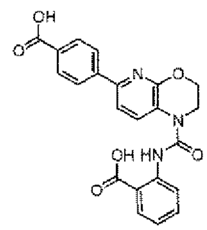 | 1.67 | 420.2 | A |
| I-260 | 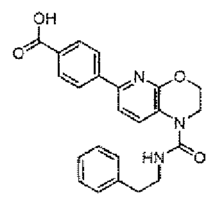 | 1.71 | 404.3 | A |
| I-261 | 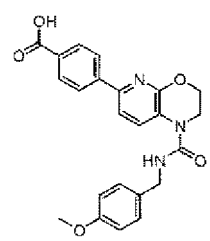 | 1.62 | 420.3 | A |

[Table 1-42]

| Compound No. | Structure | LC/MS Retention Time (min) or NMR Spectrum | MS[M+H]+ | LC Method |
|---|---|---|---|---|
| I-262 | 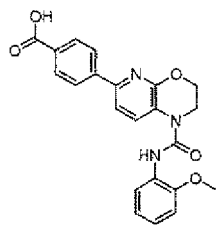 | 1.74 | 406.3 | A |
| I-263 | 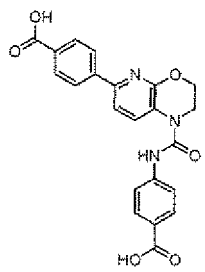 | 1.34 | 420.3 | A |
| I-264 | 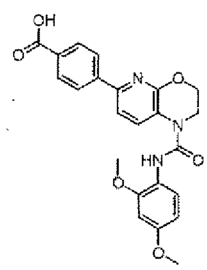 | 1.67 | 436.3 | A |
| I-265 | 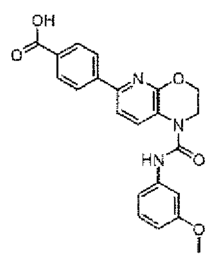 | 1.67 | 406.2 | A |

[Table 1-43]

| Compound No. | Structure | LC/MS Retention Time (min) or NMR Spectrum | MS[M+H] | LC Method |
|---|---|---|---|---|
| I-266 | 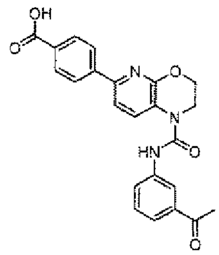 | 1.56 | 418.3 | A |
| I-267 | 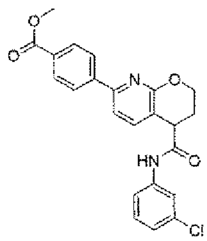 | 2.27 | 424.2 | A |

[Table 1-44]

| Compound No. | Structure | LC/MS Retention Time (min) or NMR Spectrum | MS[M+H]+ | LC Method |
|---|---|---|---|---|
| I-268 | 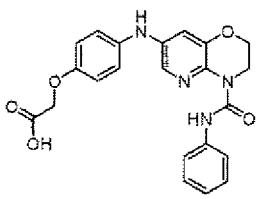 | 2.20 | 421.6 | A |
| I-269 | 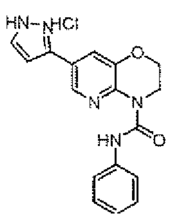 | 1.80 | 322.1 | A |
| I-270 | 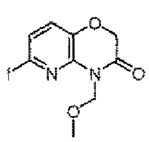 | 1.76 | 321.2 | A |

(continued)

| Compound No. | Structure | LC/MS Retention Time (min) or NMR Spectrum | MS[M+H]+ | LC Method |
|---|---|---|---|---|
| I-271 | 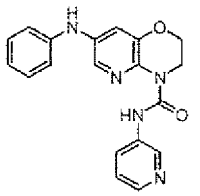 | 1.40 | 348.1 | A |
| I-272 | 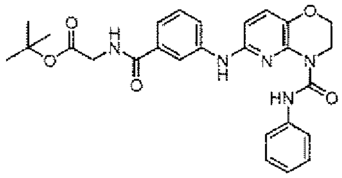 | 2.19 | 504.5 | A |
| I-273 | 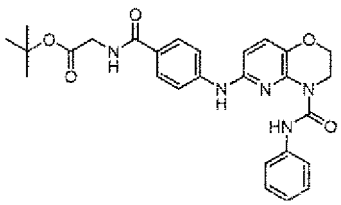 | 2.14 | 504.5 | A |

[Table 1-45]

| Compound No. | Structure | LC/MS Retention Time (min) or NMR Spectrum | MS[M+H]+ | LC Method |
|---|---|---|---|---|
| I-274 | 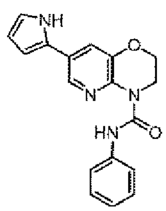 | 2.24 | 321.3 | A |
| I-275 | 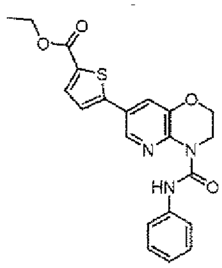 | 2.70 | 410.3 | A |

(continued)

| Compound No. | Structure | LC/MS Retention Time (min) or NMR Spectrum | MS[M+H]+ | LC Method |
|---|---|---|---|---|
| I-276 | 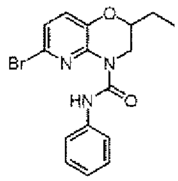 | 2.83 | 364.2 | A |
| I-277 | 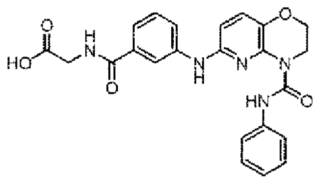 | 1.64 | 448.4 | A |
| I-278 | 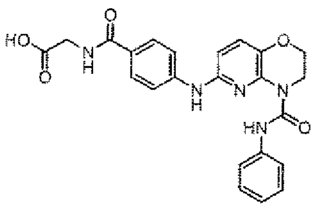 | 1.57 | 448.3 | A |
| I-279 | 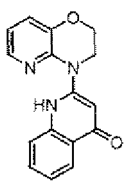 | 0.64 | 280.1 | A |

[Table 1-46]

| Compound No. | Structure | LC/MS Retention Time (min) or NMR Spectrum | MS[M+H]+ | LC Method |
|---|---|---|---|---|
| I-280 | 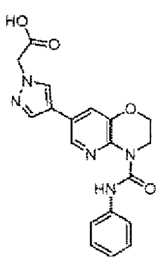 | 1.74 | 380.3 | A |
| I-281 | 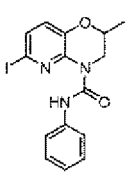 | 2.73 | 396.2 | A |

(continued)

| Compound No. | Structure | LC/MS Retention Time (min) or NMR Spectrum | MS[M+H]+ | LC Method |
|---|---|---|---|---|
| I-282 | 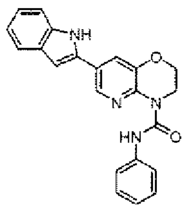 | 2.58 | 371.4 | A |
| I-283 | 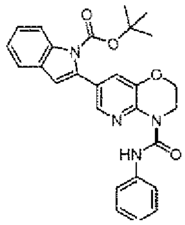 | 3.06 | 471.5 | A |
| I-284 | 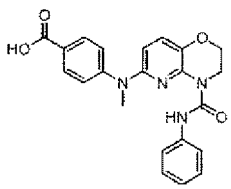 | 2.02 | 405.4 | A |
| I-285 | 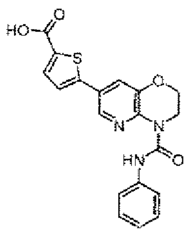 | 2.11 | 382.4 | A |

[Table 1-47]

| Compound No. | Structure | LC/MS Retention Time (min) or NMR Spectrum | MS[M+H]+ | LC Method |
|---|---|---|---|---|
| I-286 | 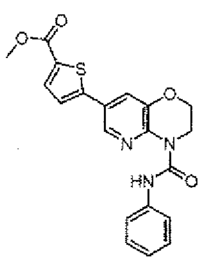 | 2.60 | 396.4 | A |

(continued)

| Compound No. | Structure | LC/MS Retention Time (min) or NMR Spectrum | MS[M+H]+ | LC Method |
|---|---|---|---|---|
| I-287 | 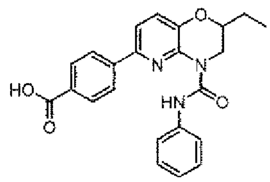 | 2.42 | 404.5 | A |
| I-288 | 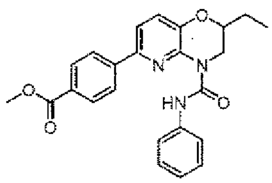 | 2.90 | 418.4 | A |
| I-289 | 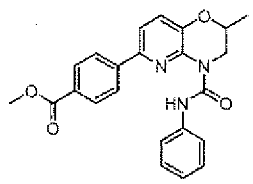 | 2.74 | 404.5 | A |
| I-290 | 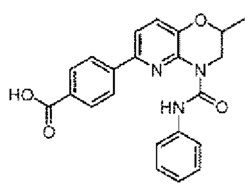 | 2.25 | 390.4 | A |
| I-291 | 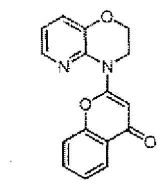 | 1.06 | 281.1 | A |

[Table 1-48]

| Compound No. | Structure | LC/MS Retention Time (min) or NMR Spectrum | MS[M+H]+ | LC Method |
|---|---|---|---|---|
| I-282 | 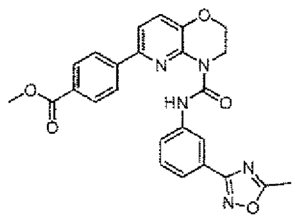 | 2.79 | 472.0 | A |

(continued)

| Compound No. | Structure | LC/MS Retention Time (min) or NMR Spectrum | MS[M+H]+ | LC Method |
|---|---|---|---|---|
| I-293 | 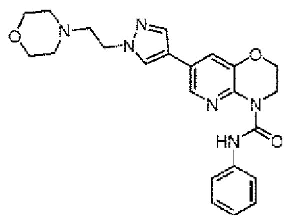 | 1.16 | 435.5 | A |
| I-294 | 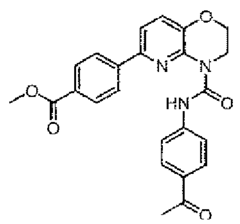 | 2.54 | 432.4 | A |
| I-295 | 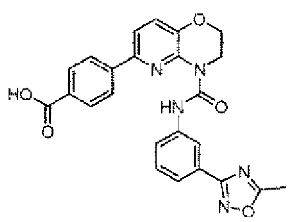 | 2.26 | 458.4 | A |
| I-296 | 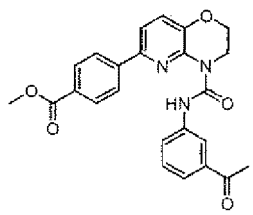 | 2.47 | 432.3 | A |
| I-297 | 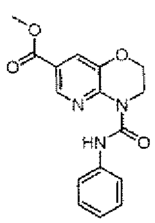 | 2.35 | 314.3 | A |

[Table 1-49]

| Compound No. | Structure | LC/MS Retention Time (min) or NMR Spectrum | MS[M+H]+ | LC Method |
|---|---|---|---|---|
| I-298 | 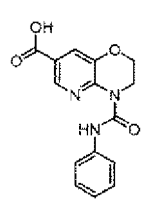 | 1.85 | 300.2 | A |

(continued)

| Compound No. | Structure | LC/MS Retention Time (min) or NMR Spectrum | MS[M+H]+ | LC Method |
|---|---|---|---|---|
| I-299 | 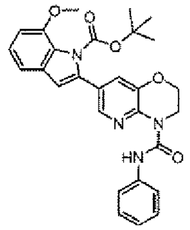 | 2.95 | 501.3 | A |
| I-300 | 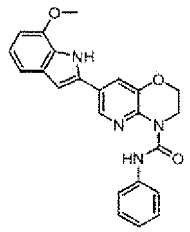 | 2.73 | 401.2 | A |
| I-301 | 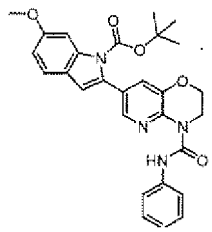 | 3.00 | 501.2 | A |
| I-302 | 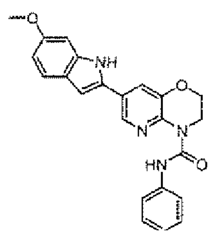 | 2.63 | 401.2 | A |
| I-303 | 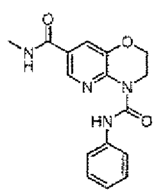 | 1.73 | 313.2 | A |

[Table 1-50]

| Compound No. | Structure | LC/MS Retention Time (min) or NMR Spectrum | MS[M+H]+ | LC Method |
|---|---|---|---|---|
| I-304 | 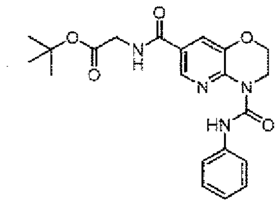 | 2.17 | 413.3 | A |
| I-305 | 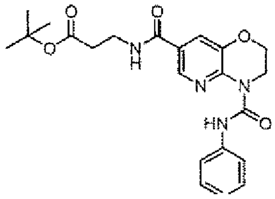 | 2.29 | 427.2 | A |
| I-306 | 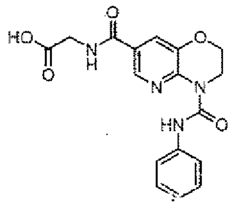 | 1.67 | 357.0 | A |
| I-307 | 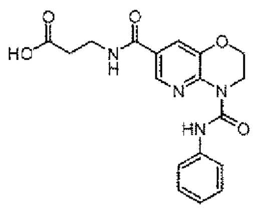 | 1.57 | 371.1 | A |
| I-308 | 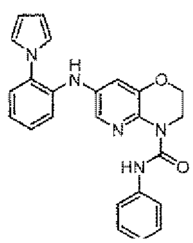 | 2.65 | 412.2 | A |
| I-309 | 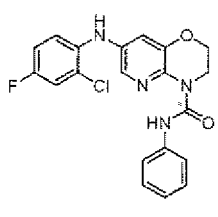 | 2.69 | 399.1 | A |

[Table 1-51]

| Compound No. | Structure | LC/MS Retention Time (min) or NMR Spectrum | MS[M+H]+ | LC Method |
|---|---|---|---|---|
| I-310 | 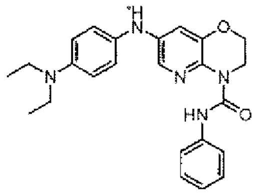 | 1.32 | 418.3 | A |
| I-311 | 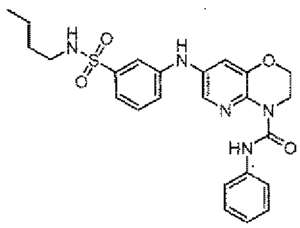 | 2.41 | 482.2 | A |
| I-312 | 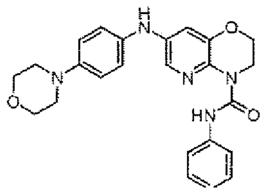 | 2.01 | 432.2 | A |
| I-313 | 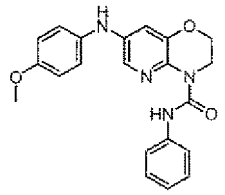 | 2.43 | 377.1 | A |
| I-314 | 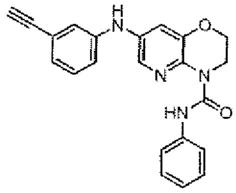 | 2.32 | 371.2 | A |
| I-315 | 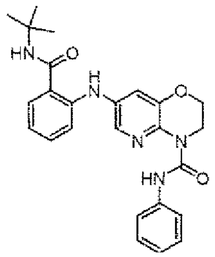 | 2.75 | 446.2 | A |

[Table 1-52]

| Compound No. | Structure | LC/MS Retention Time (min) or NMR Spectrum | MS[M+H]+ | LC Method |
|---|---|---|---|---|
| I-316 | 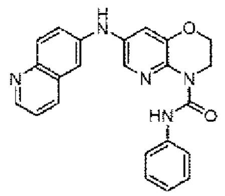 | 1.39 | 398.2 | A |
| I-317 | 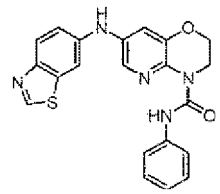 | 2.24 | 404.1 | A |
| I-318 | 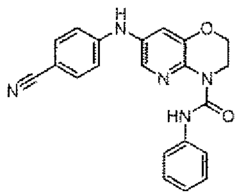 | 2.20 | 372.1 | A |
| I-319 | 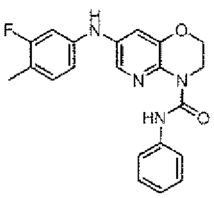 | 2.42 | 379.1 | A |
| I-320 | 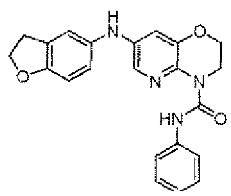 | 2.32 | 389.1 | A |
| I-321 | 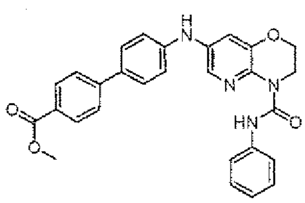 | 2.74 | 481.2 | A |

[Table 1-53]

| Compound No. | Structure | LC/MS Retention Time (min) or NMR Spectrum | MS[M+H]+ | LC Method |
|---|---|---|---|---|
| I-322 | 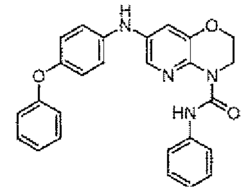 | 2.70 | 439.0 | A |
| I-323 | 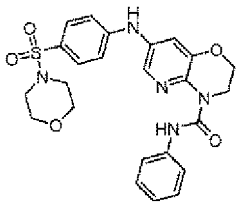 | 2.19 | 496.1 | A |
| I-324 | 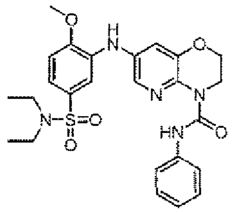 | 2.39 | 512.2 | A |
| I-325 | 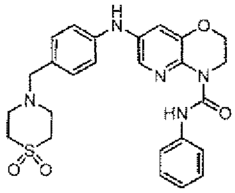 | 1.78 | 494.1 | A |
| I-326 | 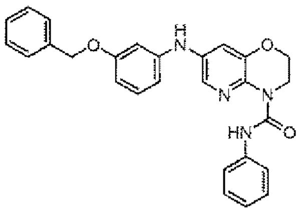 | 2.68 | 453.2 | A |
| I-327 | 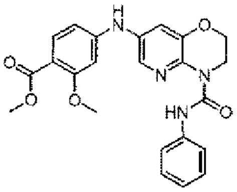 | 2.23 | 435.2 | A |

[Table 1-54]

| Compound No. | Structure | LC/MS Retention Time (min) or NMR Spectrum | MS[M+H]+ | LC Method |
|---|---|---|---|---|
| I-328 | 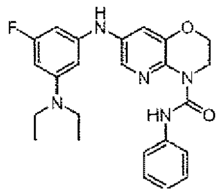 | 2.52 | 436.2 | A |
| I-329 | 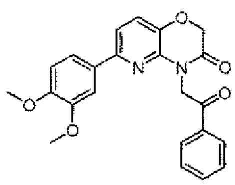 | 2.17 | 405.1 | A |
| I-330 | 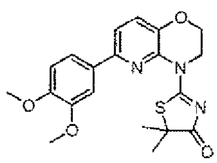 | 2.10 | 400.0 | A |
| I-331 | 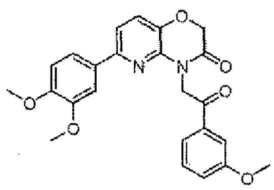 | 2.26 | 435.2 | A |
| I-332 | 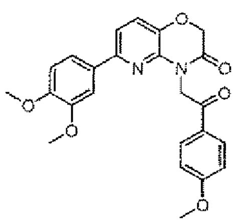 | 2.06 | 435.0 | A |
| I-333 | 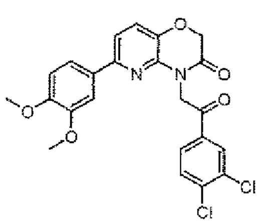 | 2.45 | 472.9 | A |

[Table 1-55]

| Compound No. | Structure | LC/MS Retention Time (min) or NMR Spectrum | MS[M+H]+ | LC Method |
|---|---|---|---|---|
| I-334 | 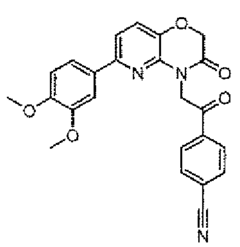 | 1.99 | 430.1 | A |
| I-335 | 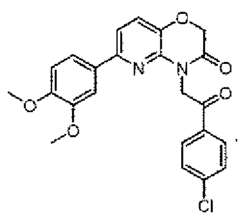 | 2.31 | 439.0 | A |
| I-336 | 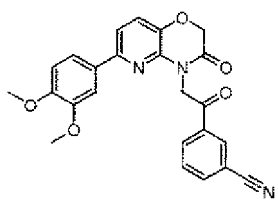 | 1.98 | 430.1 | A |
| I-337 | 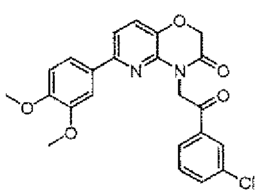 | 2.30 | 439.0 | A |

(Example 10: Biological activity: Measurement of PI3Kγ inhibitory activity)

For each compound synthesized in the above Examples, PI3Kγ inhibitory activity was measured.

(Method)

The PI3Kγ inhibitory activity of a compound was evaluated using P13-kinase HTRF™ assay (Millipore) according to the following procedure:

To each well of a testing plate, 5 μL of a compound solution containing 10% DMSO (200 μmol/L or 40 μmol/L as the concentration of the compound), 5 μL of a substrate solution (40 μmol/L phosphatidylinositol (4,5)-bisphosphate, 20 mmol/L $MgCl_2$, 10mmol/L DTT), and 5 μL of a enzyme solution (80 μg/mL PI-3 kinase γ, 10 mmol/L $MgCl_2$ 5 mmol/L DTT) were added, and then allowed to stand for 10 minutes.

Then, 5 μL of a reaction solution (40 μmol/LATP, 10 mmol/L $MgCl_2$, 5 mmol/L DTT) was added thereto. After reacting for 30 minutes at room temperature, 5μL of a solution containing EDTA and biotinylated phosphatidylinositol (3,4,5)-triphosphate were added to quench the reaction.

5 μL of a detection reagent containing a europium-labeled anti-GST antibody, the GST-tagged PH domain, and allophycocyanin-labeled streptavidin was added thereto. After 18 hours, HTRF (excitation wavelength: 330 nm, measured wavelengths: 620 nm and 665 nm) was measured.

HTRF ratio was defined as the value obtained by dividing the amount of fluorescence obtained at the measured wavelength 665 nm by the amount of fluorescence obtained at 620 nm. The HTRF ratio in the absence of a compound was defined as 100% activity and the HTRF ratio in the absence of PI-3 kinase γ was defined as 0% activity to calculate

an inhibition ratio which was defined as the PI3Kγ inhibitory activity of a compound at 50 μmol/L or 10 μmol/L.

(Results)

Results are shown in the tables below. In the tables, "μM" refers to "μmol/L."

[Table 2-1]

| Compound No. | Inhibition Ratio % (50 μM) | Compound No. | Inhibition Ratio % (50 μM) | Compound No. | Inhibition Ratio % (50 μM) |
|---|---|---|---|---|---|
| I-1 | 87 | I-99 | ≧95 | I-147 | ≧95 |
| I-2 | 89 | I-100 | ≧95 | I-148 | 94 |
| I-3 | 80 | I-101 | ≧95 | I-150 | 87 |
| I-4 | 84 | I-102 | 94 | I-151 | 94 |
| I-5 | 90 | I-103 | 94 | I-152 | ≧95 |
| I-6 | 81 | I-104 | ≧95 | I-160 | 75 |
| I-7 | 75 | I-106 | ≧95 | I-161 | 88 |
| I-17 | 88 | I-107 | ≧95 | I-162 | ≧95 |
| I-38 | 76 | I-108 | 88 | I-163 | 76 |
| I-40 | 80 | I-109 | ≧95 | I-164 | ≧95 |
| I-49 | ≧95 | I-111 | 87 | I-173 | ≧95 |
| I-51 | 93 | I-118 | ≧95 | I-174 | 79 |
| I-60 | 74 | 1-122 | 87 | I-179 | 79 |
| I-69 | 73 | I-123 | ≧95 | I-180 | ≧95 |
| I-70 | 71 | I-124 | 76 | I-181 | ≧95 |
| I-77 | 82 | I-125 | ≧95 | I-182 | ≧95 |
| I-83 | 92 | I-126 | ≧95 | I-183 | 86 |
| I-84 | 72 | I-130 | ≧95 | I-184 | ≧95 |
| I-86 | 82 | I-131 | ≧95 | I-185 | 76 |
| I-87 | 75 | I-132 | 83 | I-187 | ≧95 |
| I-88 | 91 | I-137 | ≧95 | I-188 | ≧95 |
| I-89 | ≧95 | I-139 | ≧95 | I-189 | 83 |
| I-90 | 72 | I-141 | 82 | I-190 | ≧95 |
| I-92 | ≧95 | I-142 | 78 | I-203 | ≧95 |
| I-94 | ≧95 | I-143 | ≧95 | I-205 | ≧95 |
| I-95 | ≧95 | I-144 | 81 | I-207 | 81 |
| I-97 | 89 | I-145 | 81 | I-208 | ≧95 |
| I-98 | 73 | I-146 | ≧95 | I-209 | 90 |

[Table 2-2]

| Compound No. | Inhibition Ratio % (50 μM) | Compound No. | Inhibition Ratio % (50 μM) | Compound No. | Inhibition Ratio % (50 μM) |
|---|---|---|---|---|---|
| I-210 | ≧95 | I-239 | ≧95 | I-284 | 82 |
| I-211 | ≧95 | I-240 | ≧95 | I-285 | 88 |
| I-212 | ≧95 | I-241 | ≧95 | I-290 | 86 |
| I-213 | ≧95 | I-242 | ≧95 | I-293 | ≧95 |
| I-214 | ≧95 | I-243 | 77 | I-302 | 87 |
| I-215 | ≧95 | I-244 | 80 | I-303 | 89 |
| I-216 | ≧95 | I-245 | ≧95 | I-305 | 84 |
| I-217 | 77 | I-246 | ≧95 | I-307 | 92 |
| I-218 | 87 | I-248 | ≧95 | I-309 | 90 |
| I-219 | ≧95 | I-249 | ≧95 | I-310 | ≧95 |
| I-220 | ≧95 | I-250 | 91 | I-311 | 81 |
| I-221 | ≧95 | I-251 | ≧95 | I-313 | 92 |
| I-222 | ≧95 | I-252 | ≧95 | I-314 | 78 |
| I-223 | ≧95 | I-259 | ≧95 | I-315 | 93 |
| I-224 | ≧95 | I-265 | 75 | I-316 | 87 |
| I-225 | ≧95 | I-268 | ≧95 | I-317 | ≧95 |
| I-226 | ≧95 | I-269 | 71 | I-318 | ≧95 |
| I-227 | ≧95 | I-271 | ≧95 | I-319 | 92 |
| I-229 | ≧95 | I-272 | ≧95 | I-320 | 95 |
| I-230 | ≧95 | I-273 | 76 | I-321 | 76 |
| I-231 | ≧95 | I-274 | ≧95 | I-322 | 78 |
| I-232 | ≧95 | I-277 | ≧95 | I-323 | 89 |
| I-233 | ≧95 | I-278 | ≧95 | I-325 | 82 |
| I-234 | ≧95 | I-280 | 88 | I-327 | 86 |
| I-237 | ≧95 | I-282 | 74 | I-328 | 91 |
| I-238 | 83 | I-283 | 85 | I-330 | 95 |

(Example 11: Measurement of AKT phosphorylation inhibitory activity)

Human monocyte-like cells were used to measure in *vitro* inhibitory activity.

(Method).

(1) The AKT phosphorylation inhibitory activity of a compound was evaluated according to the following procedure.
(2) Human monocyte-like cell line THP-1 was washed with RPMI-1640 media, incubated in the presence of 5% $CO_2$ at 37 ˚C for 3 hours, washed with Hank's balanced salt solution (HBSS), adjusted to a cell concentration of 6.6 x $10^6$ /mL, and then used in an experiment.
(3) 30 μL of the cell suspension and 60 μL of each compound solution containing 0.2% DMSO/HBSS were mixed and then preincubated at 37˚C for 5 minutes. 30 μL of HBSS containing 4 μg/mL of MCP-1 was added thereto and then incubated for 30 seconds at 37˚C.

(4) 30 μL of a cell lysate (20 mmol/L Tris-HCl (pH 7.5), 150 mmol/L NaCl, 1 mmol/L $Na_2EDTA$, 1 mmol/L EGTA, 1% Triton, 2. 5 mmol/L sodium pyrophosphate, 1mmol/L β-glycerophosphate, 1 mmol/L $Na_3VO_4$, 1 μg/ml leupeptin, 50 nmol/L APMSF) was added thereto to lyse cells.

(5) The amount of AKT phosphorylation in the cell lysate was measured by ELISA method.

(6) Anti-phospho-Akt (Ser473) antibodies (clone 193H12, derived from a rabbit) were immobilized onto the solid phase of a micro well plate. 100 μL of the prepared cell lysate was added to the micro well plate, incubated for 2 hours at 37˚C, and then washed four times with Phosphate Buffered Saline containing 0.05% Tween-20.

(7) An anti-AKT1 antibody (clone 2H10, derived from a mouse) was added thereto, incubated for 1 hour at 37˚C, washed similarly, and then reacted with an HRP-labeied anti-mouse IgG antibody.

(8) After incubating at 37˚C for 30 minutes and then washing similarly, 100 μL af TMB (3,3',5,5"-tetramethylbenzidine) was added thereto, followed by reacting at room temperature for 30 minutes.

(9) 100 μL of 1 mol/L sulfuric acid was added to quench the color reaction and then the absorbance at 450 nm was measured.

(10) A series of diluted cell lysates of a positive control (a sample in the absence of a compound) were used to prepare a calibration curve, the amount of AKT phosphorylation in a sample in the absence of MCP-1 was defined as 0% activity to calculate an inhibition ratio. The measurement was performed for a 1 μmol/L solution of each compound.

(Results) The inhibition ratio for each compound at 1 μmol/L is shown.

Compound No. I-145: >99.5%
Compound No. I-146: >99.5%
Compound No. I-330: >99.5%.

(Example 12 Biological activity: Measurement of PI3Kγ inhibitory activity (Ki value))

The PI3Kγ inhibitory activity (Ki value) of a compound was evaluated according to the following procedure:

5 μL of a compound solution containing 10% DMSO and 200 μmol/L of a compound was changed to 5 μL of a compound solution containing 10% DMSO and either 200, 64, 20, 6.4, 2, 0.64, or 0.20 μmol/L of the compound (optionally, this is diluted to a lower concentration) . Using a method similar to the method for measuring PI3Kγ inhibitory activity, inhibition ratios were measured in the presence of the compound at 50, 16, 5, 1.6, 0.5, 0.16, and 0.05 μmol/L (optionally, a lower concentration). Then an $IC_{50}$ value was calculated by a logistic approximation method or the linear regression method using two concentrations that across 50% inhibition. Separately, the ATP concentration in the reaction solution (40 μmol/L ATP, 10 mmol/L $MgCl_2$ 5 mmol/L DTT) at the time of the start of a reaction in the absence of the compound was changed to 80, 40, 20, 10, 5, 2.5, 1.25, or 0.625 μmol/L. Then, HTRF ratios were measured by a similar method. The value obtained by subtracting the HTRF ratio at each ATP concentration from the HTRF ratio in the absence of PI-3 kinase γ was defined as the value obtained by multiplying reaction rate (v) at each ATP concentration with a constant. The Michaelis-Menten constant Km was then calculated by the Lineweaver-Burk plot method. The Ki value of a compound was calculated by the following formula.

$$Ki = IC_{50}\ value/(1 + 10\ \mu mol/L\ (test\ ATP\ concentration)/Km\ (\mu mol/L))$$

(Results)

Compound No. I-248: 0.025 μmol/L
Compound No. I-208: 0.031 μmol/L
Compound No. I-145: 0.044 μmol/L
Compound No. I-146: 0.046 μmol/L
Compound No. I-240: 0.048 μmol/L
Compound No. I-330: 0.027 μmol/L

(Example 13 Biological activity: Measurement of PI3Kα inhibitory activity)

The PI3Kα inhibitory activity of a compound was evaluated according to the following procedure:
According to Example 10 above, after 80 μg/mL PI-3 kinase γ of the enzyme solution (80 μg/mL PI-3 kinase γ, 10 mmol/L $MgCl_2$, 5 mmol/L DTT) was changed to 0.8 μg/mL PI-3 kinase α, an inhibition ratio was calculated by a method similar to the method for measuring PI3Kγ inhibitory activity, and then defined as the PI3Kα inhibitory activity at 50 μmol/L.

(Example 14 Biological activity: Measurement of PI3Kα inhibitory activity (Ki value))

The α inhibitory activity (Ki value) of a compound was evaluated according to the following procedure:
According to Example 12 above, after 80μg/mL PI-3 kinase γ of the enzyme solution (80 μg/mL PI-3 kinase γ, 10 mmol/L $MgCl_2$, 5 mmol/L DTT) was changed to 0.8 μg/mL PI-3 kinase α, a Km value measured with PI3Kα was used to calculate a Ki value for PI3Kα by a method similar to the PI3Kγ inhibitory activity (Ki value).

(Example 15 Measurement of PI3Kβ inhibitory activity)

The PI3Kβ inhibitory activity of a compound was evaluated according to the following procedure.
According to Example 10 above, after 80 μg/mL PI-3 kinase γ of the enzyme solution (80 μg/mL PI-3 kinase γ, 10 mmol/L $MgCl_2$, 5 mmol/L DTT) was changed to 60 μg/mL PI-3 kinase β, a method similar to the method for measuring PI3Kγ inhibitory activity was used to calculate an inhibition ratio, which was defined as the PI3Kβ inhibitory activity at 50 μmol/L.

(Example 16 Measurement of PI3Kβ inhibitory activity (Ki value))

The β inhibitory activity (Ki value) of a compound was evaluated according to the following procedure:
According to Example 12 above, after 80 μg/mL PI-3 kinase γ of the enzyme solution (80 μg/mL PI-3 kinase γ, 10 mmol/L $MgCl_2$, 5 mmol/L DTT) was changed to 60 μg/mL PI-3 kinase β, a Km value measured with PI3Kβ was used to calculate a Ki value for PI3Kβ by a method similar to the PI3Kγ inhibitory activity (Ki value).

(Example 17 Method for calculating the selectivity of PI3Kγ and PI3Kα)

The PI3Kγ/α selectivity of a compound was expressed by the value obtained by dividing the Ki value for PI3Kα by the Ki value for PI3Kγ.

(Example 18 Method for calculating the selectivity of PI3Kγ and PI3Kβ)

The PI3Kγ/β selectivity of a Compound was expressed by the value obtained by dividing the Ki value for PI3Kβ by the Ki value for PI3Kγ.
According to Examples 19 to 24 shown below, compounds of the present invention were evaluated.

(Example 19 Solubility test)

(Method)

The solubility of a compound was determined under a condition in which 1% DMSO was added. 10 mmol/L compound solution was prepared using DMSO, and then 6 μL of the compound solution was added to 594 μL of artificial intestinal juice inpH6.8 (to 250 mL of 0.2 mol/L potassium dihydrogen phosphate reagent solution was added 118 mL of 0.2 mol/L NaOH reagent solution and water to provide a final volume of 1000 mL). After standing at 25˚C for 16 hours, the mixed solution was filtered with suction. The filtrate was diluted twice with methanol/water (1/1), and then a concentration in the filtration was measured with HPLC or LC/MS/MS by the absolute calibration method.

(Results)

Compound No. 1-248: >50 μol/L
Compound No. I-40: >50 μmol/L

Compound No. I-109: >50 μmol/L
Compound No. I-1: >50 μmol/L
Compound No. I-49 . >50 μmol/L
Compound No. I-139: >50 μmol/L
Compound No. I-138: >50 μmol/L
Compound No. I-239: >50 μmol/L

(Example 20 Metabolic stability test)

(Method)

After a subject compound was reacted for a certain time using a commercially available pooled human liver microsome, a reacted sample and an unreacted sample are compared to calculate a survival ratio, and then the degree of metabolism in the liver was evaluated.

0.2 mL of a buffer solution (50 mmol/L of Tris-HCl in pH 7.4, 150 mmol/L of potassium chloride, and 10 mmol/L of magnesium chloride) containing a human liver microsome of 0.5 mg protein/mL was reacted in the presence of 1 mmol/L NADPH at 37°C for 0 or 30 minutes (oxidative reaction). After reacting, 50 μL of the reaction solution was added to 100 μL of a solution of methanol/acetonitrile (1/1 (v/v)), mixed, and then centrifuged at 3000 rpm for 15 minutes. The test compound in the supernatant was quantitated with LC/MS/MS. The amount of the compound at a reaction time of 0 minute was defined as 100% and, based on that, the percentage of the test compound remained after reacting for 30 minutes was calculated.

(Results) The percentage of each test compound remained at 2 μmol/L of compound concentration is shown below.

Compound No. I-145: 88.4%
Compound No. I-146: 100%
Compound No. I-240: 89.9%
Compound No. I-49 : 90.1%
Compound No. I-139: 83%
Compound No. I-239: 100%

(Example 21 CYP inhibition test)

(Method)

A commercially available pooled human liver microsome was used in evaluating the degree of produced-metabolite inhibition exhibited by a test compound. O-de-ethylation of 7-ethoxyresorufin (CYP1A2) , methyl-hydroxylation of tolbutamide (CYP2C9), 4'-hydroxylation of mephenytoin (CYP2C19), O-demethylation of dextromethorphan (CYP2D6), and hydroxylation of terfenadine (CYP3A4), which are typical substrate for metabolic reactions of human main CYP5 molecular species (CYP1A2, 2C9, 2C19, 2D6, and 3A4), were adopted as indexes.

The reaction conditions are as follows: substrate: 0.5 μmol/L of ethoxyresorufin (CYPIA2), 100 μmol/L of tolbutamide (CYP2C9), 50 μmol/L of S-mephenytoin (CYP2C19), 5 μmol/L of dextromethorphan (CYP2D6), and 1 μmol/L of terfenadine (CYP3A4); reaction time; 15 minutes; reaction temperature: 37°C; enzyme: pooled human liver microsome 0.2 mg protein/mL; test drug concentration: 1, 5, 10, and 20 μmol/L (4 points).

In a 96-well plate, as a reaction solution, five kinds of substrates, a human liver microsome, and a test drug were added to 50 mmol/L Hepes buffer solution in the aforementioned ratio. A coenzyme NADPH was added to start a metabolic reaction, which is an index. After reacting at 37°C for 15 minutes, a solution of methanol/acetonitrile (1/1 (v/v)) was added to quench the reaction. After centrifugation at 3000 rpm for 15 minutes, resorufin (CYP1A2 metabolite) in the supernatant was quantitated with a fluorescence multilabel counter, and hydroxylated tolbutamide (CYP2C9 metabolite), 4'-hydroxylated mephenytoin (CYP2C19 metabolite), dextromethorphan (CYP2D6 metabolite), terfenadine in alcohol form (CYP3A4 metabolite) were quantitated with LC/MS/MS.

The case wherein only DMSO (a solvent which dissolved a drug) was added to a reaction system was defined as a control (100%). When a test drug solution was added, the remaining activity (%) at each concentration was calculated, and then $IC_{50}$ was calculated with a concentration and an inhibition ratio by an inverse estimation using a logistic model.

(Results)

Compound No. 1-240: 5 kinds >20 μmol/L
Compound No. I-49 : 5 kinds >20 μmol/L
Compound No. 1-239: 5 kinds >20 μmol/L

(Example 22 CYP3A4 fluorescence AMBI test)

(Method)

The CYP3A4 fluorescence MBI test is a test to examine the enhancement of CYP3A4 inhibition of a compound by a metabolic reaction. The test was performed using as an index, a reaction to produce a metabolite 7-hydroxytrifluoromethylcoumarin (HFC) which emits fluorescence, in which CYP3A4 expressed in *E. coli* was used as an enzyme and 7-benzyloxytrifluoromethylcoumarin (BFC) is debenzylated by CYP3A4 enzyme.

The reaction conditions are as follows: substrate: 5.6 μmol/L 7-BFC; pre-reaction time: 0 or 30 minutes; reaction time: 15 minutes; reaction temperature: 25˚C (room temperature); content of CYP3A4 (an enzyme expressed in *E. coli*) : 62.5 pmol/mL at the time of a pre-reaction and 6.25pmol/mL (when diluted 10 times) at the time of a reaction; the concentrations of a test drug: 0.625, 1.25, 2.5, 5, 10, and 20 μmol/L (6 points).

In a 96-well plate, as a pre-reaction solution, an enzyme solution and a test drug solution were added into K-Pi buffer solution (pH 7.4) in the aforementioned ratio. Then, a portion thereof was transferred to another 96-well plate so as to be diluted ten times with a substrate and a K-Pi buffer solution. A coenzyme NADPH was then added to start a reaction that is an index (without a pre-reaction). After reacting for a predetermined time, 4/1 of acetonitrile/0.5 mol/L Tris (trishydroxyaminomethane) was added to quench the reaction. To the remaining pre-reaction solution, NADPH was also added to start a pre-reaction (with a pre-reaction). After pre-reacting for a predetermined time, a portion thereof was transferred to another plate so as to be diluted ten times with a substrate and K-Pi buffer solution, and thereby the reaction that is an index started. After reacting for a predetermined time, 4/1 of acetonitrile/0.5 mol/L Tris (trishydroxyaminomethane) was added to quench the reaction. For each plate in which the index reaction was performed, the fluorescence value of a metabolite 7-HFC was measured with a fluorescent plate reader (Ex = 420 nm, Em = 535 nm).

The case wherein only DMSO (a solvent which dissolved the drug) was added to a reaction system was defined as a control (100%). When a test drug solution was added, the remaining activity (%) at each concentration was calculated, and then $IC_{50}$ was calculated with a concentration and an inhibition ratio by an inverse estimation using a logistic model. The case that the difference of $IC_{50}$ values was 5 μmol/L or higher was determined as (+). The case that it was 3 μmol/L or lower was determined as (-).

(Results)

Compound No. 1-240 : (-)
Compound No. I-49 : (-)
Compound No. I-239 : (-)

(Example 23 FAT test)

(Method)

20 μL of *Salmonella enterica* subsp. *typhimurium* (*salmonella typhimurium* TA98 line, TA100 line) cryopreserved was inoculated to 10 mL of liquid nutrient medium (2.5% Oxoid nutrient broth No. 2 and then precultured at 37˚C for 10 hours with shaking. Regarding TA98 line, after 9 mL of a bacterial suspension was centrifuged (2000 X g, 10 minutes) to remove the culture solution, the bacteria was suspended in 9 mL of Micro F buffer solution ($K_2HPO_4$: 3.5 g/L, $KH_2PO_4$: 1 g/L, $(NHa)_2SO_4$: 1g/L, tri-sodium citric acid dihydrate: 0.25 g/L, $MgSO_4 \cdot 7H_2O$: 0.1 g/L), and then added to 110 mL of Exposure media (Micro F buffer solution containing biotin: 8 μg/mL, histidine: 0.2 μg/mL, glucose: 8 mg/mL). Regarding TA 100 line, 120 mL of Exposure media was added to 3.16 mL of the bacterial suspension to prepare a test bacterial suspension. 12 μL of a solution of a test substance in DMSO (diluted eight times in a common ratio of 2 from the maximum dose of 50 mg/mL); 12 μL of DMSO as a negative control; 50 μg/mL 4-nitroquinoline-1-oxide solution in DMSO for TA98 line or 0.25 μg/mL 2-(2-furyl)-3-(5-nitro-2-furyl)acrylamide solution in DMSO as a positive control for

TA 100 line in the case of a non-metabolism-activation condition; 12 μL of 40 μg/mL 2-aminoanthracene solution in DMSO for TA98 line or 20 μg/mL of 2-aminoanthracene solution in DMSO for TA100 line in the case of a metabolism-activation condition; were each mixed with 588 μL of the test bacterial suspension (in the case of a metabolism activation condition, a mixed solution of 498 μL of the test bacterial suspension and 90 μL S9 mix) and then cultured at 37˚C at 90 minutes with shaking. 460 μL of the bacterial suspension to which a test substance was exposed was mixed with 2300 μL of Indicator media (a Micro F buffer solution containing 8 μg/mL biotin, 0.2 μg/mL histidine, 8 mg/mL glucose, and 37.5 μg/mL bromocresol purple). 50 μL thereof was dispensed to microplate 48 wells/dose and then statically cultured at 37 ˚C for 3 days. In a well containing bacteria which obtained proliferation potency by mutation of the amino acid (histidine) synthetase gene, the change of pH caused the color change from purple to yellow. Thus, in 48 wells per dose, the number of the bacteria-proliferation wells in which the color changed to yellow was counted, compared with a group of negative controls, and then evaluated. When the mutagenicity is negative, the compound is shown as (-). When positive, the compound is shown as (+).

(Results)

Compound No. I-145: (-)
Compound No. I-1 : (-)
Compound No. 1-138: (-)

(Example 24 hERG test)

(Method)

For the purpose of risk evaluation of QT interval extension of electrocardiogram, the activity on delayed rectification $K^+$ current ($I_{Kr}$), which plays an important role in a cardiac ventricle repolarization process, was examined using the HEK293 cell that was made to express human ether-a-go-go related gene (hERG) channel.

Using an automatic patch-clamp system (PatchXpress 7000A, Axon Instruments Inc.), by a whole-cell patch-clamp method, after the cell was maintained at a membrane potential of -80 mV, $I_{Kr}$ induced upon applying depolarizing stimulation of +50 mV for 2 seconds and further applying repolarizing stimulation of -50 mV for 2 seconds was recorded. After generated electric current became stable, a test substance dissolved at an intended concentration in extracellular fluid (137 mmol/L NaCl, 4 mmol/L KCl, 1.8 mmol/L $CaCl_2 \cdot 2H_2O$, 1 mmol/L $MgCl_2 \cdot 6H_2O$, 10 mmol/L glucose, 10 mmol/L HEPES (4-(2-hydroxyethyl)-1-piperazineethanesulfonic acid), pH = 7.4) was applied to the cell for 10 minutes under room temperature condition. From the obtained $I_{Kr}$, the absolute value of the maximum tail current was measured using analysis software (DataXpress ver. 1, Molecular Devices Corporation) and the value of the electric current in a maintained membrane potential as baseline. Moreover, an inhibition ratio for the maximum tail current prior to the application of the test substance was calculated and then compared with a group of media-application (0.1 % dimethylsulfoxide solution) to evaluate influence of the test substance on $I_{Kr}$.

(Results) The inhibition ratio at 1 μmol/L of compound concentration is shown.

Compound No. I-145: 15%
Compound No. I-109: 1%
Compound No. I-139: 8.7%
Compound No. I-138: 11.1%

(Discussion)

As described above, the compound of the present invention exhibits excellent PI3K inhibitory activity, particularly, PI3Kγ inhibitory activity. The compound of the present invention also encompasses compounds that exhibit PI3Kα and PI3Kγ inhibitoryactivity. Accordingly, the pharmaceutical composition of the present invention may be used for the prophylaxis of and/or as a therapeutic agent for diseases such as encephalitis, myelitis and encephalomyelitis, meningitis, inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpits, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonarydiseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivitypneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like; or used as a therapeutic agent for burn or traumatic inflammation.

(Example 25 Formulation example 1 Tablet)

A tablet consisting of the following constitution is produced by a conventional method.

| | |
|---|---|
| The compound of the present invention | 100 mg |
| Lactose | 60 mg |
| Potato starch | 30 mg |
| Polyvinyl alcohol | 2 mg |
| Magnesium stearate | 1 mg |
| Tar dye | minute amount |

(Example 26 Formulation example 2 Powder)

A powder consisting of the following constitution is produced by a conventional method.

| | |
|---|---|
| The compound of the present invention | 150 mg |
| Lactose | 280 mg |

(Example 27 Formulation example 3 Syrup)

Syrup consisting of the following constitution is produced by a conventional method.

| | |
|---|---|
| The compound of the present invention | 100 mg |
| Refined white sugar | 40 g |
| Ethyl *p*-hydroxybenzoate | 40 mg |
| Propyl *p*-hydroxybenzoate | 10 mg |
| Chocolate flavor | 0.1 cc |

Water was added to this to provide a total amount of 100 cc.

The present invention has been exemplified so far by referring to prefer able embodiments of the present invention, but it should not be construed that the present invention is restricted by the embodiments of the present invention. It should be understood that the scope of the present invention should be construed only by the claims. It

would be understood that those skilled in the art can perform an invention practically equivalent to the present invention, based on the description of the present invention and technical common sense from the specific description of preferable embodiments of the present invention. It would be understood that the patents, patent applications and literature cited herein should be incorporated herein by reference to the present specification in their entire contents, similarly to the case where the contents themselves are described specifically herein.

The present application claims priority to Japanese Patent Application Nos. 2008-228905 and 2009-169598, which were filed in Japan, and which are herein incorporated by reference in their entirety.

INDUSTRIAL APPLICABILITY

The present invention provides medicaments for the treatment of phosphatidylinositol-3-kinase dependent diseases, a compound used therefor, a pharmaceutically acceptable salt thereof, or a solvate thereof.

## Claims

**1.** A compound represented by formula (I):

[Formula 1]

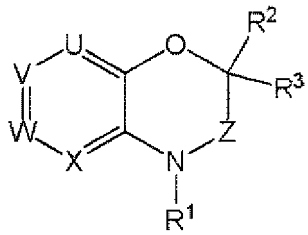

(I)

wherein
U and W are each independently =C(-R')- or =N-;
V and X are each independently =C(-R")- or =N-;
R' and R" are each independently hydrogen, halogen, cyano, nitro, hydroxy, carboxy, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted alkoxy, substituted or unsubstituted aryloxy, substituted or unsubstituted cycloalkyloxy, substituted or unsubstituted heteroaryloxy, substituted or unsubstituted heterocyclyloxy, substituted or unsubstituted acyl, substituted or unsubstituted alkoxycarbonyl, a group represented by the formula: $-SO-R^A$, a group represented by the formula: $-SO_2R^A$, or a group represented by the formula: $-SR^A$;
$R^A$ is hydrogen, substituted or unsubstituted alkyl, substituted or unsubstituted amino, substituted or unsubstituted aryl, substituted or unsubstitutedcycloalkyl, substituted or unsubstituted heteroaryl, or substituted or unsubstituted heterocyclyl;
$R^1$ is substituted or unsubstituted alkyl, substituted or unsubstituted alkenyl, substituted or unsubstituted aryl, substituted or unsubstituted cycloalkyl, substituted or unsubstituted heteroaryl, substituted or unsubstituted heterocyclyl, a group represented by the formula: $-C(=O)-NR^BR^C$, or a group represented by the formula: $-C (=S)-NR^BR^C$;
$R^B$ and $R^C$ are each independently hydrogen, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted alkoxy, substituted or unsubstituted alkenyloxy, substituted or unsubstituted alkynyloxy, substituted or unsubstituted aryloxy, substituted or unsubstituted cycloalkyloxy, substituted or unsubstituted cycloalkenyloxy, substituted or unsubstituted heteroaryloxy, substituted or unsubstituted heterocyclyloxy, or substituted or unsubstituted amino, or
$R^B$ and $R^C$ may be taken together with the adjacent nitrogen atom to form substituted or unsubstituted nitrogenated heterocycle;
$R^2$ and $R^3$ are each independently hydrogen, hydroxy, halogen, cyano, substituted or unsubstituted alkyl, substituted or unsubstituted alkenyl, substituted or unsubstituted aryl, substituted or unsubstituted cycloalkyl, substituted or

unsubstituted cycloalkenyl, substituted or unsubstituted heteroaryl, substituted or unsubstituted heterocyclyl, or substituted or unsubstituted alkoxy, or

$R^2$ and $R^3$ may be taken together with the adjacent carbon atom to form substituted or unsubstituted saturated hydrocarbon ring or substituted or unsubstituted heterocycle, or

$R^2$ and $R^3$ may be taken together to form oxo;

Z is a single bond or $-(CR^4R^5)_n-$;

$R^4$ and $R^5$ are each independently hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino, or

$R^4$ and $R^5$ may be taken together to form oxo;

n is an integer from 1 to 2; and

provided that at least one of U, V, W, and X is =N-, and all four of them are not simultaneously =N-;

Z is not -C (=O) - when U and W are each =C (-R') -, V is =C (-R")-, X is =N-, and $R^1$ is substituted or unsubstituted aryl, substituted or unsubstituted heterocyclyl, or unsubstituted cycloalkyl;

Z is not -C (=O) - when U is =N-, V and X are each =C(-R")-, and W is =C(-R')-; and

$R^1$ is not substituted or unsubstituted thiazolyl or fused thiazolyl when U is =N-; and

provide that the compounds shown below are excluded:

[Formula 2]

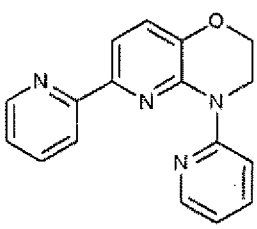
,
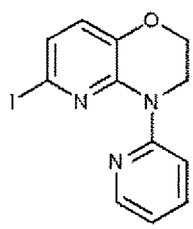
,
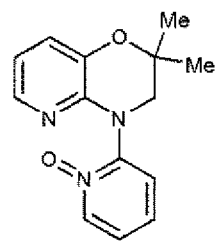

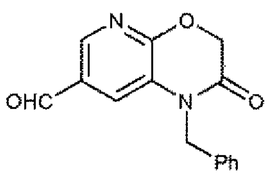
,
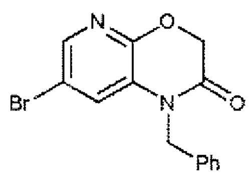
and
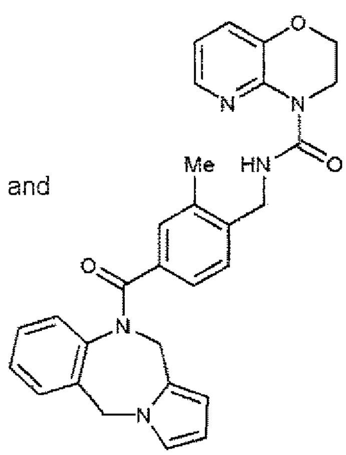
,

or a pharmaceutically acceptable salt thereof, or a solvate thereof.

**2.** The compound according to claim 1, wherein formula (I) :

[Formula 3]

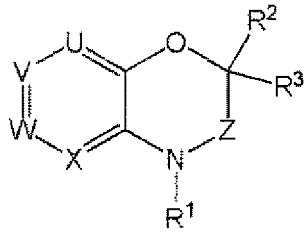 (I)

is selected from formula (II) or formula (III):

[Formula 4]

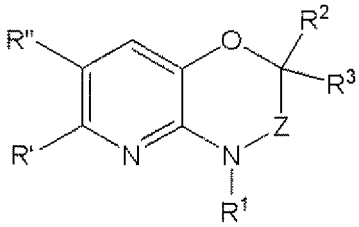 (II) or 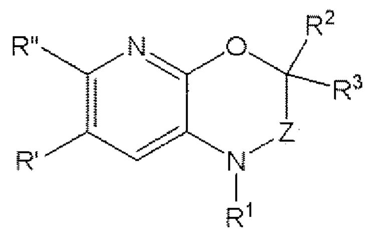 (III)

wherein R', R", $R^2$, and $R^3$ are defined as in claim 1; $R^1$ is substituted or unsubstituted aryl, substituted or unsubstituted cycloalkyl, substituted or unsubstituted heteroaryl, substituted or unsubstituted heterocyclyl, a group represented by the formula: $-C(=O)-NR^BR^C$ wherein $R^B$ and $R^C$ are defined as in claim 1, or a group represented by the formula: $-C(=S)-NR^BR^C$ wherein $R^B$ and $R^C$ are defined as in claim 1; Z is $-(CR^4R^5)_n-$; $R^4$ and $R^5$ are defined as in claim 1; and n is 1,
or a pharmaceutically acceptable salt thereof, or a solvate thereof.

**3.** The compound according to claim 2, wherein formula (I) :

[Formula 5]

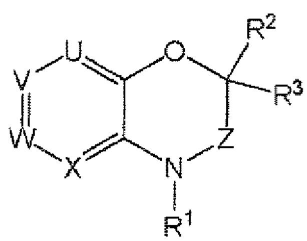 (I)

is formula (II):

[Formula 6]

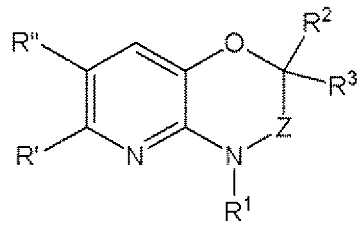

( II )

wherein R', R", $R^2$, and $R^3$ are defined as in claim 1; Z is $-(CR^4R^5)_n-$; $R^4$ and $R^5$ are defined as in claim 1; and n is 1, or a pharmaceutically acceptable salt thereof, or a solvate thereof.

**4.** The compound according to claim 2, wherein formula (I) :

[Formula 7]

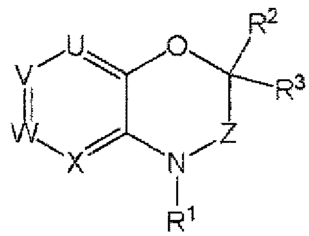

( I )

is formula (III):

[Formula 8]

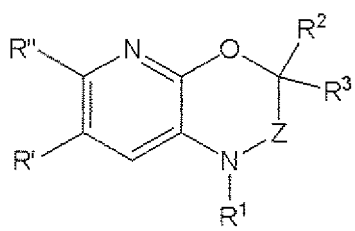

( III )

wherein R', R", $R^2$, and $R^3$ are defined as in claim 1; Z is $-(CR^4R^5)_n-$; $R^4$ and $R^5$ are defined as in claim 1; and is 1, or a pharmaceutically acceptable salt thereof, or a solvate thereof.

**5.** The compound according to any of claims 1 to 4, wherein R' and R" are each independently hydrogen, halogen, nitro, carboxy, substituted or unsubstituted alkyl, substituted or unsubstituted alkenyl, substituted or unsubstitutedaryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted acyl, or substituted or unsubstituted alkoxycarbonyl, or a pharmaceutically acceptable salt thereof, or a solvate thereof.

**6.** The compound according to any of claims 1 to 4, wherein R' is hydrogen, halogen, nitro, carboxy, substituted or unsubstituted alkyl, substituted or unsubstituted alkenyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted amino, substituted or unsubstituted carbamoyl, substituted or unsubstituted acyl, or substituted or unsubstituted alkoxycarbonyl; and
R" is hydrogen, halogen, substituted or unsubstituted alkyl, substituted or unsubstituted aryl, substituted or unsubstituted amino, or substituted or unsubstituted carbamoyl,
or a pharmaceutically acceptable salt thereof, or a solvate thereof.

**7.** The compound according to any of claims 1 to 4, wherein $R^1$ is substituted or unsubstituted aryl, substituted or unsubstituted heteroaryl, substituted or unsubstituted heterocyclyl, or a group represented by the formula: -C (=O)-$NR^BR^C$ wherein $R^B$ and $R^C$ are defined as in claim 1, or a pharmaceutically acceptable salt thereof, or a solvate thereof.

**8.** The compound according to any of claims 1 to 4, wherein $R^1$ is a group represented by the formula: -C (=O) -$NR^BR^C$ wherein $R^B$ and $R^C$ are defined as in claim 1,
or a pharmaceutically acceptable salt thereof, or a solvate thereof.

**9.** The compound according to claim 8, wherein $R^B$ is hydrogen; and $R^C$ is substituted or unsubstituted alkyl, substituted or unsubstituted aryl, substituted or unsubstituted cycloalkyl, substituted or unsubstituted heteroaryl, or substituted or unsubstituted heterocyclyl,
or a pharmaceutically acceptable salt thereof, or a solvate thereof.

**10.** The compound according to any of claims 1 to 4, wherein $R^2$ and $R^3$ are each hydrogen,
or a pharmaceutically acceptable salt thereof, or a solvate thereof.

**11.** The compound according to any of claims 1 to 4, wherein Z is - $(CR^4R^5)_n$- wherein $R^4$, $R^5$, and n are defined as in claim 1, or a pharmaceutically acceptable salt thereof, or a solvate thereof.

**12.** The compound according to claim 11, wherein $R^4$ and $R^5$ are each hydrogen; and n is 1,
or a pharmaceutically acceptable salt thereof, or a solvate thereof.

**13.** A pharmaceutical composition containing the compound according to any of claims 1 to 12, or a pharmaceutically acceptable salt thereof, or a solvate thereof.

**14.** The pharmaceutical composition according to claim 13, which is a phosphatidylinositol-3-kinase inhibitor.

**15.** Use of the compound, or a pharmaceutically acceptable salt thereof, or a solvate thereof according to any of claims 1 to 12 for producing a therapeutic agent and/or a prophylactic agent for inflammation.

**16.** The compound, or a pharmaceutically acceptable salt thereof, or a solvate thereof according to any of claims 1 to 12 for the therapy and/or prophylaxis of inflammation.

**17.** A method for the therapy and/or prophylaxis of inflammation, **characterized by** administering the compound, or a pharmaceutically acceptable salt thereof, or a solvate thereof according to any of claims 1 to 12.

**INTERNATIONAL SEARCH REPORT**

International application No.
PCT/JP2009/065366

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | PERRY, B. et al, Achieving multi-isoform PI3K inhibition in a series of substituted 3, 4-dihydro-2H-benzo[1, 4]oxazines, Bioorganic & Medicinal Chemistry Letters, 2008.07.05, Vol.18, No.16, p.4700-4704, entire text, particularly, compounds 19, 21, table 1 | 1,5-7,10-16<br>1,5,6,10-16<br>2-4,8,9 |
| Y | WO 2008/044022 A1 (UCB PHARMA S.A.), 17 April 2008 (17.04.2008), entire text; particularly, example 9; pages 22 to 23<br>& EP 2076512 A1 | 1,5,6,10-16 |

☒ Further documents are listed in the continuation of Box C.  ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 01 December, 2009 (01.12.09) | 08 December, 2009 (08.12.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2009/065366

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2006-510661 A (Warner-Lambert Company L.L.C.), 30 March 2006 (30.03.2006), entire text; particularly, claim 1; examples; table 1 & WO 2004/052373 A1 & CA 2508601 A1 & AU 2003280188 A1 & EP 1569653 A1 & BR 2003016386 A & US 2004/0121996 A1 & US 7205295 B2 & MX 2005005585 A | 1,5,6,10-16 |
| X | WO 2008/064274 A1 (RIGEL PHARMACEUTICALS, INC., USA), 29 May 2008 (29.05.2008), entire text; particularly, compound 1; paragraphs [0281] to [0287] & CA 2673137 A1 & EP 2078026 A1 | 1,5,6,11,13, 15,16 |
| X | WO 2007/024949 A2 (SCHERING CORP., USA), 01 March 2007 (01.03.2007), entire text; particularly, compound 117; pages 122 to 123 & WO 2007/024949 A3 & AU 2006283109 A1 & CA 2620173 A1 & EP 1934203 A2 & JP 2009-506051 A & MX 2008002722 A & KR 2008037070 A & NO 2008001427 A & ZA 2008002495 A & CN 101374830 A & JP 2009-185016 A | 1,5,6,10-13, 15,16 |
| X | WO 2007/022257 A2 (CHEMOCENTRYX, INC., USA), 22 February 2007 (22.02.2007), entire text; particularly, examples 24, 27; table 2 & WO 2007/022257 A3 & US 2007/0066583 A1 & AU 2006279525 A1 & CA 2618844 A1 & EP 1931348 A2 & JP 2009-504754 A | 1,5,6,10,11, 13,15,16 |
| X | WO 2006/133426 A2 (RIGEL PHARMACEUTICALS, INC., USA), 14 December 2006 (14.12.2006), entire text; particularly, example 36; table XII & WO 2006/133426 A3 & AU 2006254840 A1 & CA 2608367 A1 & EP 1904457 A2 & JP 2008-543778 A & MX 2007015464 A & CN 101282945 A & NO 2008000075 A & KR 2008017454 A & US 2008/0306099 A1 & US 2009/0041786 A1 | 1,5,6,11,13, 15,16 |
| X | WO 2006/114706 A1 (PFIZER LTD., UK), 02 November 2006 (02.11.2006), entire text; particularly, preparation examples 9, 10, 26, 36; examples 6, 7; table 4 & CA 2605899 A1 & EP 1877399 A1 & JP 2008-539220 A & US 2008/0188478 A1 | 1,5-7,13,15, 16 |

Form PCT/ISA/210 (continuation of second sheet) (April 2007)

**INTERNATIONAL SEARCH REPORT**

International application No.
PCT/JP2009/065366

C (Continuation). DOCUMENTS CONSIDERED TO BE RELEVANT

| Category* | Citation of document, with indication, where appropriate, of the relevant passages | Relevant to claim No. |
|---|---|---|
| X | JP 2008-527048 A (Rigel Pharmaceuticals, Inc.), 24 July 2008 (24.07.2008), entire text; particularly, compound 1; paragraphs [0237] to [0245]<br>& WO 2006/078846 A1 & AU 2006206458 A1<br>& CA 2591948 A1 & US 2006/0211657 A1<br>& US 7449458 B2 & EP 1856135 A1<br>& ZA 2007005410 A & US 2006/0234983 A1<br>& US 7538108 B2 & US 7563892 B1<br>& CN 101115761 A & NO 2007004179 A<br>& KR 2007098926 A & US 2009/0124580 A1 | 1,5,6,11,13, 15,16 |
| X | JP 2007-536263 A (Neurogen Corp.), 13 December 2007 (13.12.2007), entire text; particularly, compound 133; example 18<br>& WO 2005/110416 A2 & WO 2005/110416 A3<br>& AU 2005244104 A1 & CA 2563607 A1<br>& US 2005/0277654 A1 & US 7482350 B2<br>& EP 1745033 A2 & CN 1976918 A | 1,5,6,10-13, 15,16 |
| X | JP 2007-500722 A (Rigel Pharmaceuticals, Inc.), 18 January 2007 (18.01.2007), entire text; particularly, compound 549; tables 1, 2<br>& WO 2005/016893 A2 & WO 2005/016893 A3<br>& AU 2004265288 A1 & CA 2533377 A1<br>& US 2005/0209224 A1 & US 7122542 B2<br>& US 2005/0234049 A1 & EP 1656372 A2<br>& BR 2004013018 A & CN 1849318 A<br>& SG 145698 A1 & RU 2356901 C2<br>& KR 2006056353 A & MX 2006001099 A | 1,5,6,11,13, 15,16 |
| X | JP 60-004188 A (Kabushiki Kaisha Kayaku Kosei Busshitsu Kenkyusho), 10 January 1985 (10.01.1985), entire text; particularly, examples; experimental examples<br>(Family: none) | 1,5,6,11-13, 15,16 |
| X | WO 2008/051493 A2 (SIGNAL PHARMACEUTICALS, L.L.C., USA), 02 May 2008 (02.05.2008), entire text; particularly, compound 86; paragraphs [00892] to [00893]<br>& WO 2008/051493 A3 & AU 2007309467 A1<br>& CA 2666618 A1 & US 2009/0023724 A1<br>& EP 2078016 A2 & KR 2009082407 A<br>& EP 2090577 A2 & EP 2090577 A3 | 1,5,6,13,15, 16 |

Form PCT/ISA/210 (continuation of second sheet) (April 2007)

**INTERNATIONAL SEARCH REPORT**

International application No.
PCT/JP2009/065366

C (Continuation). DOCUMENTS CONSIDERED TO BE RELEVANT

| Category* | Citation of document, with indication, where appropriate, of the relevant passages | Relevant to claim No. |
|---|---|---|
| X | US 2006/0161001 A1 (AMGEN INC., USA), 20 July 2006 (20.07.2006), entire text; particularly, example 98; paragraphs [0353] to [0432]<br>& WO 2006/069258 A1 & AU 2005319137 A1<br>& AU 2005319137 B2 & CA 2591946 A1<br>& EP 1833831 A1 | 1,5-7,10-13, 15,16 |
| X | JP 2008-510684 A (Ferring B.V.), 10 April 2008 (10.04.2008), entire text; particularly, compound 736; example A<br>& WO 2006/021213 A2 & WO 2006/021213 A3<br>& EP 1632494 A1 & AU 2005276790 A1<br>& AU 2005276790 B2 & CA 2567776 A1<br>& EP 1781661 A2 & BR 2005014661 A<br>& KR 2009083475 A & RU 2370497 C2<br>& CN 101563347 A & KR 2007032313 A<br>& MX 2007001392 A & ZA 2007001257 A | 1,5,6,10-13, 16 |
| X | WO 2007/138974 A1 (Toyama Chemical Co., Ltd.), 06 December 2007 (06.12.2007), entire text; particularly, example 112; table 6<br>& AU 2007268749 A1 & CA 2652501 A1<br>& EP 2022793 A1 & NO 2008004701 A<br>& MX 2008014908 A & US 2009/0198063 A1<br>& CN 101454319 A & KR 2009018976 A | 1,5,6,10-13, 16 |
| X | WO 2007/125952 A1 (Shionogi & Co., Ltd.), 08 November 2007 (08.11.2007), entire text; particularly, compound (Ii-75); table 1<br>& AU 2007244358 A1 & CA 2650683 A1<br>& EP 2017261 A1 & MX 2008013511 A<br>& KR 2009007571 A & NO 2008004983 A<br>& CN 101479238 A & JP 2009-221230 A | 1,5,6,13,16 |
| X | MA, C. et al, Synthesis and biological evaluation of pyridoxazine-tetrahydroisoquinoline derivatives as MDR modulators, Chemistry Letters, 2006.07.29, Vol.35, No.9, p.1010-1011,entire text, particularly, compound 7 | 1,5,6,10,11, 16 |
| X | WO 2001/030782 A2 (SYNGENTA PARTICIPATIONS A.-G., SWITZ.), 03 May 2001 (03.05.2001), entire text; particularly, compounds (1.006), (1.008)<br>& WO 2001/030782 A3 | 1,5,6,10,11, 16 |

Form PCT/ISA/210 (continuation of second sheet) (April 2007)

**INTERNATIONAL SEARCH REPORT**

International application No.
PCT/JP2009/065366

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 2009/025847 A2 (NODALITY, INC., USA), 26 February 2009 (26.02.2009), entire text; particularly, compound R788 & WO 2009/025847 A3 & US 2009/0098594 A1 | 1,5,6,11, 13-16 |
| P,X | WO 2009/012421 A1 (RIGEL PHARMACEUTICALS, INC., USA), 22 January 2009 (22.01.2009), entire text; particularly, compound (II-26); table VII (Family: none) | 1,5,6,10,11, 13,15,16 |
| P,X | WO 2009/073779 A1 (SCHERING CORP., USA), 11 June 2009 (11.06.2009), entire text; particularly, pages 67, 117, 118 (Family: none) | 1,5,6,10-13, 16 |

Form PCT/ISA/210 (continuation of second sheet) (April 2007)

**INTERNATIONAL SEARCH REPORT**

International application No.
PCT/JP2009/065366

Continuation of A. CLASSIFICATION OF SUBJECT MATTER
(International Patent Classification (IPC))

*C07D498/04*(2006.01)i, *A61K31/5383*(2006.01)i, *A61K31/541*(2006.01)i, *A61P1/00*(2006.01)i, *A61P1/02*(2006.01)i, *A61P1/04*(2006.01)i, *A61P1/16*(2006.01)i, *A61P1/18*(2006.01)i, *A61P3/04*(2006.01)i, *A61P3/10*(2006.01)i, *A61P5/14*(2006.01)i, *A61P7/00*(2006.01)i, *A61P7/02*(2006.01)i, *A61P7/06*(2006.01)i, *A61P9/00*(2006.01)i, *A61P9/04*(2006.01)i, *A61P9/10*(2006.01)i, *A61P11/00*(2006.01)i, *A61P11/04*(2006.01)i, *A61P11/06*(2006.01)i, *A61P13/00*(2006.01)i, *A61P13/08*(2006.01)i, *A61P13/10*(2006.01)i, *A61P13/12*(2006.01)i, *A61P15/02*(2006.01)i, *A61P15/08*(2006.01)i, *A61P17/00*(2006.01)i, *A61P17/02*(2006.01)i, *A61P17/06*(2006.01)i, *A61P17/14*(2006.01)i, *A61P19/00*(2006.01)i, *A61P19/02*(2006.01)i, *A61P19/04*(2006.01)i, *A61P19/08*(2006.01)i, *A61P21/00*(2006.01)i, *A61P21/04*(2006.01)i, *A61P25/00*(2006.01)i, *A61P27/02*(2006.01)i, *A61P27/06*(2006.01)i, *A61P27/14*(2006.01)i, *A61P27/16*(2006.01)i, *A61P29/00*(2006.01)i, *A61P31/04*(2006.01)i, *A61P35/00*(2006.01)i, *A61P37/06*(2006.01)i, *A61P37/08*(2006.01)i, *A61P43/00*(2006.01)i

(According to International Patent Classification (IPC) or to both national classification and IPC)

Continuation of B. FIELDS SEARCHED
Minimum documentation searched (International Patent Classification (IPC))

C07D498/04, A61K31/5383, A61K31/541, A61P1/00, A61P1/02, A61P1/04, A61P1/16, A61P1/18, A61P3/04, A61P3/10, A61P5/14, A61P7/00, A61P7/02, A61P7/06, A61P9/00, A61P9/04, A61P9/10, A61P11/00, A61P11/04, A61P11/06, A61P13/00, A61P13/08, A61P13/10, A61P13/12, A61P15/02, A61P15/08, A61P17/00, A61P17/02, A61P17/06, A61P17/14, A61P19/00, A61P19/02, A61P19/04, A61P19/08, A61P21/00, A61P21/04, A61P25/00, A61P27/02, A61P27/06, A61P27/14, A61P27/16, A61P29/00, A61P31/04, A61P35/00, A61P37/06, A61P37/08, A61P43/00

Minimum documentation searched (classification system followed by classification symbols)

Form PCT/ISA/210 (extra sheet) (April 2007)

**INTERNATIONAL SEARCH REPORT**

International application No.
PCT/JP2009/065366

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 17
because they relate to subject matter not required to be searched by this Authority, namely:
Claim 17 pertains to methods for treatment of the human body by therapy and thus relates to a subject matter which this International Searching Authority is not required, under the provisions of Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

**INTERNATIONAL SEARCH REPORT**

International application No.
PCT/JP2009/065366

<Claims 1 and 5-16>

The inventions of claims 1 and 5-16 of the present application include great many compounds. However, only compounds of formula (I) wherein U or X is "=N-", V is "=C(-R'')-" and W is "=C(-R')-", which are mentioned in the description, are disclosed in the meaning within PCT Article 5. Therefore, the inventions are not supported in the meaning within PCT Article 6.

In this international search report, the search was made on the compounds represented by formula (I) recited in claim 1.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7145051 A
- JP 7029673 A
- JP 2004056820 A
- JP 2004052373 A
- JP 2004007491 A
- JP 2004006916 A
- WO 2006040318 A
- WO 2007089034 A
- WO 20070117785 A
- WO 2006114706 A
- WO 2006018443 A
- WO 2005100349 A
- WO 2002020011 A
- WO 2002014315 A
- WO 2001030782 A
- WO 2008044022 A
- US 20010053853 A
- US 20010053853 A1
- JP 2008228905 A
- JP 2009169598 A


### Non-patent literature cited in the description


- **M.P. Wymann et al.** *Biochemical Society Transactions,* 2003, vol. 31, 275-280
- **Rueckle T. et al.** *NATURE REVIEWS DRUG DISCOVERY,* 2006, vol. 5, 903-918
- **Laffargue M. et al.** *Immunity,* 2002, vol. 16, 441-451
- **Hirsch E. et al.** *Science,* 2000, vol. 287, 1049-1053
- **Li Z. et al.** *Science,* 2000, vol. 287, 982-983
- **Sasaki T. et al.** *Science,* 2000, vol. 287, 1040-1046
- **Lupia E. et al.** *Am J Pathol.,* 2004, vol. 165, 2003-2011
- **Barber DF et al.** *Nat Med,* 2005, vol. 11, 933-935
- **Camps.** *Nat Med,* 2005, vol. 11, 936-943
- **Campbell et al.** *Circ Res.,* 2005, vol. 96, 197-206
- **Yadav M. et al.** *J Immunol.,* 2006, vol. 176, 5494-503
- *Japanese Journal of Clinical Immunology,* 2007, vol. 30 (5), 369-374
- **Ui MT et al.** *Trends Biochem. Sci.,* 1995, vol. 20, 303-307
- **Grice C. A. et al.** *Bioorganic & Medicinal Chemistry Letters,* 2006, vol. 16 (8), 2209-2212
- *Hecheng Huaxue,* 2003, vol. 11 (6), 513-516
- *Chemical & Pharmaceutical Bulletin,* 1999, vol. 47 (7), 971-979
- **Shin D.-S. ; Yoon Y.-J. et al.** *Journal of Organic Chemistry,* 2003, vol. 68 (20), 7918-7920
- **Enguang Feng et al.** *Journal of Organic Chemistry,* 2009, vol. 74 (7), 2846-2849
- **Benjamin Perry et al.** *Bioorganic & Medicinal Chemistry Letters,* 2008, vol. 18, 4700-4704
- Isotopes in the Physical and Biomedical Science. Labeled Compounds. 1987, vol. 1
- *Journal of Organic Chemistry,* 2003, vol. 68, 7918-7920
- **T. W. Green.** Protective Groups in Organic Synthesis. John Wiley & Sons Inc, 1991
- **T. W. Greene.** Protective Groups in Organic Synthesis. John Wiley & Sons Inc, 1991
- **R. C. Larock.** Comprehensive Organic Transformations. 1989